# EUROPEAN PATENT SPECIFICATION

(11) **EP 4 347 574 B1**
(45) Date of publication and mention of the grant of the patent: **25.11.2026**
(21) Application number: 22732817.6
(22) Date of filing: 27.05.2022
(51) Int. Cl.: C07D 263/20, C07C 311/05, C07D 498/04, A61P 35/00

(54) **PROCESS FOR THE PREPARATION OF BENZOXAZEPIN OXAZOLIDINONE COMPOUNDS**
VERFAHREN ZUR HERSTELLUNG VON BENZOXAZEPAN OXAZOLIDINON VERBINDUNGEN
PROCÉDÉ POUR LA PRÉPARATION DES COMPOSÉS DES BENZOXAZEPAN ET OXAZOLIDINONES

(30) Priority: 28.05.2021 US 202163194382 P
(43) Date of publication of application: 10.04.2024
(73) Proprietor: Genentech, Inc., South San Francisco, CA 94080-4990 (US)
(72) Inventor: ANGELAUD, Remy, San Francisco, California 94080-4990 (US); BRUETSCH, Tobias, 5605 Dottikon (CH); GOSSELIN, Francis, San Francisco, California 94080 (US); HAN, Chong, San Francisco, California 94080 (US); STUTZ, Alfred, 5605 Dottikon (CH)
(74) Representative: Sauer, Frank
(86) International application number: PCT/US2022/031243
(87) International publication number: WO 2022/251567

(56) References cited:
- EP-A1- 3 805 231
- WO-A1-2017/001645
- WO-A1-2018/109204
- CN-A- 109 265 408
- Y. ZHAO ET. AL.: "Efficient and Direct Nucleophilic Difluoromethylation of Carbonyl and Imine Compounds with Me3SiCF2H at Ambient or Low Temperature", ORGANIC LETTERS, vol. 13, no. 19, 12 September 2011 (2011-09-12), pages 5342 - 5345, XP055951346, DOI: 10.1021/ol202208b

## Description

### CROSS REFERENCE TO RELATED APPLICATIONS

This application claims the benefit of and priority to U.S. Provisional Patent Application No. 63/194,382, filed 28 May 2021.

### FIELD OF THE INVENTION

The invention relates to methods of making benzoxazepin oxazolidinone compounds, and useful intermediates.

### BACKGROUND OF THE INVENTION

The PI3 kinase/Akt/PTEN pathway is an attractive target for cancer drug development since such agents would be expected to inhibit cellular proliferation, to repress signals from stromal cells that provide for survival and chemoresistance of cancer cells, to reverse the repression of apoptosis and surmount intrinsic resistance of cancer cells to cytotoxic agents. PI3K is activated through receptor tyrosine kinase signaling as well as activating mutations in the p110 catalytic subunit of PI3K, loss of the tumor suppressor PTEN, or through rare activating mutations in AKT.

Benzoxazepin compounds have potent and selective activity as inhibitors of the PI3K alpha isoform. Taselisib (GDC-0032, Roche RG7604, CAS Reg. No. 1282512-48-4, Genentech Inc.), named as 2-(4-(2-(1-isopropyl-3-methyl-1H-1,2,4-triazol-5-yl)-5,6-dihydrobenzo[f]imidazo[1,2-d][1,4]oxazepin-9-yl)-1H-pyrazol-1-yl)-2-methylpropanamide, has potent PI3K activity (Ndubaku, C. O. et al (2013) J. Med. Chem. 56:4597-4610; WO 2011/036280; US 8242104; US 8343955) and is being studied in patients with locally advanced or metastatic solid tumors. Taselisib (GDC-0032) is a beta-isoform sparing inhibitor of the PI3K catalytic subunit, 31x more selective for the alpha subunit, compared to beta. Taselisib displays greater selectivity for mutant PI3Kα isoforms than wild-type PI3Kα (Olivero AG et al, AACR 2013. Abstract DDT02-01). Taselisib is currently being developed as a treatment for patients with oestrogen receptor (ER)-positive, HER2-negative metastatic breast cancer (mBC) and non-small cell lung cancer (NSCLC). There is a need for new selective inhibitors of mutant PI3Kα isoforms.

Inavolisib, also known as GDC-0077 or by the IUPAC name: (S)-2-((2-((S)-4-(difluoromethyl)-2-oxooxazolidin-3-yl)-5,6-dihydrobenzo[f]imidazo[1,2-d][1,4]oxazepin-9-yl)amino)propanamide, has potent PI3K activity (WO 2017/001645, US 2017/0015678, Edgar K. et al, #156, "Preclinical characterization of GDC-0077, a specific PI3K alpha inhibitor in early clinical development", and Staben. S. #DDT02-0 "Discovery of GDC-0077, a highly isoform selective inhibitor of PI3K alpha that promotes selective loss of mutant-p110alpha", American Assoc. for Cancer Res. (AACR) annual meeting, April 2, 2017, Washington DC), and is being studied in patients with locally advanced or metastatic solid tumors. There remains a need for new processes for making benzoxazepin oxazolidinone compounds such as inavolisib. WO 2018/109204 A1 discloses structurally related compounds. The compounds are active in the inhibition of P13K.

### SUMMARY OF THE INVENTION

The invention relates to methods of making benzoxazepin oxazolidinone compounds and intermediates thereof.

In one aspect, provided is a compound of Formula (8A): 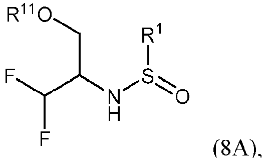 or a salt thereof, wherein:
R¹ is an optionally substituted C₁₋₁₂ alkyl, an optionally substituted C₃₋₁₄ cycloalkyl, or an optionally substituted C₆₋₁₄ aryl; and
R¹¹ is hydrogen or a hydroxyl protecting group.

In some embodiments, R¹ is the optionally substituted C₁₋₁₂ alkyl. In some embodiments, R¹ is an optionally substituted tertiary C₄₋₁₂ alkyl. In some embodiments, R¹ is selected from the group consisting of tert-butyl, tert-pentyl, 3-ethylpentan-3-yl, 1-methylcyclohexyl, 1-adamantyl, phenyl, and naphthyl.

In some embodiments, R¹¹ is hydrogen. In some embodiments, R¹¹ is benzyl.

In some embodiments, the compound of the Formula (8A) is of Formula (8B): 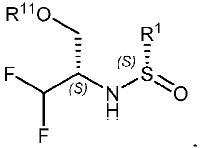 or a salt thereof. wherein R¹ is an optionally substituted C₁₋₁₂ alkyl, an optionally substituted C₃₋₁₄ cycloalkyl, or an optionally substituted C₆₋₁₄ aryl; and R¹¹ is hydrogen or a hydroxyl protecting group.

In some embodiments, the compound of the Formula (8A) is of Formula (8-1): 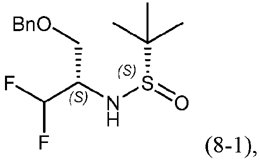 or a salt thereof; or Formula (8-2): 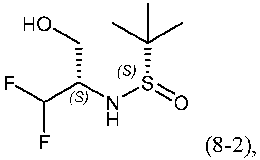 or a salt thereof.

In another aspect, provided is a compound of Formula (7A): 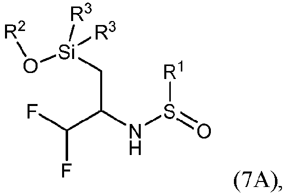 or a salt thereof, wherein:
R¹ is an optionally substituted C₁₋₁₂ alkyl, an optionally substituted C₃₋₁₄ cycloalkyl, or an optionally substituted C₆₋₁₄ aryl;
R² is an optionally substituted C₁₋₁₂ alkyl or an optionally substituted C₆₋₁₄ aryl; and 10
each R³ is independently an optionally substituted C₁₋₁₂ alkyl, an optionally substituted C₆₋₁₄ aryl, or OR².

In some embodiments, the compound of the Formula (7A) is of the formula (7): 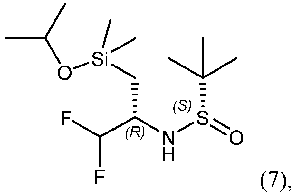 or a salt thereof.

In yet another aspect, provided is a process for the preparation of a compound of Formula (8C): 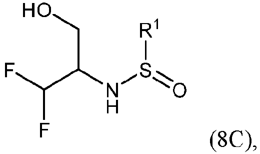 or a salt thereof, wherein:
R¹ is an optionally substituted C₁₋₁₂ alkyl, an optionally substituted C₃₋₁₄ cycloalkyl, or an optionally substituted C₆₋₁₄ aryl;
the process comprising the steps of:
(iii) reacting a compound of Formula (4A): 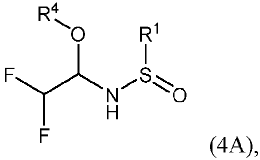 or a salt thereof, wherein:
   R¹ is an optionally substituted C₁₋₁₂ alkyl, an optionally substituted C₃₋₁₄ cycloalkyl, or an optionally substituted C₆₋₁₄ aryl; and
   R⁴ is an optionally substituted C₁₋₆ alkyl or hydrogen;
   with a Grignard reagent of formula (5A): 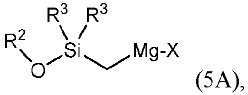 wherein:
   R² is an optionally substituted C₁₋₁₂ alkyl or an optionally substituted C₆₋₁₄ aryl;
   each R³ is independently an optionally substituted C₁₋₁₂ alkyl, an optionally substituted C₆₋₁₄ aryl, or OR²; and
   X is a halide;
   to thereby form a compound of Formula (7A): 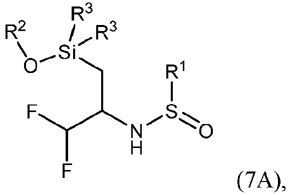 or a salt thereof,
   and
(iv) reacting the compound of Formula (7A) with a fluoride salt, a base, and an oxidant to form the compound of Formula (8C).

In some embodiments, the process further comprises the steps of:
(i) partially reducing a compound of Formula (1A): 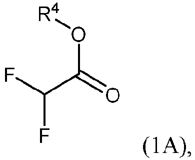 or a salt thereof,
   wherein R⁴ is an optionally substituted C₁₋₆ alkyl or hydrogen, to form a compound of Formula (2A): 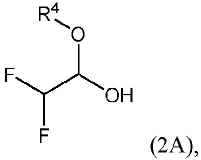 or a salt thereof,
   and
(ii) reacting the compound of Formula (2A) with a sulfonamide compound of Formula (3A): 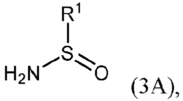 wherein R¹ is an optionally substituted C₁₋₁₂ alkyl, an optionally substituted C₃₋₁₄ cycloalkyl, or an optionally substituted C₆₋₁₄ aryl, in the presence of a dehydrating reagent to form the compound of Formula (4A): 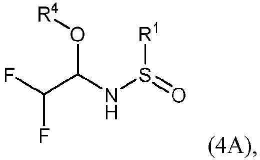 or a salt thereof.

In some of these embodiments, the process further comprises the step of:
(v) reacting the compound of Formula (8C): 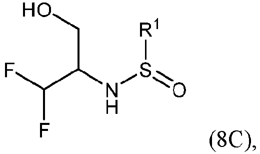 or a salt thereof,
   wherein R¹ is as defined in claim 11, with an acid to thereby yield an amine compound of Formula (9-1): 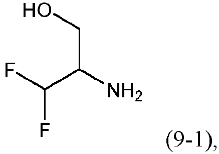
or an acid addition salt thereof.

In some of these embodiments, the process further comprises the step of:
(vi) reacting the compound of Formula (9-1), or the acid addition salt thereof, with an acylating reagent to form a compound of Formula (10-1): 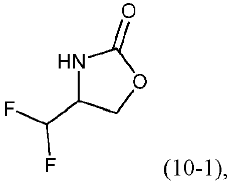 or a salt thereof.

In some embodiments, the compound of Formula (7A) is of Formula (7B): 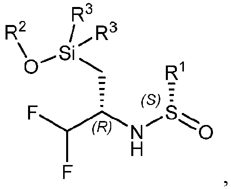 or a salt thereof, and the compound of Formula (8C) is of Formula (8D): 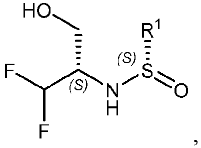 or a salt thereof, wherein R¹, R², and R³ are as defined above.

In some embodiments, the compound of Formula (3A) is of Formula (3B): 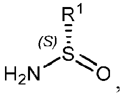 and the compound of Formula (4A) is of Formula (4B): 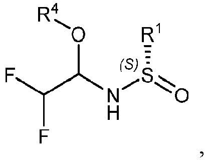 or a salt thereof, wherein R¹ and R⁴ are as defined above.

In some embodiments, the compound of Formula (9-1) is of Formula (9-3): 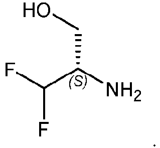 In some of these embodiments, the compound of Formula (10-1) is of Formula (10-2):

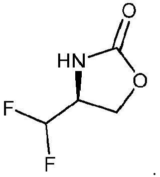

In some of these embodiments, R¹ is *tert-butyl.* In some of these embodiments, R² is 2-propyl, each R³ is methyl, and X is chloride. In some of these embodiments, R⁴ is ethyl.

In some of these embodiments, the acid for step (v) is HCl, and the acid addition salt of the compound of Formula (9-1) is a hydrochloride salt having the structure (9-2):

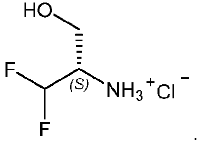

In one embodiment, the process of preparing a compound of Formula (8C) is a process comprising the steps of:
(iii) reacting a compound of formula (4): 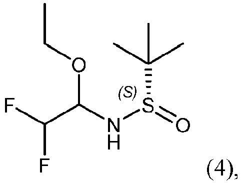 or a salt thereof,
   with a compound of formula (5): 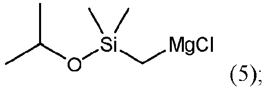 in a solvent (e.g., THF) to form a compound of formula (7): 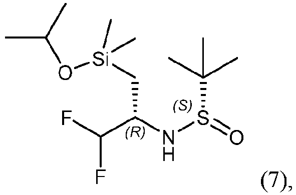 or a salt thereof;
   and
(iv) reacting the compound of formula (7) with potassium fluoride, potassium bicarbonate, and hydrogen peroxide in a solvent (e.g., methanol) to form a compound of formula (8-2):

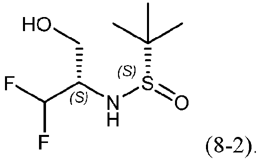

In yet another aspect, provided is a process for the preparation of a compound of Formula (8A): 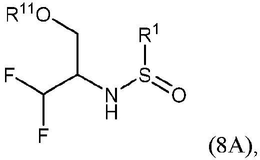 or a salt thereof,
wherein:
R¹ is an optionally substituted C₁₋₁₂ alkyl, an optionally substituted C₃₋₁₄ cycloalkyl, or an optionally substituted C₆₋₁₄ aryl; and
R¹¹ is a hydroxyl protecting group, the process comprising the steps of:
   (b) reacting a compound of Formula (12A): 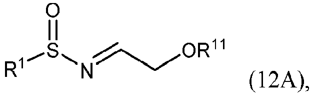 with a compound of Formula (13A): 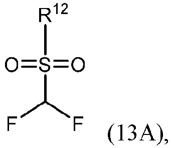 wherein R¹² is optionally substituted C₆₋₁₄ aryl and a base at a temperature below 0°C to form a compound of Formula (14A): 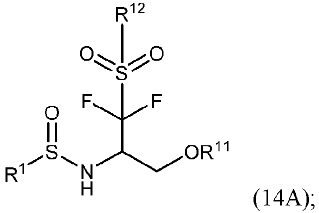 and
   (c) reacting the compound of Formula (14A) with magnesium in the presence of an acetate buffer to thereby form the compound of Formula (8A).

In some of these embodiments, the process further comprises the step of: (a) reacting a compound of Formula (11A): 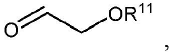 with a sulfonamide compound of Formula (3A): 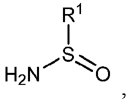 in the presence of a dehydrating reagent to form the compound of Formula (12A): 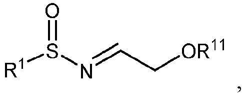 wherein R¹ and R¹¹ are as defined above. In some embodiments, the process further comprises the step of: (d) reacting the compound of Formula (8A): 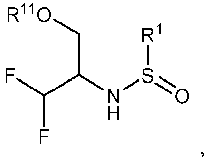 or a salt thereof, with an acid to yield an amine compound of Formula (9A): 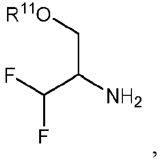 or an acid addition salt thereof, wherein R¹ and R¹¹ are as defined above. In some embodiments, the process further comprises the steps of: (e) removing the hydroxyl protecting group of the compound of Formula (9A) to form a compound of Formula (9-1): 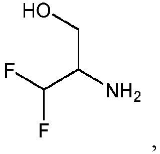 or an acid addition salt thereof; and (f) reacting the compound of Formula (9-1), or an acid addition salt thereof, with an acylating reagent to form a compound of Formula (10-1): 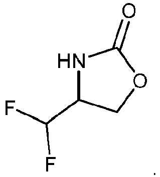 In some of these embodiments, the compound of Formula (12A) is of Formula (12B): 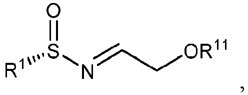 the compound of Formula (14A) is of Formula (14B): 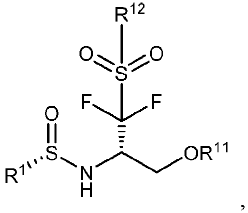 and the compound of Formula (8A) is of Formula (8B): 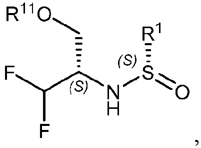 or a salt thereof, wherein R¹, R¹¹, and R¹² are as defined above. In some embodiments, where applicable, the compound of Formula (3A) is of Formula (3B): 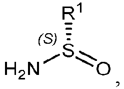 wherein R¹ is as defined above. In some embodiments, where applicable, the compound of Formula (9A) is of Formula (9B): 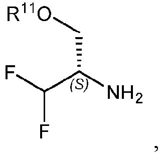 or a salt thereof, wherein R¹¹ is as defined above. In some embodiments, where applicable, the acid in step (d) is HCl and the acid addition salt of the compound of Formula (9A) or (9B) is a hydrochloride salt having the structure (9C): 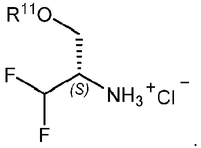 In some embodiments, where applicable, the compound of Formula (9-1) is of Formula (9-3): 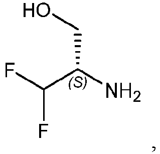 or the acid addition salt thereof; and the compound of Formula (10-1) is of Formula (10-2): 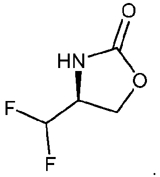 In some embodiments, R¹ is *tert*-butyl. In some embodiments, R¹¹ is benzyl. In some embodiments, R¹² is phenyl, and the compound of Formula (13A) has the structure (13): 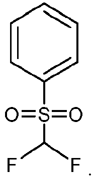 In some embodiments, the base in step (b) is NaHMDS, and step (b) is performed at a temperature of about -70 °C. In some embodiments, the acetate buffer in step (c) comprises HOAc and NaOAc. In some embodiments, the dehydrating reagent in step (a) comprises CuSO₄.

In some of these embodiments, the process further comprises reacting a compound of Formula (10-1) having the structures: 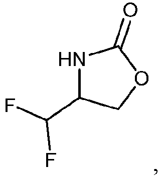 or Formula (10-2) having the structures: 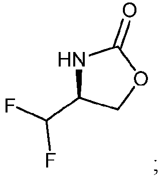 with compound 15, having the structure: 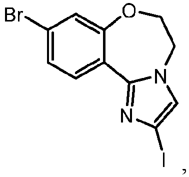 a copper salt and a ligand to form compound 16, having the structure: 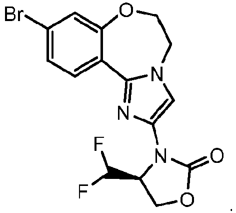 In some of these embodiments, the copper salt is copper(II) acetate. In some of these embodiments, the copper salt is copper (I) iodide. In some embodiments, the ligand is *trans-N,N-dimethylcyclohexane-1,2-*diamine.

In some of these embodiments, the process further comprises reacting compound 16 with (S)-2-aminopropanoic acid and a copper (I) catalyst to form compound 17, having the structure: 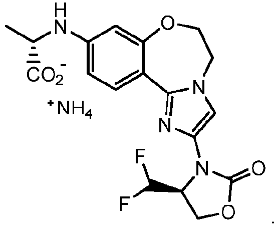 In some embodiments, the copper (I) catalyst is copper(I) oxide. In some of these embodiments, the process further comprises reacting compound **17** with ammonia (or an ammonia equivalent) and a peptide coupling reagent to form compound **18,** having the structure:

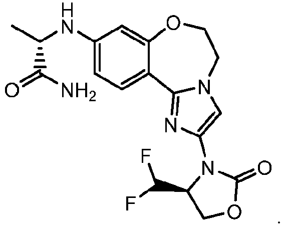

In yet another aspect, provided is a process for the preparation of a compound of Formula (8A): 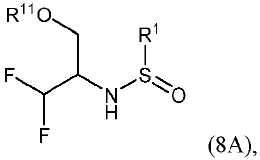 or a salt thereof,
wherein:
R¹ is an optionally substituted C₁₋₁₂ alkyl, an optionally substituted C₃₋₁₄ cycloalkyl, or an optionally substituted C₆₋₁₄ aryl; and
R¹¹ is a hydroxyl protecting group, the process comprising the steps of:
   (ii) reacting a compound of Formula (4A): 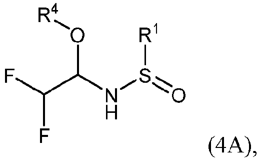 or a salt thereof,
      wherein R⁴ is an optionally substituted C₁₋₆ alkyl or hydrogen;
      with a Grignard reagent, to thereby prepare the compound having formula (8-A). In some of these embodiments, the Grignard reagent is prepared by reacting iodomethyl pivalate with sec-butylmagnesium chloride. In some embodiments, the process further comprises a step of (iii) hydrolyzing the compound having formula (8-A) using an acid to thereby yield an amine compound of Formula (9-1): 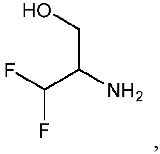 or an acid addition salt thereof.

In another aspect, provided is a process for the preparation of a compound of Formula (9-1): 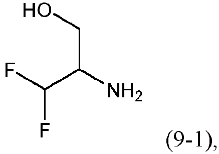 or an acid addition salt thereof;
the process comprising:
(i) reacting a compound of Formula (2A): 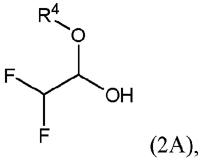 or a salt thereof;
   wherein R⁴ is an optionally substituted C₁₋₆ alkyl or hydrogen;
   with (S)-2-methylpropane-2-sulfinamide to thereby prepare (S,E)-N-(2,2-difluoroethylidene)-2-methylpropane-2-sulfinamide having structure: 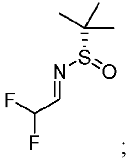
(ii) reacting (S,E)-N-(2,2-difluoroethylidene)-2-methylpropane-2-sulfinamide with trimethylsilyl-cyanide to give the aminonitrile (S)-N-((S)-1-cyano-2,2-difluoroethyl)-2-methylpropane-2-sulfinamide having the structure: 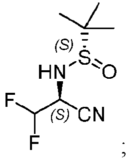
(iii) hydrolyzing (S)-N-((S)-1-cyano-2,2-difluoroethyl)-2-methylpropane-2-sulfinamide in acid to give the product (S)-2-(chloro-λ⁵-azancyl)-3,3-difluoropropanoic acid: 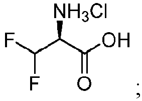 and
(iv) reducing (S)-2-(chloro-λ⁵-azaneyl)-3,3-difluoropropanoic acid to provide the intermediate compound of Formula (9-1) or the acid addition salt thereof.

Compounds of Formulae (7A) and (8A) are useful intermediates for the synthesis of 4-(difluoromethyl)oxazolidin-2-one, which is a key building block in the synthesis of a variety of compounds such as (S)-2-((2-((S)-4-(difluoromethyl)-2-oxooxazolidin-3-yl)-5,6 dihydrobenzo[f]imidazo[1,2-d][1,4]oxazepin-9-yl)amino)propanamide (inavolisib). The process of making 4-(difluoromethyl)oxazolidin-2-one is highly complex because of being a small chiral molecule with a challenging difluoromethyl group. Provided herein are efficient processes for making (S)-4-(difluoromethyl)oxazolidin-2-one with high stereo-specificity and optical purity. Also provided is a process of making inavolisib using the (S)-4-(difluoromethyl)oxazolidin-2-one product as a key intermediate.

### DETAILED DESCRIPTIONS OF THE INVENTION

### Definitions

The articles "a" and "an" are used in this disclosure to refer to one or more than one (i.e., to at least one) of the grammatical object of the article. By way of example, "an element" means one element or more than one element.

The term "and/or" is used in this disclosure to mean either "and" or "or" unless indicated otherwise. The use of the term "or" is used to mean "and/or" unless explicitly indicated to refer to alternatives only or the alternative are mutually exclusive, although the disclosure supports a definition that refers to only alternatives and "and/or."

As used herein, the term "about" is used to indicate that a value includes the standard deviation of error for the device or method being employed to determine the value. In certain embodiments, the term "about" refers to a range of values that fall within 25%, 20%, 19%, 18%, 17%, 16%, 15%, 14%, 13%, 12%, 11%, 10%, 9%, 8%, 7%, 6%, 5%, 4%, 3%, 2%, 1%, or less in either direction (greater than or less than) of a stated value, unless otherwise stated or otherwise evident from the context (e.g., where such number would exceed 100% of a possible value).

By "optional" or "optionally," it is meant that the subsequently described event or circumstance may or may not occur, and that the description includes instances where the event or circumstance occurs and instances in which it does not. For example, "optionally substituted aryl" encompasses both "aryl" and "substituted aryl" as defined herein. It will be understood by those ordinarily skilled in the art, with respect to any group containing one or more substituents, that such groups are not intended to introduce any substitution or substitution patterns that are sterically impractical, synthetically non-feasible, and/or inherently unstable.

The term "optionally substituted" unless otherwise specified means that a group may be unsubstituted or substituted by one or more (e.g., 0, 1, 2, 3, 4, or 5 or more, or any range derivable therein) of the substituents listed for that group in which said substituents may be the same or different. In an embodiment, an optionally substituted group has 1 substituent. In another embodiment, an optionally substituted group has 2 substituents. In another embodiment, an optionally substituted group has 3 substituents. In another embodiment, an optionally substituted group has 4 substituents. In another embodiment, an optionally substituted group has 5 substituents. For instance, an alkyl group that is optionally substituted can be a fully saturated alkyl chain (i.e., a pure hydrocarbon). Alternatively, the same optionally substituted alkyl group can have substituents different from hydrogen. For instance, it can, at any point along the chain be bonded to a halogen atom, a hydroxyl group, or any other substituent described herein. Thus, the term "optionally substituted" means that a given chemical moiety has the potential to contain other functional groups but does not necessarily have any further functional groups.

As used herein, "alkyl" may mean a straight chain or branched saturated chain having from 1 to 12 carbon atoms, including primary, secondary, and tertiary alkyl groups. Representative saturated alkyl groups include, but are not limited to, methyl, ethyl, n-propyl, isopropyl, 2-methyl-1-propyl, 2-methyl-2-propyl, 2-methyl-1-butyl, 3-methyl-1-butyl, 2-methyl-3-butyl, 2,2-dimethyl-1-propyl, 2-methyl-1-pentyl, 3-methyl-1-pentyl, 4-methyl-1-pentyl, 2-methyl-2-pentyl, 3-methyl-2-pentyl, 4-methyl-2-pentyl, 2,2-dimethyl-1-butyl, 3,3-dimethyl-1-butyl, 2-ethyl-1-butyl, butyl, isobutyl, t-butyl, n-pentyl, isopentyl, neopentyl, n-hexyl and the like, and longer alkyl groups, such as heptyl, and octyl and the like. An alkyl group can be unsubstituted or substituted. Alkyl groups containing three or more carbon atoms may be straight or branched. As used herein, "lower alkyl" means an alkyl having from 1 to 6 carbon atoms.

"Cycloalkyl" refers to a single saturated all carbon ring having 3 to 20 annular carbon atoms (i.e., C₃-C₂₀ cycloalkyl), for example from 3 to 15 annular atoms, for example, from 3 to 12 annular atoms. In certain embodiments, the cycloalkyl group is either monocyclic ("monocyclic cycloalkyl") or contains a fused, bridged or spiro ring system such as a bicyclic system ("bicyclic cycloalkyl") and can be saturated. "Cycloalkyl" includes ring systems where the cycloalkyl ring, as defined above, is fused with one or more cycloalkyl, cycloalkenyl, heterocyclyl, aryl or heteroaryl groups, wherein the point of attachment is on a cycloalkyl ring, and, in such instances, the number of carbon atoms recited continues to designate the number of carbons in the cycloalkyl ring containing the point of attachment. Examples of cycloalkyl groups include cyclopropyl, cyclobutyl, cyclopentyl, cyclohexyl, cycloheptyl, cyclooctyl, 2-adamantyl ( 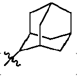 ), 2-(2,3-dihydro-1H-indene) ( 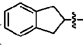 ), and 9-fluorenyl ( 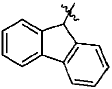 ). As noted above, cycloalkyl rings can be further characterized by the number of annular atoms. For example, a cyclohexyl ring is a C₆ cycloalkyl ring with 6 annular atoms, while 2-(2,3-dihydro-1H-indene) is a C₅ cycloalkyl ring with 9 annular atoms. Also, for example, 9-fluorenyl is a C₅ cycloalkyl ring with 13 annular atoms and 2-adamantyl is a C₆ cycloalkyl with 10 annular atoms.

The term "aryl" as used herein refers to a single all carbon aromatic ring or a multiple condensed all carbon ring system wherein at least one of the rings is aromatic. For example, in certain embodiments, an aryl group has 5 to 20 annular carbon atoms, 5 to 14 annular carbon atoms, or 5 to 12 annular carbon atoms. Aryl also includes multiple condensed ring systems (e.g., ring systems comprising 2, 3 or 4 rings) having about 9 to 20 carbon atoms in which at least one ring is aromatic and wherein the other rings may be aromatic or not aromatic (i.e., cycloalkyl). "Aryl" includes ring systems where the aryl ring, as defined above, is fused with one or more cycloalkyl, cycloalkenyl, heterocyclyl, aryl or heteroaryl groups, and wherein the point of attachment is on an aryl ring, and, in such instances, the number of carbon atoms recited continues to designate the number of carbon atoms in the aryl ring containing the point of attachment. Examples of aryl groups include phenyl, naphthyl, anthracenyl, azulenyl, and 5-(2,3-dihydro-1H-indene): 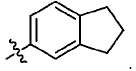 As noted above, aryl rings can be further characterized by the number of annular atoms. For example, phenyl is a C₆ aryl with 6 annular atoms, while 5-(2,3-dihydro-1H-indene) is a C₆ aryl with 9 annular atoms.

A "hydroxyl protecting group" is a chemical moiety introduced into a molecule by chemical modification of a hydroxyl group to thereby obtain chemoselectivity in a subsequent chemical reaction. Examples of hydroxyl protecting groups include, without limitation, acetyl, trimethylacetyl, benzyl: 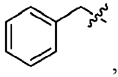 and silyl ethers including trimethylsilyl, triethylsilyl, triiso-propylsilyl, tert-butyldimethylsilyl, *tert*-butyldiphenylsilyl, and di-*tert*-butylmethylsilyl.

The term "chiral" refers to molecules which have the property of non-superimposability of the mirror image partner, while the term "achiral" refers to molecules which are superimposable on their mirror image partner.

The term "stereoisomers" refers to compounds which have identical chemical constitution but differ with regard to the arrangement of the atoms or groups in space.

"Diastereomer" refers to a stereoisomer with two or more centers of chirality and whose molecules are not mirror images of one another. Diastereomers have different physical properties, e.g. melting points, boiling points, spectral properties, and reactivities. Mixtures of diastereomers may separate under high resolution analytical procedures such as electrophoresis and chromatography.

"Enantiomers" refer to two stereoisomers of a compound which are non-superimposable mirror images of one another.

Stereochemical definitions and conventions used herein generally follow S. P. Parker, Ed., McGraw-Hill Dictionary of Chemical Terms (1984) McGraw-Hill Book Company, New York; and Eliel, E. and Wilen, S., "Stereochemistry of Organic Compounds", John Wiley & Sons, Inc., New York, 1994. The compounds of the invention may contain asymmetric or chiral centers (stereocenters), and therefore exist in different stereoisomeric forms. It is intended that all stereoisomeric forms of the compounds of the invention, including but not limited to, diastereomers, enantiomers and atropisomers, as well as mixtures thereof such as racemic mixtures, form part of the present invention. Many organic compounds exist in optically active forms, i.e., they have the ability to rotate the plane of plane-polarized light. In describing an optically active compound, the prefixes D and L, or *R* and *S*, are used to denote the absolute configuration of the molecule about its chiral center(s). The prefixes d and l or (+) and (-) are employed to designate the sign of rotation of plane-polarized light by the compound, with (-) or 1 meaning that the compound is levorotatory. A compound prefixed with (+) or d is dextrorotatory. For a given chemical structure, these stereoisomers are identical except that they are mirror images of one another. A specific stereoisomer may also be referred to as an enantiomer, and a mixture of such isomers is often called an enantiomeric mixture. A 50:50 mixture of enantiomers is referred to as a racemic mixture or a racemate, which may occur where there has been no stereoselection or stereospecificity in a chemical reaction or process. The terms "racemic mixture" and "racemate" refer to an equimolar mixture of two enantiomeric species, devoid of optical activity.

The term "tautomer" or "tautomeric form" refers to structural isomers of different energies which are interconvertible via a low energy barrier. For example, proton tautomers (also known as prototropic tautomers) include interconversions via migration of a proton, such as keto-enol and imine-enamine isomerizations. Valence tautomers include interconversions by reorganization of some of the bonding electrons.

The phrase "pharmaceutically acceptable salt" as used herein, refers to pharmaceutically acceptable organic or inorganic salts of a compound of the invention. Exemplary salts include, but are not limited, to sulfate, citrate, acetate, oxalate, chloride, bromide, iodide, nitrate, bisulfate, phosphate, acid phosphate, isonicotinate, lactate, salicylate, acid citrate, tartrate, oleate, tannate, pantothenate, bitartrate, ascorbate, succinate, maleate, gentisinate, fumarate, gluconate, glucuronate, saccharate, formate, benzoate, glutamate, methanesulfonate "mesylate", ethanesulfonate, benzenesulfonate, p-toluenesulfonate, and pamoate (i.e., 1,1'-methylene-bis-(2-hydroxy-3-naphthoate)) salts. A pharmaceutically acceptable salt may involve the inclusion of another molecule such as an acetate ion, a succinate ion or other counter ion. The counter ion may be any organic or inorganic moiety that stabilizes the charge on the parent compound. Furthermore, a pharmaceutically acceptable salt may have more than one charged atom in its structure. Instances where multiple charged atoms are part of the pharmaceutically acceptable salt can have multiple counter ions. Hence, a pharmaceutically acceptable salt can have one or more charged atoms and/or one or more counter ion.

If the compound of the invention is a base, the desired pharmaceutically acceptable salt may be prepared by any suitable method available in the art, for example, treatment of the free base with an inorganic acid, such as hydrochloric acid, hydrobromic acid, sulfuric acid, nitric acid, methanesulfonic acid, phosphoric acid and the like, or with an organic acid, such as acetic acid, maleic acid, succinic acid, mandelic acid, fumaric acid, malonic acid, pyruvic acid, oxalic acid, glycolic acid, salicylic acid, a pyranosidyl acid, such as glucuronic acid or galacturonic acid, an alpha hydroxy acid, such as citric acid or tartaric acid, an amino acid, such as aspartic acid or glutamic acid, an aromatic acid, such as benzoic acid or cinnamic acid, a sulfonic acid, such as p-toluenesulfonic acid or ethanesulfonic acid, or the like.

If the compound of the invention is an acid, the desired pharmaceutically acceptable salt may be prepared by any suitable method, for example, treatment of the free acid with an inorganic or organic base, such as an amine (primary, secondary or tertiary), an alkali metal hydroxide or alkaline earth metal hydroxide, or the like. Illustrative examples of suitable salts include, but are not limited to, organic salts derived from amino acids, such as glycine and arginine, ammonia, primary, secondary, and tertiary amines, and cyclic amines, such as piperidine, morpholine and piperazine, and inorganic salts derived from sodium, calcium, potassium, magnesium, manganese, iron, copper, zinc, aluminum and lithium.

A "solvate" refers to an association or complex of one or more solvent molecules and a compound of the invention. Examples of solvents that form solvates include, but are not limited to, water, isopropanol, ethanol, methanol, DMSO, ethyl acetate, acetic acid, and ethanolamine. The term "hydrate" refers to the complex where the solvent molecule is water.

It is intended that all stereoisomeric forms of the compounds of the invention, including but not limited to, diastereomers, enantiomers and atropisomers, as well as mixtures thereof such as racemic mixtures, form part of the present invention. In addition, the present invention embraces all geometric and positional isomers. In the structures shown herein, where the stereochemistry of any particular chiral atom is not specified, then all stereoisomers are contemplated and included as the compounds of the invention. Where stereochemistry is specified by a solid wedge or dashed line representing a particular configuration, then that stereoisomer is so specified and defined.

The compounds of the invention may exist in unsolvated as well as solvated forms with pharmaceutically acceptable solvents such as water, ethanol, and the like, and it is intended that the invention embrace both solvated and unsolvated forms.

The compounds of the invention may also exist in different tautomeric forms, and all such forms are embraced within the scope of the invention. The term "tautomer" or "tautomeric form" refers to structural isomers of different energies which are interconvertible via a low energy barrier. For example, proton tautomers (also known as prototropic tautomers) include interconversions via migration of a proton, such as keto-enol and imine-enamine isomerizations. Valence tautomers include interconversions by reorganization of some of the bonding electrons.

The compounds of the invention also include isotopically-labeled compounds which are identical to those recited herein, but for the fact that one or more atoms are replaced by an atom having an atomic mass or mass number different from the atomic mass or mass number usually found in nature. All isotopes of any particular atom or element as specified are contemplated within the scope of the compounds of the invention, and their uses. Exemplary isotopes that can be incorporated into compounds of the invention include isotopes of hydrogen, carbon, nitrogen, oxygen, phosphorus, sulfur, fluorine, chlorine and iodine, such as ²H, ³H, ¹¹C, ¹³C, ¹⁴C, ¹³N, ¹⁵N, ¹⁵O, ¹⁷O, ¹⁸O, ³²P, ³³P, ³⁵S, ¹⁸F, ³⁶Cl, ¹²³I and ¹²⁵I. Certain isotopically-labeled compounds of the present invention (e.g., those labeled with ³H and ¹⁴C) are useful in compound and/or substrate tissue distribution assays. Tritiated (³H) and carbon-14 (¹⁴C) isotopes are useful for their ease of preparation and detectability. Further, substitution with heavier isotopes such as deuterium (i.e., ²H) may afford certain therapeutic advantages resulting from greater metabolic stability (e.g., increased in vivo half-life or reduced dosage requirements) and hence may be preferred in some circumstances. Positron emitting isotopes such as ¹⁵O, ¹³N, ¹¹C and ¹⁸F are useful for positron emission tomography (PET) studies to examine substrate receptor occupancy. Isotopically labeled compounds of the present invention can generally be prepared by following procedures analogous to those disclosed in the Examples herein below, by substituting an isotopically labeled reagent for a non-isotopically labeled reagent.

### Intermediates useful in the preparation of benzoxazepin oxazolidinone compounds

In some embodiments, the present invention is directed to intermediates suitable for use in the preparation of benzoxazepin oxazolidinone compounds.

In some embodiments, the intermediate is a compound of Formula (8A): 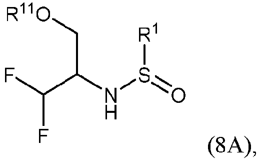 or a salt thereof,
wherein:
R¹ is an optionally substituted C₁₋₁₂ alkyl, an optionally substituted C₃₋₁₄ cycloalkyl, or an optionally substituted C₆₋₁₄ aryl; and
R¹¹ is hydrogen or a hydroxyl protecting group.

In some embodiments, R¹ is the optionally substituted C₁₋₁₂ alkyl. Alkyl groups include methyl, ethyl, n-propyl, isopropyl, 2-methyl-1-propyl, 2-methyl-2-propyl, 2-methyl-1-butyl, 3-methyl-1-butyl, 2-methyl-3-butyl, 2,2-dimethyl-1-propyl, 2-methyl-1-pentyl, 3-methyl-1-pentyl, 4-methyl-1-pentyl, 2-methyl-2-pentyl, 3-methyl-2-pentyl, 4-methyl-2-pentyl, 2,2-dimethyl-1-butyl, 3,3-dimethyl-1-butyl, 2-ethyl-1-butyl, butyl, isobutyl, t-butyl, n-pentyl, isopentyl, neopentyl, n-hexyl and the like, and longer alkyl groups, such as heptyl, and octyl and the like. In some embodiments, R¹ is an optionally substituted tertiary C₄₋₁₂ alkyl. In some embodiments, the tertiary C₄₋₁₂ alkyl may be selected from among tert-butyl, tert-pentyl, 2,3-dimethylbutyl, 3-ethylpentan-3-yl, 3-ethylpentan-2-yl, and others. In some embodiments, R¹ is selected from the group consisting of tert-butyl, tert-pentyl, 3-ethylpentan-3-yl, 1-methylcyclohexyl, 1-adamantyl, phenyl, and naphthyl. In some embodiments, R¹ is tert-butyl.

In some embodiments, R¹¹ is hydrogen. In some embodiments, R¹¹ is a hydroxyl protecting group selected from the group consisting of optionally substituted acetyl, trimethylacetyl, benzyl, and silyl ethers including trimethylsilyl, triethylsilyl, triiso-propylsilyl, tert-butyldimethylsilyl, tert-butyldiphenylsilyl, and di-tert-butylmethylsilyl. In some embodiments, R¹¹ is benzyl.

In some embodiments, the intermediate is a compound of Formula (8B): 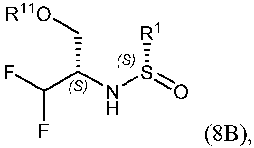 or a salt thereof,
wherein R¹ and R¹¹ are as defined above in connection with the compound of Formula (8A).

In some embodiments, the intermediate is a compound of Formula (8C): 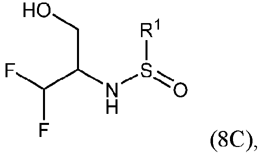 or a salt thereof,
wherein R¹ is as defined above in connection with the compound of Formula (8A).

In some embodiments, the intermediate is a compound of Formula (8D): 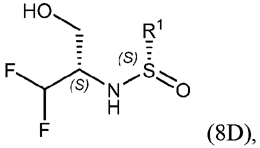 or a salt thereof,
wherein R¹ is as defined above in connection with the compound of Formula (8A).

In some embodiments, the intermediate is a compound of Formula (8-1) or Formula (8-2): 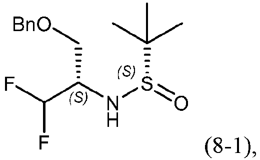 or a salt thereof; or 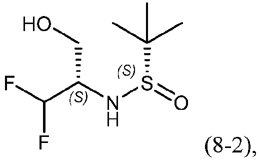 or a salt thereof.

In some embodiments, the intermediate is a compound of Formula (7A): 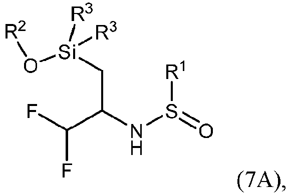 or a salt thereof,
wherein:
R¹ is an optionally substituted C₁₋₁₂ alkyl, an optionally substituted C₃₋₁₄ cycloalkyl, or an optionally substituted C₆₋₁₄ aryl;
R² is an optionally substituted C₁₋₁₂ alkyl or an optionally substituted C₆₋₁₄ aryl; and
R³ is an optionally substituted C₁₋₁₂ alkyl, an optionally substituted C₆₋₁₄ aryl, or OR².

In some embodiments, R¹ is the optionally substituted C₁₋₁₂ alkyl. In some embodiments, R¹ is selected from the group consisting of tert-butyl, tert-pentyl, 3-ethylpentan-3-yl, 1-methylcyclohexyl, 1-adamantyl, phenyl, and naphthyl. In some embodiments, R¹ is tert-butyl.

In some embodiments, R² is the optionally substituted C₁₋₁₂ alkyl. Alkyl groups include methyl, ethyl, n-propyl, isopropyl, 2-methyl-1-propyl, 2-methyl-2-propyl, 2-methyl-1-butyl, 3-methyl-1-butyl, 2-methyl-3-butyl, 2,2-dimethyl-1-propyl, 2-methyl-1-pentyl, 3-methyl-1-pentyl, 4-methyl-1-pentyl, 2-methyl-2-pentyl, 3-methyl-2-pentyl, 4-methyl-2-pentyl, 2,2-dimethyl-1-butyl, 3,3-dimethyl-1-butyl, 2-ethyl-1-butyl, butyl, isobutyl, t-butyl, n-pentyl, isopentyl, neopentyl, n-hexyl and the like, and longer alkyl groups, such as heptyl, and octyl and the like. In some embodiments, R² is an optionally substituted secondary C₃₋₁₂ alkyl. In some embodiments, R² is an optionally substituted tertiary C₄₋₁₂ alkyl. In some embodiments, R² is selected from among methyl, ethyl, n-propyl, isopropyl, n-butyl, sec-butyl, tert-butyl among others. In some embodiments, R² is isopropyl.

In some embodiments, R¹ is independently optionally substituted C₁₋₁₂ alkyl. Alkyl groups include methyl, ethyl, n-propyl, isopropyl, 2-methyl-1-propyl, 2-methyl-2-propyl, 2-methyl-1-butyl, 3-methyl-1-butyl, 2-methyl-3-butyl, 2,2-dimethyl-1-propyl, 2-methyl-1-pentyl, 3-methyl-1-pentyl, 4-methyl-1-pentyl, 2-methyl-2-pentyl, 3-methyl-2-pentyl, 4-methyl-2-pentyl, 2,2-dimethyl-1-butyl, 3,3-dimethyl-1-butyl, 2-ethyl-1-butyl, butyl, isobutyl, t-butyl, n-pentyl, isopentyl, neopentyl, n-hexyl and the like, and longer alkyl groups, such as heptyl, and octyl and the like. In some embodiments, R³ is an optionally substituted secondary C₃₋₁₂ alkyl. In some embodiments, R³ is an optionally substituted tertiary C₄₋₁₂ alkyl. In some embodiments, R³ is independently selected from among methyl, ethyl, n-propyl, isopropyl, n-butyl, sec-butyl, tert-butyl, among others. In some embodiments, each R³ is methyl.

In some embodiments, the intermediate is a compound of Formula (7B): 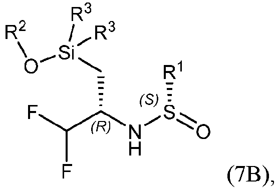 or a salt thereof,
wherein R¹, R², and R³ are as defined above in connection with the compound of Formula (7A).

In some embodiments, the intermediate is a compound of Formula (7): 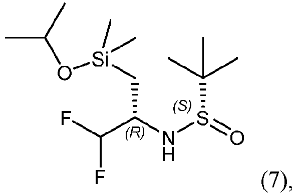 or a salt thereof.

### Preparation of intermediates useful in the preparation of benzoxazepin oxazolidinone compounds

In some embodiments, the present invention is directed to methods of preparing intermediates suitable for use in the preparation of benzoxazepin oxazolidinone compounds.

The intermediates prepared according to the methods disclosed herein are useful in preparing compounds having the structures (10-1) and (10-2):

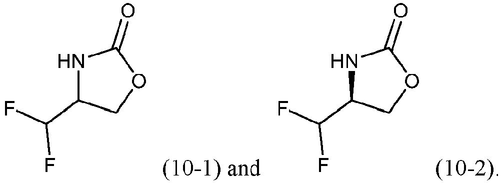

Compounds having the structures (10-1) and (10-2) are useful intermediates in the preparation of benzoxazepin oxazolidinone compounds.

### 1. The Grignard- Tamao Route

In some embodiments, the process is for preparing an intermediate compound of Formula (8C): 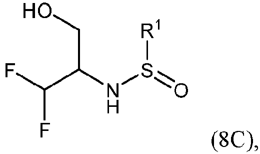 or a salt thereof,
wherein R¹ is as defined above in connection with the compound of Formula (8A).

In some embodiments, the process is for preparing an intermediate compound of Formula (8D): 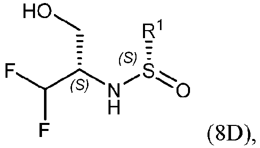 or a salt thereof,
wherein R¹ is as defined above in connection with the compound of Formula (8A).

In some embodiments, the process is for preparing an intermediate compound of Formula (8-2): 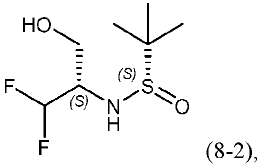 or a salt thereof.

The process comprises a step of (i) partially reducing a compound of Formula (1A): 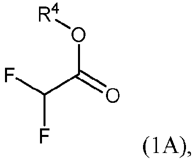 or a salt thereof,
wherein R⁴ is an optionally substituted C₁₋₆ alkyl or hydrogen, to form a compound of Formula (2A): 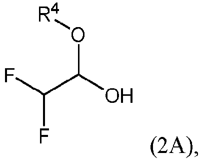 or a salt thereof.

In some embodiments, R⁴ is an optionally substituted C₁₋₆ alkyl. The optionally substituted C₁₋₆ alkyl is selected from among methyl, ethyl, n-propyl, isopropyl, 2-methyl-1-propyl, 2-methyl-2-propyl, 2-methyl-1-butyl, 3-methyl-1-butyl, 2-methyl-3-butyl, 2,2-dimethyl-1-propyl, 2-methyl-1-pentyl, 3-methyl-1-pentyl, 4-methyl-1-pentyl, 2-methyl-2-pentyl, 3-methyl-2-pentyl, 4-methyl-2-pentyl, 2,2-dimethyl-1-butyl, 3,3-dimethyl-1-butyl, 2-ethyl-1-butyl, butyl, isobutyl, t-butyl, n-pentyl, isopentyl, neopentyl, n-hexyl and the like. In some embodiments, R⁴ is ethyl.

In some embodiments, the compound of Formula (1A) is a compound of Formula (1), and the compound of Formula (2A) is a compound of Formula (2): 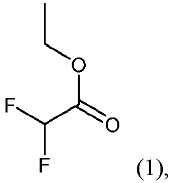 or a salt thereof and 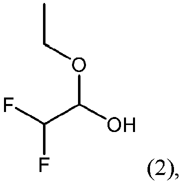 or a salt thereof.

The reducing agent may be selected from among Red-Al (sodium bis(2-methoxyethoxy)aluminum hydride), lithium aluminum hydride (LAH), lithium *tri-tert-*butoxyaluminum hydride, and diisobutylaluminum hydride (DIBAL). This reaction may occur in a suitable solvent, such as methyl tert-butyl ether, cyclopentyl methyl ether, diethylether, diisoproylether, tetrahydrofuran, 2-methyltetrahydrofuran, 1,4-dioxane, dimethoxyethane (glyme), 1-methoxy-2-(2-methoxyethoxy)ethane (diglyme), diethoxyethane, toluene, anisole, dichloromethane, dichloroethane, hexanes, heptane. The reducing reaction preferably occurs at a lower temperature, such as less than about 20°C, or such as between about 0°C to about 10°C. The hemiacetal of Formula (2A) may be obtained in a solution in the organic solvent. Isolation of the hemiacetal is optional and not necessarily required.

The process further comprises a step of (ii) reacting the compound of Formula (2A) with a sulfonamide compound of Formula (3A): 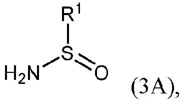 wherein R¹ is an optionally substituted C₁₋₁₂ alkyl, an optionally substituted C₃₋₁₄ cycloalkyl, or an optionally substituted C₆₋₁₄ aryl, in the presence of a dehydrating reagent to form the compound of Formula (4A): 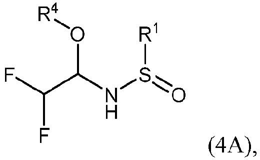 or a salt thereof.

In some embodiments, R¹ is the optionally substituted C₁₋₁₂ alkyl. In some embodiments, R¹ is selected from the group consisting of tert-butyl, tert-pentyl, 3-ethylpentan-3-yl, 1-methylcyclohexyl, 1-adamantyl, phenyl, and naphthyl. In some embodiments, R¹ is *tert*-butyl.

In some embodiments, R⁴ is an optionally substituted C₁₋₆ alkyl selected from among methyl, ethyl, n-propyl, isopropyl, n-butyl, sec-butyl, tert-butyl, among others. In some embodiments, R⁴ is ethyl.

In some embodiments, the compound of Formula (3A) is of Formula (3B):

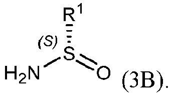

In some embodiments, the compound of Formula (3A) is of Formula (3):

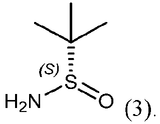

In some embodiments, the compound of Formula (4A) is of Formula (4B): 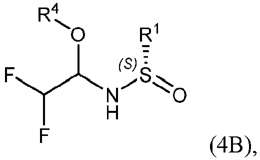 or a salt thereof.

In some embodiments, the compound of Formula (4A) is of Formula (4): 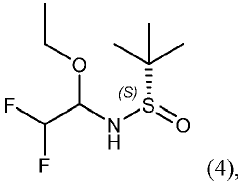 or a salt thereof.

The dehydrating agent may be a titanium alkoxide having the general formula Ti(OR)₄, wherein R is a C₁₋₆ alkyl group. In some embodiments, R is ethyl, and the dehydrating agent is Ti(OCH₂CH₃)₄. Additional suitable dehydrating agents include magnesium sulfate, copper sulfate, molecular sieves, triisopropylborate, tetramethyl orthosilicate, tetraethyl orthosilicate, and bis(trimethylsilyl)acetamide. The reaction may occur by adding the sulfonamide compound of Formula (3A) and the dehydrating agent to the solution comprising the hemiacetal of Formula (2A). The reaction occurs at an elevated temperature, such as at least about 50°C, or at least about 70°C, such as between about 80°C to about 90°C or at about 85°C.

The process further comprises a step of (iii) reacting a compound of Formula (4A): 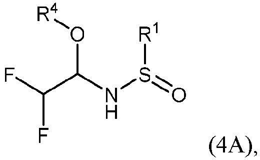 or a salt thereof,
with a Grignard reagent of formula (5A): 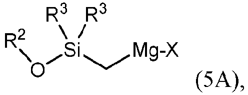 to thereby form a compound of Formula (7A): 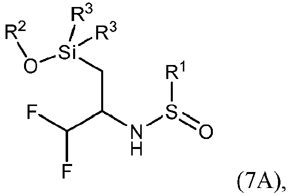 or a salt thereof.

In some embodiments, R² is an optionally substituted C₁₋₁₂ alkyl or an optionally substituted C₆₋₁₄ aryl. In some embodiments, R² is the optionally substituted C₁₋₁₂ alkyl. In some embodiments, R² is an optionally substituted secondary C₃₋₁₂ alkyl. In some embodiments, R² is an optionally substituted tertiary C₄₋₁₂ alkyl. In some embodiments, R² is selected from among methyl, ethyl, n-propyl, isopropyl, n-butyl, sec-butyl, tert-butyl among others. In some embodiments, R² is isopropyl.

In some embodiments, R³ is an optionally substituted C₁₋₁₂ alkyl, an optionally substituted C₆₋₁₄ aryl, or OR². In some embodiments, R¹ is optionally substituted C₁₋₁₂ alkyl. In some embodiments, R³ is an optionally substituted secondary C₃₋₁₂ alkyl. In some embodiments, R³ is an optionally substituted tertiary C₄₋₁₂ alkyl. In some embodiments, R³ is selected from among methyl, ethyl, n-propyl, isopropyl, n-butyl, sec-butyl, tert-butyl, among others. In some embodiments, R³ is methyl.

In some embodiments, X is a halide, such as chloride, bromide, or iodide. In some embodiments, X is chloride.

The Grignard reagent of formula (5A) may be prepared in situ by reacting the corresponding alkyl halide of formula (5'A): 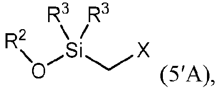 with magnesium. The reaction may be initiated by adding a small amount of an initiator such as 1,2-dibromoethane.

In some embodiments, the compound of Formula (5A) is a compound of formula (5) and the compound of Formula (5'A) is a compound of formula (5'):

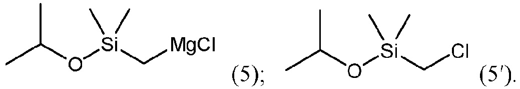

In some embodiments, the compound of Formula (7A) is a compound of Formula (7B): 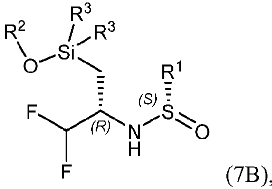 or a salt thereof.

In some embodiments, the compound of Formula (7A) is a compound of Formula (7): 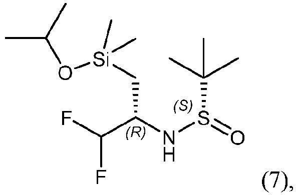 or a salt thereof.

The Grignard reaction may occur in a suitable solvent, such as methyl tert-butyl ether, cyclopentyl methyl ether, diethylether, diisoproylether, tetrahydrofuran, 2-methyltetrahydrofuran, 1,4-dioxane, dimethoxyethane (glyme), 1-methoxy-2-(2-methoxyethoxy)ethane (diglyme), diethoxyethane, toluene, anisole, hexanes, and n-heptane. The reaction occurs at a lower temperature, such as less than about 20°C, or less than 10°C, such as between about -40°C to about 0°C, or such as about -25°C. The first equivalent of Grignard reagent eliminates ethanol from the compound of Formula (4A) to release the corresponding imine. Without being held to any particular theory, according to Ellman and co-workers (J. Am. Chem. Soc. 1997, 119, 9913-9914.) the reaction proceeds through a six membered transition state 6 which induces high stereo-control:

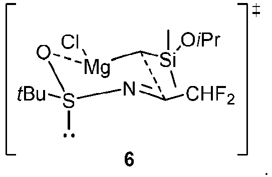

The reaction proceeds by adding a molar excess of the Grignard reagent compared to the compound of Formula (4A), such as a molar ratio of at least about 1.1:1, at least about 1.2:1, or at least about 1.5:1, or at least about 2:1, such as about 2.2:1. The reaction proceeds to prepare the compound of Formula (7A) with high stereo-control, such as at least about 80:20 in favor of the (R),(S) configuration, or at least about 90:10, or even at least about 75:5, such as about 97:3 or about 94:6.

The process further comprises a step of (iv) reacting the compound of Formula (7A) or a salt thereof with a fluoride salt, a base, and an oxidant to form the compound of Formula (8C) or a salt thereof. The reaction proceeds by Fleming-Tamao oxidation. (See, e.g., Org. Process Res. Dev. 2014, 18, 66-81.) Suitable fluoride salts include sodium fluoride, potassium fluoride, and potassium bifluoride. Suitable bases include sodium bicarbonate, potassium bicarbonate, disodium phosphate, and potassium hydroxide. Suitable oxidants include hydrogen peroxide and meta-chloroperoxybenzoic acid (mCPBA). This reaction may occur in a suitable solvent, such as tetrahydrofuran, dimethylformamide, methanol, ethanol, and 1-propanol. In some embodiments, the solvent is methanol. In some embodiments, the reaction occurs at an elevated temperature, such as at least about 30°C, such as between about 40°C and about 50°C or at about 45°C.

In some embodiments of the process, in the step of (iv), the compound of Formula (7A) may be converted to a compound of formula (7' A): 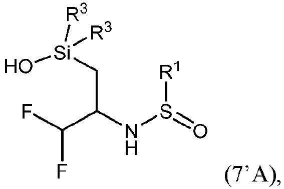 wherein R¹ and R³ are as defined in Formula (7A),
before contacting a fluoride salt, a base, and an oxidant to form the compound of Formula (8C) or a salt thereof. The reaction may occur in a biphasic system wherein an organic phase (e.g., in THF) is mixed with an aqueous phase. In such case, a biphasic catalyst (such as tetrabutylammonium hydrogen sulfate) may be used.

The process further comprises a step of (v) reacting the compound of Formula (8C): 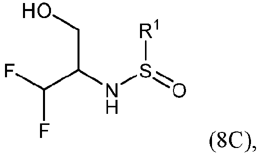 or a salt thereof,
with an acid to thereby yield an amine compound of Formula (9-1): 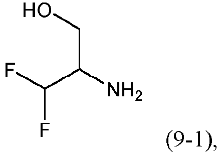 or an acid addition salt thereof.

Suitable acids include hydrogen halides, such as hydrogen bromide, hydrogen chloride, and hydrogen iodide. Additional suitable acids include trifluoroacetic acid, sulfonic acid, methanesulfonic acid, benzenesulfonic acid, and *p*-toluenesulfonic acid. In some embodiments, the acid for step (v) is HCl, and the acid addition salt of the compound of Formula (9-1) is a hydrochloride salt having the structure (9-2):

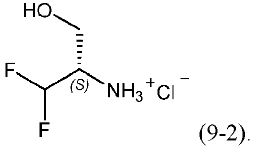

In some embodiments, the compound of Formula (9-1) is a compound of Formula (9-3):

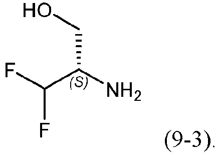

This reaction may occur in a suitable solvent, such as methyl tert-butyl ether, tetrahydrofuran, 1,4-dioxanemethanol, methanol, ethanol, 1-propanol, and 2-propanol. The reaction may occur at temperatures between about 15°C and about 25°C.

The process further comprises a step of (vi) reacting the compound of Formula (9-1), or the acid addition salt thereof having structure (9-3), with an acylating reagent to form a compound of Formula (10-1):

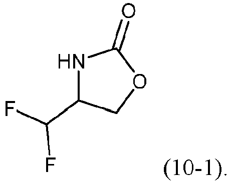

In some embodiments, the compound of Formula (10-1) is a compound of Formula (10-2):

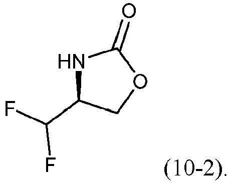

The reaction may occur in the presence of a base to release the free amine followed by addition of the acylating agent. The acylating reagent may be selected from among 1,1'-carbonyldiimidazole (CDI), phosgene, diphosgene, triphosgene, bis(2,2,2-trifluoroethyl) carbonate, bis(2,5-dioxopyrrolidin-1-yl) carbonate, 4-nitrophenyl chloroformate, di(pyridin-2-yl) carbonate, and diphenyl carbonate. The base may be selected from sodium carbonate, sodium bicarbonate, potassium carbonate, potassium bicarbonate, tripotassium phosphate, dipotassium hydrogen phosphate, diisopropylethylamine (DIPEA), triethylamine, N-methylmorpholine, and pyridine. The reaction may occur at temperatures between about 10°C and about 35°C. This reaction may occur in a suitable solvent, such as methyl tert-butyl ether, toluene, tetrahydrofuran, 2-methyltetrahydrofuran, N-methyl-2-pyrrolidone, acetonitrile, dimethylformamide, dichloromethane, methanol, ethanol, trifluoroethanol, and 1-propanol.

In some embodiments, the process of making a compound of formula (10-2) is according to the following sequence of steps:

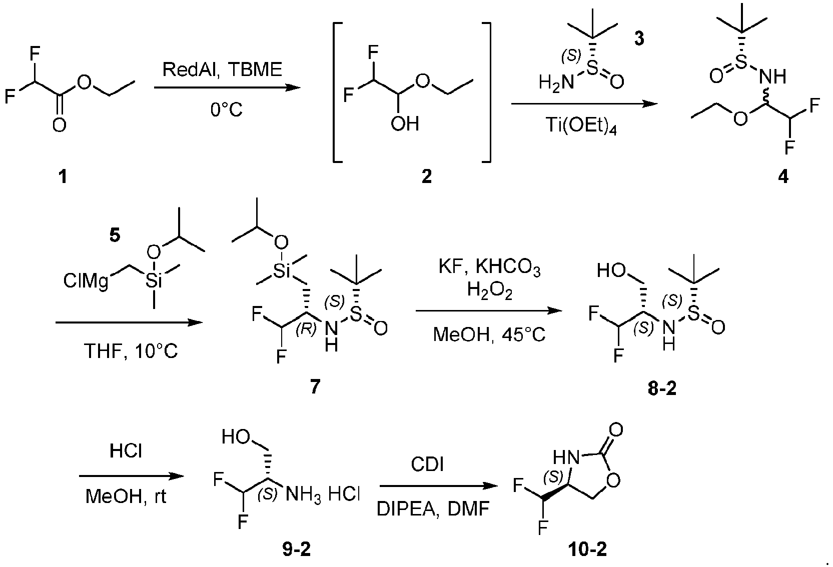

In some embodiments, the process of making a compound of formula (10-2) is according to the following sequence of steps:

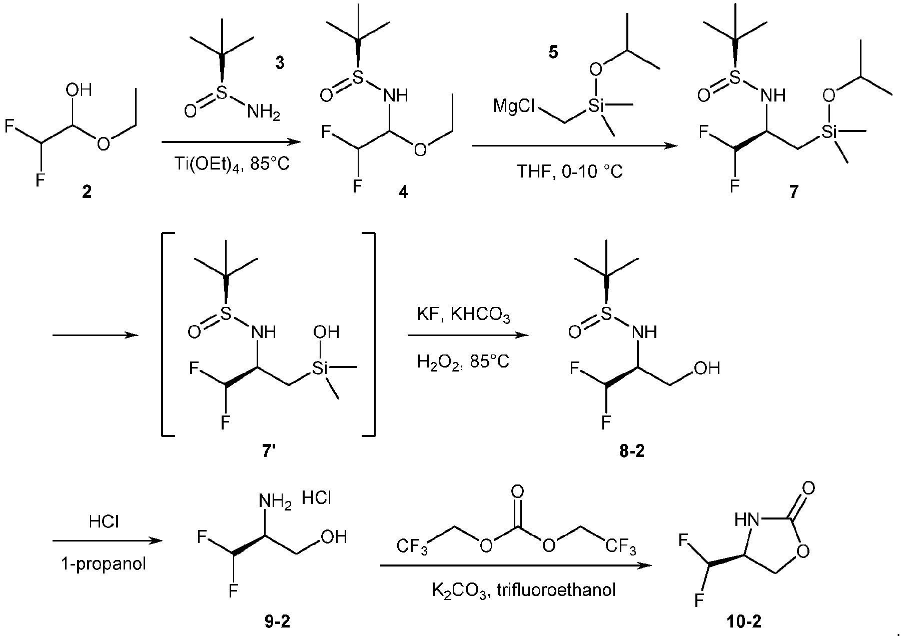

The Grignard-Tamao route provides a safe, short, highly robust and cost effective process with increased yields, decreased infavorable /hazardous reactants/solvents, easy work up and is easy to be up-scaled.

### 2. The Grignard-Knochel Route

In some embodiments, the process is for preparing an intermediate compound of Formula (8A): 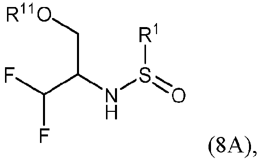 or a salt thereof, wherein:
R¹ is an optionally substituted C₁₋₁₂ alkyl, an optionally substituted C₃₋₁₄ cycloalkyl, or an optionally substituted C₆₋₁₄ aryl; and
R¹¹ is hydrogen or a hydroxyl protecting group.

In some embodiments, R¹ is the optionally substituted C₁₋₁₂ alkyl. In some embodiments, R¹ is an optionally substituted tertiary C₄₋₁₂ alkyl. In some embodiments, the tertiary C₄₋₁₂ alkyl may be selected from among *tert*-butyl*,* tert-pentyl, 2,3-dimethylbutyl, 3-ethylpentan-3-yl, 3-ethylpentan-2-yl, and others. In some embodiments, R¹ is selected from the group consisting of *tert*-butyl, tert-pentyl, 3-ethylpentan-3-yl, 1-methylcyclohexyl, 1-adamantyl, phenyl, and naphthyl. In some embodiments, R¹ is *tert*-butyl.

In some embodiments, R¹¹ is the hydroxyl protecting group selected from the group consisting of an optionally substituted acetyl, trimethylacetyl, benzyl, and silyl ethers including trimethylsilyl, triethylsilyl, triiso-propylsilyl, *tert*-butyldimethylsilyl, *tert*-butyldiphenylsilyl, and di-*tert*-butylmethylsilyl. In some embodiments, R¹¹ is optionally substituted acetyl (e.g., pivalyl).

In some embodiments, the process is for preparing an intermediate compound of Formula (8B): 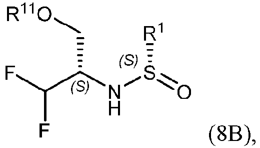 or a salt thereof,
wherein R¹ and R¹¹ are as defined above in connection with the compound of Formula (8A).

In some embodiments, the process is for preparing an intermediate compound of Formula (8-3): 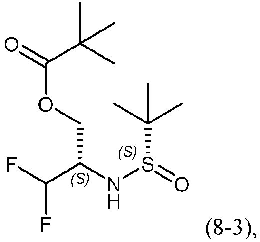 or a salt thereof.

In some embodiments, the process includes a step (i) of preparing a compound of Formula (4A): 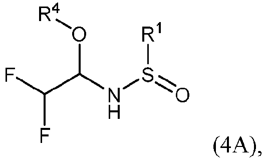 or a salt thereof, wherein:
R¹ is an optionally substituted C₁₋₁₂ alkyl, an optionally substituted C₃₋₁₄ cycloalkyl, or an optionally substituted C₆₋₁₄ aryl; and
R⁴ is an optionally substituted C₁₋₆ alkyl or hydrogen.

In some embodiments, R¹ is the optionally substituted C₁₋₁₂ alkyl. Alkyl groups include methyl, ethyl, n-propyl, isopropyl, 2-methyl-1-propyl, 2-methyl-2-propyl, 2-methyl-1-butyl, 3-methyl-1-butyl, 2-methyl-3-butyl, 2,2-dimethyl-1-propyl, 2-methyl-1-pentyl, 3-methyl-1-pentyl, 4-methyl-1-pentyl, 2-methyl-2-pentyl, 3-methyl-2-pentyl, 4-methyl-2-pentyl, 2,2-dimethyl-1-butyl, 3,3-dimethyl-1-butyl, 2-ethyl-1-butyl, butyl, isobutyl, t-butyl, n-pentyl, isopentyl, neopentyl, n-hexyl and the like, and longer alkyl groups, such as heptyl, and octyl and the like. In some embodiments, R¹ is an optionally substituted tertiary C₄₋₁₂ alkyl. In some embodiments, the tertiary C₄₋₁₂ alkyl may be selected from among *tert*-butyl, tert-pentyl, 2,3-dimethylbutyl, 3-ethylpentan-3-yl, 3-ethylpentan-2-yl, and others. In some embodiments, R¹ is selected from the group consisting of *tert*-butyl, tert-pentyl, 3-ethylpentan-3-yl, 1-methylcyclohexyl, 1-adamantyl, phenyl, and naphthyl. In some embodiments, R¹ is *tert*-butyl.

In some embodiments, R⁴ is an optionally substituted C₁₋₆ alkyl. The optionally substituted C₁₋₆ alkyl is selected from among methyl, ethyl, n-propyl, isopropyl, 2-methyl-1-propyl, 2-methyl-2-propyl, 2-methyl-1-butyl, 3-methyl-1-butyl, 2-methyl-3-butyl, 2,2-dimethyl-1-propyl, 2-methyl-1-pentyl, 3-methyl-1-pentyl, 4-methyl-1-pentyl, 2-methyl-2-pentyl, 3-methyl-2-pentyl, 4-methyl-2-pentyl, 2,2-dimethyl-1-butyl, 3,3-dimethyl-1-butyl, 2-ethyl-1-butyl, butyl, isobutyl, t-butyl, n-pentyl, isopentyl, neopentyl, n-hexyl and the like. In some embodiments, R⁴ is ethyl.

In some embodiments, the step (i) comprises reacting a compound of Formula (2A): 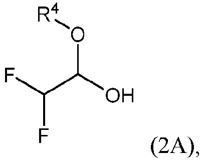 or a salt thereof, with a sulfonamide compound of Formula (3A): 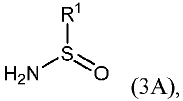 or a salt thereof, in the presence of a dehydrating reagent to form the compound of Formula (4A).

In some embodiments, the compound of Formula (2A) is a compound of Formula (2): 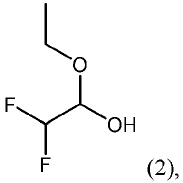 or a salt thereof.

In some embodiments, the compound of Formula (3A) is of Formula (3B):

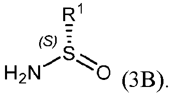

In some embodiments, the compound of Formula (3A) is of Formula (3):

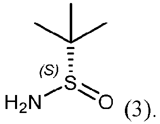

In some embodiments, the compound of Formula (4A) is of Formula (4B): 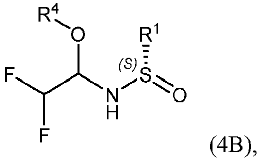 or a salt thereof.

In some embodiments, the compound of Formula (4A) is of Formula (4): 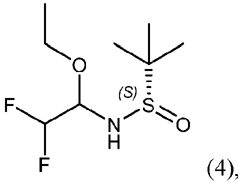 or a salt thereof.

The dehydrating agent may be a titanium alkoxide having the general formula Ti(OR)₄, wherein R is a C₁₋₆ alkyl group. In some embodiments, R is ethyl, and the dehydrating agent is Ti(OCH₂CH₃)₄. Additional suitable dehydrating agents include magnesium sulfate, copper sulfate, molecular sieves, triisopropylborate, tetramethyl orthosilicate, tetraethyl orthosilicate, and bis(trimethylsilyl)acetamide. The reaction may occur by adding the sulfonamide compound of Formula (3A) and the dehydrating agent to the solution comprising the hemiacetal of Formula (2A). The reaction occurs at an elevated temperature, such as at least about 50°C, or at least about 70°C, such as between about 80°C to about 90°C.

In some embodiments, the process includes a step (ii) of reacting the compound of Formula (4A): 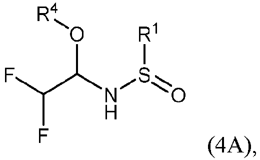 or a salt thereof,
with a Grignard reagent. The Grignard reaction may be prepared in situ. This reaction may occur in a suitable solvent, such as methyl tert-butyl ether, cyclopentyl methyl ether, diethylether, diisoproylether, tetrahydrofuran, 2-methyltetrahydrofuran, 1,4-dioxane, dimethoxyethane (glyme), 1-methoxy-2-(2-methoxyethoxy)ethane (diglyme), diethoxyethane, toluene, anisole, hexanes, and n-heptane. The Grignard reagent may be prepared by reacting iodomethyl pivalate with sec-butylmagnesium chloride, each having the structures shown below:

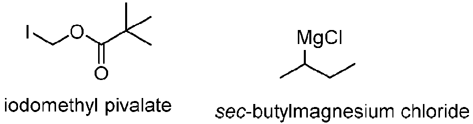

The reaction occurs by adding iodomethyl pivalate with sec-butylmagnesium chloride to a solution comprising a compound of Formula (4A). The iodomethyl pivalate and sec-butylmagnesium chloride are added in molar excess compared to the compound of Formula (4A), such as at least about 1.1:1, at least about 1.2:1, or at least about 1.5:1, or at least about 2:1, such as about 2.2:1. The reaction occurs at a low temperature, such as less than about -25°C, or less than about -35°C, or less than about -45°C, or less than about -55°C, such as about -65°C. The preparation of the Grignard-Reagent is done according to a protocol reported by Knochel (Synlett, (11), 1820-1822; 1999).

The product of the reaction is a compound of formula (8-3), which is hydrolyzed in a step (iii) with an acid to thereby yield an amine compound of Formula (9-1): 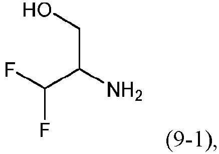 or an acid addition salt thereof.

Suitable acids include hydrogen halides, such as hydrogen bromide, hydrogen chloride, and hydrogen iodide. Additional suitable acids include trifluoroacetic acid, sulfonic acid, methanesulfonic acid, benzenesulfonic acid, and *p*-toluenesulfonic acid. In some embodiments, the acid for step (v) is HCl, and the acid addition salt of the compound of Formula (9-1) is a hydrochloride salt having the structure (9-2):

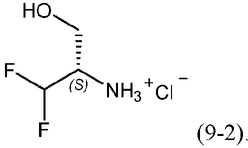

This reaction may occur in a suitable solvent, such as 1,4-dioxane, methanol, ethanol, 1-propanol, and 2-propanol. The reaction may occur at temperatures between about 15°C and about 25°C.

The process comprises a step (iv) of reacting the compound of Formula (9-1), or the acid addition salt thereof having structure (9-2), with an acylating reagent to form a compound of Formula (10-1):

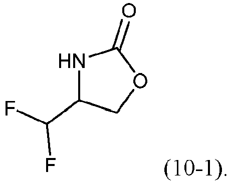

In some embodiments, the compound of Formula (10-1) is of Formula (10-2):

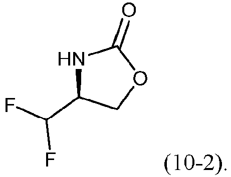

The reaction may occur in the presence of a base to release the free amine followed by addition of the acylating agent. The acylating reagent may be selected from among 1,1'-carbonyldiimidazole (CDI), phosgene, diphosgene, triphosgene, bis(2,2,2-trifluoroethyl) carbonate, bis(2,5-dioxopyrrolidin-1-yl) carbonate, 4-nitrophenyl chloroformate, di(pyridin-2-yl) carbonate, and diphenyl carbonate. The base may be selected from sodium carbonate, sodium bicarbonate, potassium carbonate, potassium bicarbonate, tripotassium phosphate, dipotassium hydrogen phosphate, diisopropylethylamine (DIPEA), triethylamine, N-methylmorpholine, and pyridine. The reaction may occur at temperatures between about 10°C and about 35°C. This reaction may occur in a suitable solvent, such as methyl tert-butyl ether, toluene, tetrahydrofuran, 2-methyltetrahydrofuran, N-methyl-2-pyrrolidone, acetonitrile, dimethylformamide, dichloromethane, methanol, ethanol, trifluoroethanol, and 1-propanol.

### 3. The Strecker Route

In some embodiments, the process is for preparing an intermediate compound of Formula (9-1): 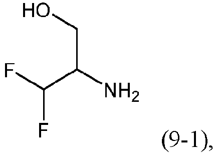 or an acid addition salt thereof.

In some embodiments, the process is for preparing an intermediate compound of Formula (9-3): 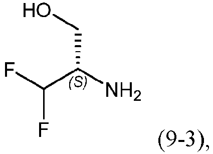 or an acid addition salt thereof.

In some embodiments, the process includes a step (i) of reacting a compound of Formula (2A): 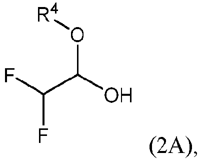 or a salt thereof;
with (S)-2-methylpropane-2-sulfinamide (Ellman's auxiliary) to thereby prepare (S,E)-N-(2,2-difluoroethylidene)-2-methylpropane-2-sulfinamide having structure:

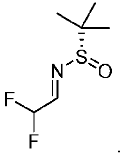

In some embodiments, R⁴ is an optionally substituted C₁₋₆ alkyl selected from among methyl, ethyl, n-propyl, isopropyl, 2-methyl-1-propyl, 2-methyl-2-propyl, 2-methyl-1-butyl, 3-methyl-1-butyl, 2-methyl-3-butyl, 2,2-dimethyl-1-propyl, 2-methyl-1-pentyl, 3-methyl-1-pentyl, 4-methyl-1-pentyl, 2-methyl-2-pentyl, 3-methyl-2-pentyl, 4-methyl-2-pentyl, 2,2-dimethyl-1-butyl, 3,3-dimethyl-1-butyl, 2-ethyl-1-butyl, butyl, isobutyl, t-butyl, n-pentyl, isopentyl, neopentyl, n-hexyl and the like. In some embodiments, R⁴ is ethyl. This reaction may occur in a suitable solvent, such as methyl tert-butyl ether, toluene, tetrahydrofuran, 2-methyltetrahydrofuran, and dichloromethane. The reactants may be refluxed in a Dean-Stark distillation apparatus.

In some embodiments, the reaction comprises a step (ii) Strecker reaction of (S,E)-N-(2,2-difluoroethylidene)-2-methylpropane-2-sulfinamide with trimethylsilyl-cyanide to give the aminonitrile (S)-N-((S)-1-cyano-2,2-difluoroethyl)-2-methylpropane-2-sulfinamide having the structure:

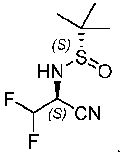

The reaction occurs in the presence of a Lewis acid, suitably scandium triflate, Yttrium triflate and trimethylsilyl triflate. This reaction may occur in a suitable solvent, such as methyl tert-butyl ether, toluene, tetrahydrofuran, acetonitrile, and dichloromethane. The reaction proceeds with high stereo-control, such as at least about 80:20 in favor of the (S) configuration, or at least about 85:15, such as about 89:11.

In some embodiments, in a step (iii), (S)-N-((S)-1-cyano-2,2-difluoroethyl)-2-methylpropane-2-sulfinamide is hydrolyzed in acid to give the product (S)-2-(chloro-λ⁵-azaneyl)-3,3-difluoropropanoic acid:

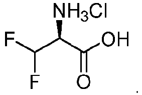

Suitable acids include hydrogen halides, such as hydrogen bromide, hydrogen chloride, and hydrogen iodide. Additional suitable acids include trifluoroacetic, sulfonic acid, methanesulfonic acid, benzenesulfonic acid, and *p*-toluenesulfonic acid. In some embodiments, the acid is HCl.

In a step (iv), (S)-2-(chloro-λ⁵-azaneyl)-3,3-difluoropropanoic acid is reduced to provide the intermediate compound of Formula (9-1): 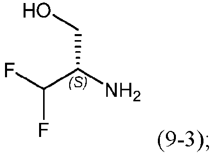 or an acid addition salt thereof. The reducing agent may be selected from among Red-Al (sodium bis(2-methoxyethoxy)aluminum hydride), lithium aluminum hydride (LAH), lithium tri-tert-butoxyaluminum hydride, and diisobutylaluminum hydride (DIBAL). A suitable reducing agent is borane (BH₃). This reaction may occur in a suitable solvent, such as methyl tert-butyl ether, cyclopentyl methyl ether, diethylether, diisoproylether, tetrahydrofuran, 2-methyltetrahydrofuran, 1,4-dioxane, dimethoxyethane (glyme), 1-methoxy-2-(2-methoxyethoxy)ethane (diglyme), diethoxyethane, toluene, anisole, dichloromethane, dichloroethane, hexanes, heptane. The reaction may occur at a temperature between 0 to 45°C.

In a step (v), the process comprises reacting the compound of Formula (9-3) with an acylating reagent to form a compound of Formula (10-1):

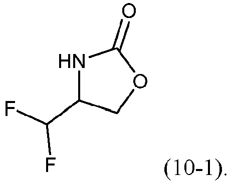

In some embodiments, the compound of Formula (10-1) is a compound of Formula (10-2):

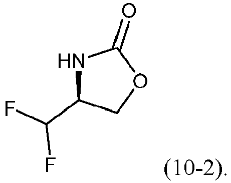

The reaction may occur in the presence of a base to release the free amine followed by addition of the acylating agent. The acylating reagent may be selected from among 1,1'-carbonyldiimidazole (CDI), phosgene, diphosgene, triphosgene, bis(2,2,2-trifluoroethyl) carbonate, bis(2,5-dioxopyrrolidin-1-yl) carbonate, 4-nitrophenyl chloroformate, di(pyridin-2-yl) carbonate, and diphenyl carbonate. The base may be selected from sodium carbonate, sodium bicarbonate, potassium carbonate, potassium bicarbonate, tripotassium phosphate, dipotassium hydrogen phosphate, diisopropylethylamine (DIPEA), triethylamine, N-methylmorpholine, and pyridine. The reaction may occur at temperatures between about 10°C and about 35°C. This reaction may occur in a suitable solvent, such as methyl tert-butyl ether, toluene, tetrahydrofuran, 2-methyltetrahydrofuran, N-methyl-2-pyrrolidone, acetonitrile, dimethylformamide, dichloromethane, methanol, ethanol, trifluoroethanol, and 1-propanol.

### 4. The Sulfone Route

In some embodiments, the process is for preparing an intermediate compound of Formula (8A): 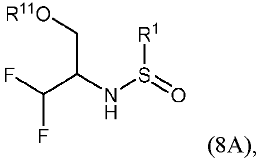 or a salt thereof, wherein:
R¹ is an optionally substituted C₁₋₁₂ alkyl, an optionally substituted C₃₋₁₄ cycloalkyl, or an optionally substituted C₆₋₁₄ aryl; and
R¹¹ is a hydroxyl protecting group.

In some embodiments, R¹ is the optionally substituted C₁₋₁₂ alkyl. In some embodiments, R¹ is an optionally substituted tertiary C₄₋₁₂ alkyl. In some embodiments, the tertiary C₄₋₁₂ alkyl may be selected from among *tert*-butyl, tert-pentyl, 2,3-dimethylbutyl, 3-ethylpentan-3-yl, 3-ethylpentan-2-yl, and others. In some embodiments, R¹ is selected from the group consisting of *tert*-butyl, tert-pentyl, 3-ethylpentan-3-yl, 1-methylcyclohexyl, 1-adamantyl, phenyl, and naphthyl. In some embodiments, R¹ is *tert*-butyl.

In some embodiments, R¹¹ is the hydroxyl protecting group selected from the group consisting of optionally substituted acetyl, trimethylacetyl, benzyl, silyl ethers including trimethylsilyl, triethylsilyl, triiso-propylsilyl, tert-butyldimethylsilyl, *tert*-butyldiphenylsilyl, and di-tert-butylmethylsilyl. In some embodiments, R¹¹ is benzyl.

In some embodiments, the process is for preparing an intermediate compound of Formula (8B): 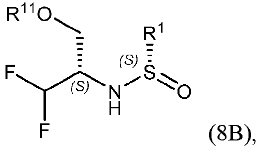 or a salt thereof,
wherein R¹ and R¹¹ are as defined as in Formula (8A).

In some embodiments, the process is for preparing an intermediate compound of Formula (8-1): 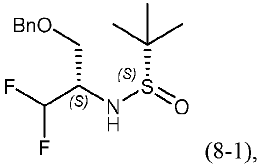 or a salt thereof.

In some embodiments, the process comprises (a) reacting a compound of Formula (11A): 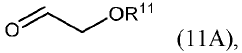 with a sulfonamide compound of Formula (3A) : 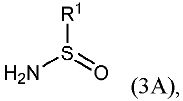 in the presence of a dehydrating reagent to form the compound of Formula (12A): 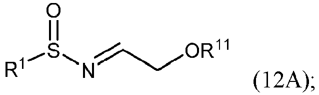 wherein R¹ and R¹¹ are as defined as in Formula (8A).

In some embodiments, R¹¹ is benzyl, and the compound of Formula (11A) is a compound of Formula (11): 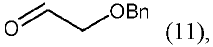 or a salt thereof.

In some embodiments, the compound of Formula (3A) is a compound of Formula (3B):

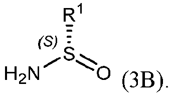

In some embodiments, R¹ is tert-butyl, and the compound of Formula (3A) is a compound of Formula (3):

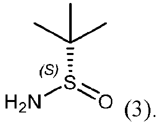

In some embodiments, the compound of Formula (12A) is a compound of Formula (12B): 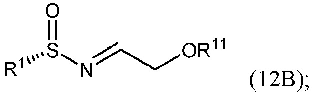 wherein R¹ and R¹¹ are as defined as in Formula (8A).

In some embodiments, the compound of Formula (12A) is a compound of Formula (12):

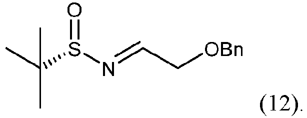

A suitable dehydrating agent may be a titanium alkoxide having the general formula Ti(OR)₄, wherein R is a C₁₋₆ alkyl group. The C₁₋₆ alkyl is selected from among methyl, ethyl, n-propyl, isopropyl, 2-methyl-1-propyl, 2-methyl-2-propyl, 2-methyl-1-butyl, 3-methyl-1-butyl, 2-methyl-3-butyl, 2,2-dimethyl-1-propyl, 2-methyl-1-pentyl, 3-methyl-1-pentyl, 4-methyl-1-pentyl, 2-methyl-2-pentyl, 3-methyl-2-pentyl, 4-methyl-2-pentyl, 2,2-dimethyl-1-butyl, 3,3-dimethyl-1-butyl, 2-ethyl-1-butyl, butyl, isobutyl, t-butyl, n-pentyl, isopentyl, neopentyl, n-hexyl and the like. In some embodiments, R is ethyl, and the dehydrating agent is Ti(OCH₂CH₃)₄. Additional suitable dehydrating agents include magnesium sulfate, copper sulfate, molecular sieves, triisopropylborate, tetramethyl orthosilicate, tetraethyl orthosilicate, and bis(trimethylsilyl)acetamide. In some embodiments, the dehydrating agent is copper sulfate. This reaction may occur in a suitable solvent, such as methyl tert-butyl ether, toluene, tetrahydrofuran, and dichloromethylene. This reaction may occur at room temperature, e.g., a temperature between about 20 and about 30 °C.

In some embodiments, the process comprises (b) reacting a compound of Formula (12A): 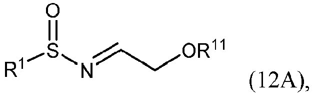 wherein R¹ and R¹¹ are as defined as in Formula (8A).
with a compound of Formula (13A): 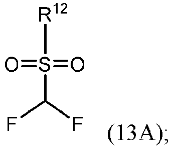 wherein R¹² is optionally substituted C₆₋₁₄ aryl and a base at a temperature below 0°C to form a compound of Formula (14A):

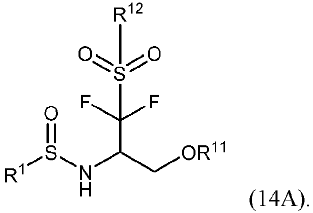

In some embodiments, R¹² is phenyl, and the compound of Formula (13A) has the structure (13):

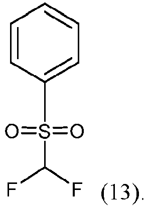

In some embodiments, the compound of Formula (14A) is a compound of Formula (14B):

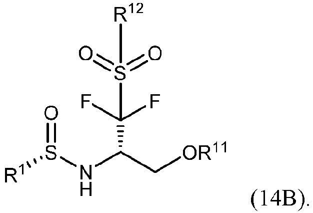

In some embodiments, the compound of Formula (14A) is a compound of Formula (14):

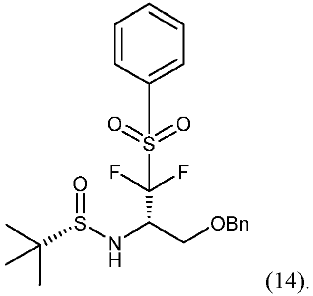

In some embodiments, the base in step (b) is sodium bis(trimethylsilyl)amide (NaHMDS). In some embodiments, step (b) is performed at a temperature below about -20°C, such as below about -30°C, such as below about -50°C, such as between about -70°C and about -80°C. This reaction may occur in a suitable solvent, such as methyl tert-butyl ether, toluene, tetrahydrofuran, N-methyl-2-pyrrolidone, acetonitrile, dimethylformamide, and dichloromethane.

In some embodiments, the process comprises (c) reacting the compound of Formula (14A) with magnesium in the presence of an acetate buffer to thereby form the compound of Formula (8A). The magnesium is elemental magnesium, which may be obtained as substantially pure (e.g., >98%) turnings. The acetate buffer comprising acetic acid and sodium acetate is suitable to control the pH to between about 4 and about 6. This reaction may occur in a suitable solvent, such as methyl tert-butyl ether, toluene, and tetrahydrofuran. This reaction may occur at room temperature, e.g., between about 20 and about 30°C.

The process comprises a step of (d) reacting the compound of Formula (8A): 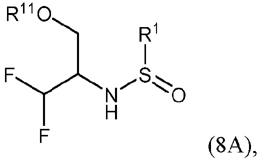 or a salt thereof,
with an acid to thereby yield an amine compound of Formula (9A): 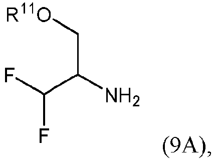 or an acid addition salt thereof.

In some embodiments, the compound of Formula (9A) is a compound of Formula (9B): 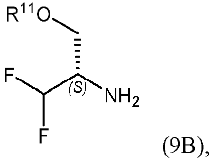 or an acid addition salt thereof.

In some embodiments, the acid in step (d) is HCl and the acid addition salt of the compound of Formula (9A) or (9B) is a hydrochloride salt having the structure (9C):

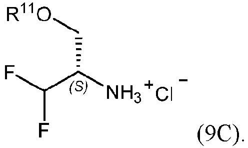

In some embodiments, the compound of Formula (9A) is a compound of Formula (9-4): 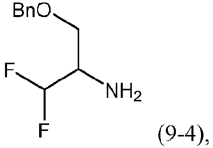 or an acid addition salt thereof having the structure (9-5):

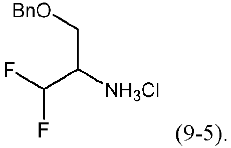

Suitable acids include hydrogen halides, such as hydrogen bromide, hydrogen chloride, and hydrogen iodide. Additional suitable acids include trifluoroacetic, sulfonic acid, methanesulfonic acid, benzenesulfonic acid, and *p*-toluenesulfonic acid. This reaction may occur in a suitable solvent, such as methyl tert-butyl ether, toluene, tetrahydrofuran, n-methyl-2-pyrrolidone, acetonitrile, dimethylformamide, dichloromethylene, methanol, and 1-propanol. This reaction may occur at room temperature, e.g., between about 20 and about 30°C.

In some embodiments, the process comprises (e) removing the hydroxyl protecting group of the compound of Formula (9A) to form a compound of Formula (9-1): 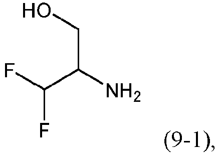 or an acid addition salt thereof.

In some embodiments, the compound of Formula (9-1) is a hydrochloride salt having the structure (9-2):

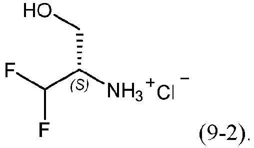

In some embodiments, the compound of Formula (9-1) is a compound of Formula (9-3): 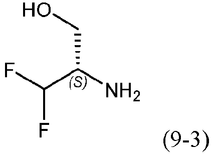 or the acid addition salt thereof.

The hydroxyl protection group may be removed by hydrogenation with palladium on activated carbon (Pd/C) in the presence of hydrogen gas. This reaction may occur at room temperature, e.g., between about 20 and about 30°C.

In some embodiments, the process comprises (f) reacting the compound of Formula (9-1), or an acid addition salt thereof, with an acylating reagent to form a compound of Formula (10-1):

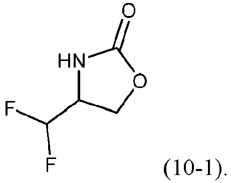

In some embodiments, the compound of Formula (10-1) is a compound of Formula (10-2):

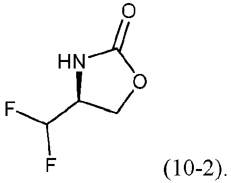

The reaction may occur in the presence of a base to release the free amine followed by addition of the acylating agent. The acylating reagent may be selected from among 1,1'-carbonyldiimidazole (CDI), phosgene, diphosgene, triphosgene, bis(2,2,2-trifluoroethyl) carbonate, bis(2,5-dioxopyrrolidin-1-yl) carbonate, 4-nitrophenyl chloroformate, di(pyridin-2-yl) carbonate, and diphenyl carbonate. The base may be selected from sodium carbonate, sodium bicarbonate, potassium carbonate, potassium bicarbonate, tripotassium phosphate, dipotassium hydrogen phosphate, diisopropylethylamine (DIPEA), triethylamine, N-methylmorpholine, and pyridine. The reaction may occur at temperatures between about 10°C and about 35°C. This reaction may occur in a suitable solvent, such as methyl tert-butyl ether, toluene, tetrahydrofuran, 2-methyltetrahydrofuran, N-methyl-2-pyrrolidone, acetonitrile, dimethylformamide, dichloromethane, methanol, ethanol, trifluoroethanol, and 1-propanol.

### Preparation of benzoxazepin oxazolidinone compounds

In some embodiments, the present invention includes processes, methods, reagents, and intermediates for the synthesis of benzoxazepin oxazolidinone compounds, including (S)-2-((2-((S)-4-(difluoromethyl)-2-oxooxazolidin-3-yl)-5,6-dihydrobenzo[f]imidazo[1,2-d][1,4]oxazepin-9-yl)amino)propanamide **18,** having the structure:

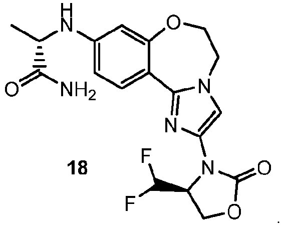

In one aspect, provided is a process for the preparation of compound **18,** having the structure: 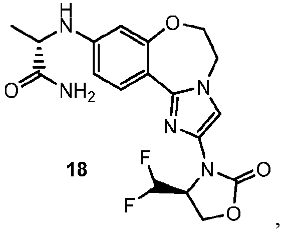 comprising reacting compound **17,** having the structure: 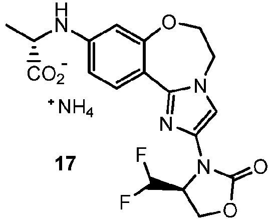 with ammonia or an ammonia equivalent through an amide bond formation reaction (i.e., in the presence of or by contacting with one or more peptide coupling reagents).

The amide bond formation reaction between compound **17** and ammonia or ammonia equivalent to form compound **18** can be facilitated using peptide coupling reagents, for example, a reagent or a combination of two reagents including, but not limited to, N-hydroxysuccinimide (HOSu) and N-(3-dimethylaminopropyl)-N'-ethylcarbodiimide hydrochloride (EDC), 1-hydroxybenzotriazole (HOBt) and EDC, 1-hydroxy-7-azabenzotriazole (HOAt) and EDC, 2-hydroxypyridine-1-oxide and EDC, ethyl (hydroxyimino)cyanoacetate (Oxyma) and EDC, 3-[bis(dimethylamino)methyliumyl]-3H-benzotriazol-1-oxide hexafluorophosphate (HBTU), 1-[bis(dimethylamino)methylene]-1H-1,2,3-triazolo[4,5-b]pyridinium 3-oxide hexafluorophosphate (HATU), and 1,1'-carbonyldiimidazole (CDI). The dehydrating reagent EDC can be replaced with other carbodiimides such as N,N-diisopropylcarbodiimide (DIC) or *N,N-*dicyclohexylcarbodiimide (DCC).

Examples of ammonia equivalents include, but are not limited to, ammonium acetate, ammonium bicarbonate, ammonium carbamate, ammonium carbonate, ammonium chloride, ammonium hydroxide, and ammonium phosphate.

In some embodiments, the process for the preparation of compound **18** comprises reacting compound **17** with ammonia or an ammonia equivalent and a peptide coupling regent. In some embodiments, the peptide coupling regent comprises a carbodiimide (e.g., DIC or EDC), and an auxiliary reagent (e.g., HOSu or HOBt). In some embodiments, the peptide coupling regent comprises CDI. Coupling reagents such as DIC/HOSu, EDC/HOSu, EDC/HOBt or CDI provide processes with higher efficiency and lower costs, and environmentally benign by-products that are easier to remove, compared to processes using coupling reagents such as HATU and HBTU, especially for syntheses on kilogram and above scales. In some embodiments, the process for the preparation of compound **18** comprises reacting compound **17** with ammonia or an ammonia equivalent and a peptide coupling regent selected from the group consisting of DIC/HOSu, EDC/HOSu, EDC/HOBt and CDI. In one embodiment, the process for the preparation of compound **18** comprises reacting compound **17** with ammonia, HOSu and EDC. In one embodiment, the process for the preparation of compound **18** comprises reacting compound **17** with ammonium bicarbonate, HOSu and DIC.

In some embodiments, compound **17** is prepared by a process comprising reacting compound **16,** having the structure: 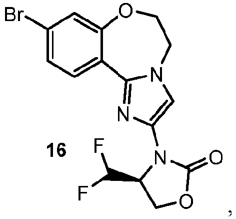 with (S)-2-aminopropanoic acid via a copper-catalyzed C-N coupling (i.e., in the presence of or by contacting with a copper catalyst).

In some embodiments, the C-N coupling between compound **16** and (S)-2-aminopropanoic acid to form compound **17** can be performed using a copper catalyst, a base, and a solvent. Examples of the copper catalyst include, but are not limited to, copper (I) oxide, copper (I) chloride, copper (I) bromide, copper (I) iodide, copper (I) trifluoromethanesulfonate, and copper (II) oxide. Examples of the base include, but are not limited to, potassium phosphate, cesium carbonate, and potassium carbonate. The solvent can be chosen from, but not limited to, dimethyl sulfoxide (DMSO), *N,N*-dimethylformamide (DMF), *N,N*-dimethylacetamide (DMA), and *N*-methyl-2-pyrrolidinone (NMP). In some embodiments, compound **17** is prepared by a process comprising reacting compound **16** and a copper (I) catalyst (e.g., copper (I) oxide). In some embodiments, compound **17** is prepared by a process comprising reacting compound 16 and (*S*)-2-aminopropanoic acid in the presence of a copper (I) catalyst (e.g., copper (I) oxide) and a base (e.g. potassium phosphate tribasic) in a solvent (e.g., DMSO).

The carboxylic acid formed from coupling of compound **16** and (*S*)-2-aminopropanoic acid is unstable, difficult to isolate, and subject to decomposition. Conversion of the acid to the ammonium salt (compound **17**) provides for a stable intermediate compound that can be isolated from the unreacted starting materials and by-products.

In some embodiments, compound **16** is prepared by a process comprising reacting compound **15,** having the structure: 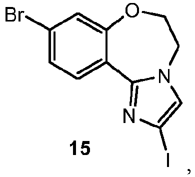 with compound (10-2), having the structure: 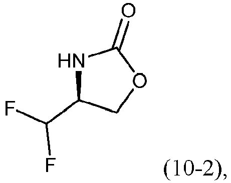 via a copper-catalyzed C-N coupling reaction.

In one embodiment, the C-N coupling reaction between compound **15** and compound **(10-2)** to form compound **16** can be performed using a copper salt, a ligand, a base, and a solvent. Examples of suitable copper salts include, but are not limited to, copper (I) oxide, copper (I) chloride, copper (I) bromide, copper (I) iodide, copper (I) trifluoromethanesulfonate, copper (II) acetate, copper (II) chloride, copper (II) bromide, copper (II) iodide, copper (II) oxide, and copper (II) trifluoromethanesulfonate. Examples of suitable ligands include, but are not limited to 1,2-diamines (e.g., as *trans*-*N,N*-dimethylcyclohexane-1,2-diamine, *trans*-1,2-diaminocyclohexane, and *N,N*'-dimethylethylenediamine), 1,10-phenanthroline or derivatives (e.g., 3,4,7,8-tetramethyl-1,10-phenanthroline), glycine, *N,N*-dimethylglycine, 2,2,6-trimethylheptane-3,5-dione, and 2-isobutyrylcyclohexan-1-one. Examples of suitable bases include, but are not limited to, potassium phosphate, cesium carbonate, and potassium carbonate. Suitable solvents include, but are not limited to, dimethyl sulfoxide (DMSO), *N,N-*dimethylformamide (DMF), *N,N*-dimethylacetamide (DMA), *N*-methyl-2-pyrrolidinone (NMP), acetonitrile, 2-methyltetrahydrofuran, toluene, and 1,4-dioxane. In some embodiments, compound **16** is prepared by a process comprising reacting compound **15,** compound **(10-2),** a copper salt (e.g., copper (II) acetate or copper(I) iodide) and a ligand (e.g., *trans-N,N-*dimethylcyclohexane-1,2-diamine or 3,4,7,8-tetramethyl-1,10-phenanthroline). In some embodiments, compound **16** is prepared by a process comprising reacting compound **15,** compound **(10-2),** a copper salt (e.g., copper (II) acetate or copper(I) iodide) and a ligand (e.g., *trans*-*N*,*N*-dimethylcyclohexane-1,2-diamine or 3,4,7,8-tetramethyl-1,10-phenanthroline) in the presence of a base (e.g. cesium carbonate or potassium phosphate tribasic) in a solvent (e.g., 2-methyltetrahydrofuran or acetonitrile). In one embodiment, compound **16** is prepared by a process comprising reacting compound **15,** compound **(10-2),** copper (II) acetate and *trans-N,N-*dimethylcyclohexane-1,2-diamine in the presence of cesium carbonate in 2-methyltetrahydrofuran. In another embodiment, compound **16** is prepared by a process comprising reacting compound **15,** compound **(10-2),** copper (II) acetate and 3,4,7,8-tetramethyl-1,10-phenanthroline in the presence of potassium phosphate tribasic in acetonitrile. In one embodiment, compound **16** is prepared by a process comprising reacting compound **15,** compound **(10-2),** copper (I) iodide and *trans-N*,*N*-dimethylcyclohexane-1,2-diamine in the presence of cesium carbonate in 2-methyltetrahydrofuran.

In some embodiments, compound **15** is prepared by a process disclosed in International Application PCT/EP2017/083143 (WO 2018/109204), the entire disclosure of which is incorporated by reference as if set forth in its entirety. Briefly, WO 2018/109204 discloses a process for preparing compound 15 comprising the following steps:
(a) reacting 9-bromo-5,6-dihydrobenzo[f]imidazo[1,2-d][1,4]oxazepine (compound **13**'), having the structure: 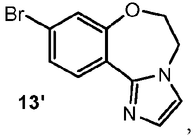 with an iodinating reagent (e.g., *N*-iodosuccinimide (NIS), iodine or iodine monochloride) to form 9-bromo-2,3-diiodo-5,6-dihydrobenzo[f]imidazo[1,2-d][1,4]oxazepine (compound **14**'), having the structure: 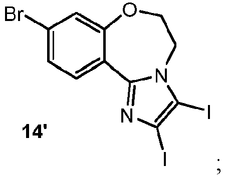 and
(b) reacting 9-bromo-2,3-diiodo-5,6-dihydrobenzo[f]imidazo[1,2-d][1,4]oxazepine (compound **14**') with a Grignard reagent (e.g., ethylmagnesium bromide or isopropylmagnesium chloride) to form compound **15.**

In some embodiments, the iodinating reagent used for converting compound **13'** to compound **14'** is iodine and sodium periodate. The reaction may occur in acetonitrile in the presence of an acid (such as aqueous sulfuric acid).

In some embodiments, the step of reacting compound **14'** with a Grignard reagent comprises a batch process whereby the reactants are added in batches to the reaction vessel to form compound **15.** In some embodiments, the step of reacting compound **14'** with a Grignard reagent and following quench of reaction mixture (for example, with acetic acid) comprises a flow process whereby the reactants are continuously fed to pipe reactors to form compound **15.**

In some embodiments, 9-bromo-5,6-dihydrobenzo[f]imidazo[1,2-d][1,4]oxazepine (compound **13'**) is prepared by a process comprising reacting a compound **12',** having the structure: 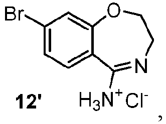 with chloroacetaldehyde to form 9-bromo-5,6-dihydrobenzo[f]imidazo[1,2-d][1,4]oxazepine.

In one embodiment, the above condensation reaction can be performed in the presence of a base in a solvent. Suitable bases include, but are not limited to, sodium bicarbonate, potassium bicarbonate, sodium carbonate, and potassium carbonate. Suitable solvents include, but are not limited to, isopropyl alcohol and 2-methyltetrahydrofuran.

The processes of this invention are highly economic and robust. They ensure a reliable high quality of product.

### ¹⁴C Labeled inavolisib

Further provided is a ¹⁴C labeled inavolisib, (2S)-2-[[2-[(4S)-4-(difluoromethyl)-2-keto-oxazolidin-3-yl]-5,6-dihydro[2-¹⁴C]imidazolo[1,2-d][1,4]benzoxazepin-9-yl]amino]propionamide, useful in studying human absorption, distribution, metabolism, and excretion of inavolisib.

(2S)-2-[[2-[(4S)-4-(difluoromethyl)-2-keto-oxazolidin-3-yl]-5,6-dihydro[2-¹⁴C]imidazolo[1,2-d][1,4]benzoxazepin-9-yl]amino]propionamide can be synthesized following the procedures described in International Application PCT/EP2017/083143 (WO 2018/109204) for making inavolisib stating from 4-bromo-2-fluoro-benzo[14C]nitrile, for example, as shown in Scheme 6.

Provided is a process of making a ^{1*4*}C labeled inavolisib comprising converting 4-bromo-2-fluoro-benzo[¹⁴C]nitrile to (2S)-2-[[2-[(4S)-4-(difluoromethyl)-2-keto-oxazolidin-3-yl]-5,6-dihydro[2-¹⁴C]imidazolo[1,2-d][1,4]benzoxazepin-9-yl]amino]propionamide. In some embodiments, the process comprises the steps as shown in Scheme 6. Detailed reaction conditions in Scheme 6 are intended to be working examples of the reagents, solvents and reaction conditions and are not to be construed as restricting. Other equivalents can be applied.

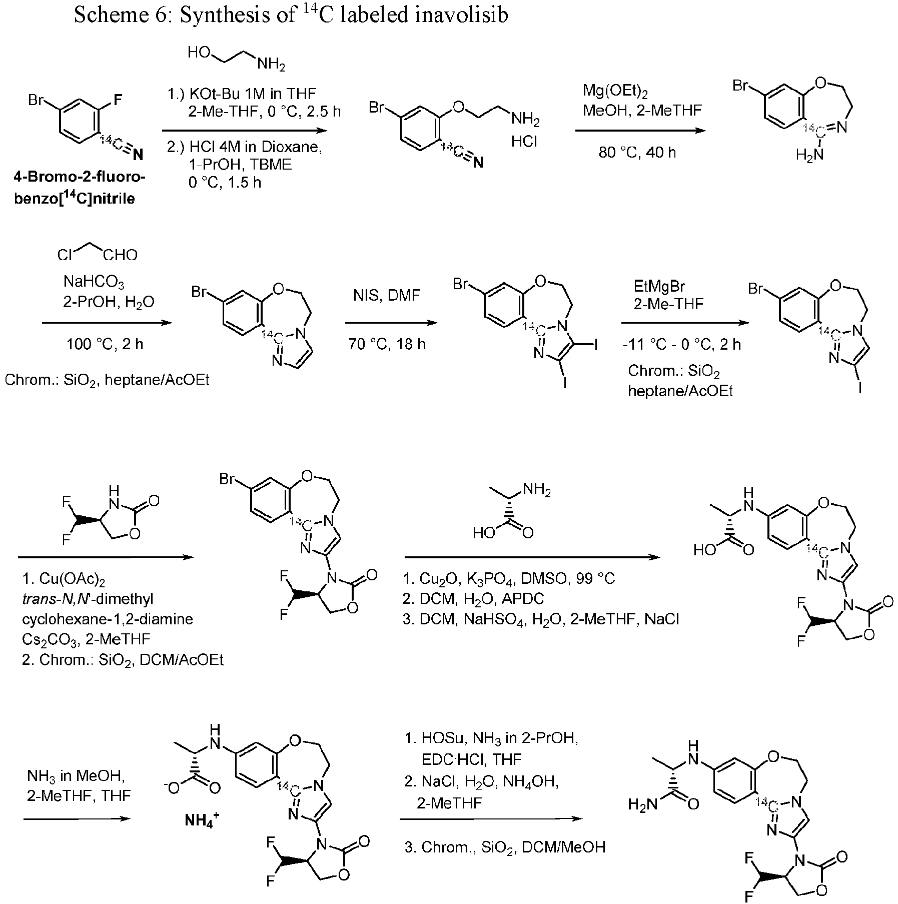

Starting materials and reagents for the preparation of compounds as disclosed herein are generally available from commercial sources or are readily prepared using methods well known to those skilled in the art (e.g., prepared by methods generally described in Louis F. Fieser and Mary Fieser, Reagents for Organic Synthesis, v. 1-19, Wiley, N.Y. (1967-1999 ed.), or Beilsteins Handbuch der organischen Chemie, 4, Aufl. ed. Springer-Verlag, Berlin, including supplements (also available via the Beilstein online database).

The following Schemes and Examples illustrate the chemical reactions, processes, and methodology for the synthesis of benzoxazepin oxazolidinone compounds, and certain intermediates and reagents. 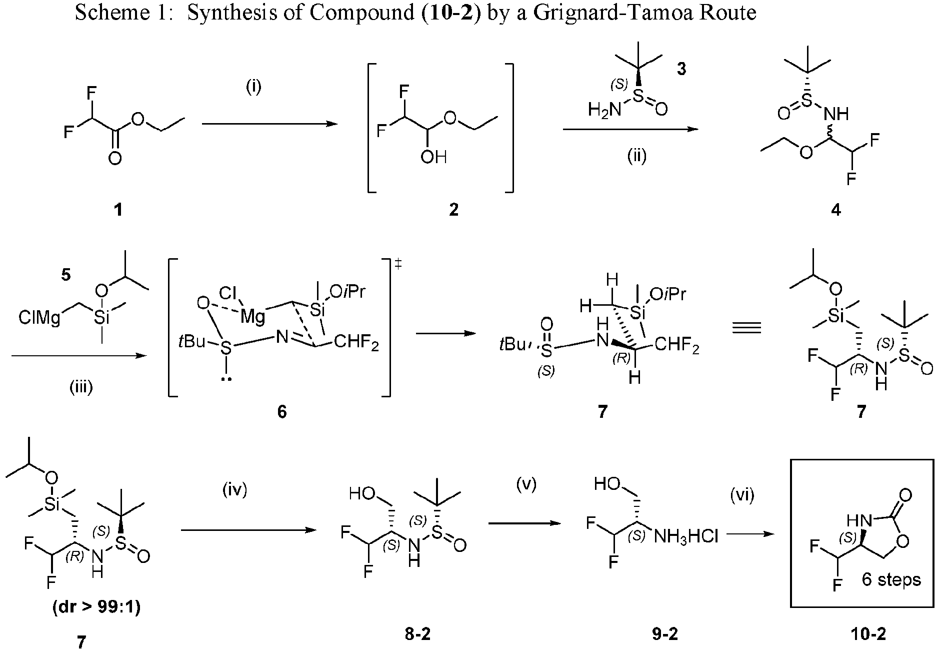 (i) Red-Al, TBME, 0°C, 40-50% o. th. in TBME; (ii) Ti(OEt)₄, greasy solid 55-60% o. th.; (iii) THF, 10°C, 75% o. th. dr: 93:7; (iv) KF, KHCO₃, H₂O₂, MeOH, 45°C, white solid 61% o. th.; (v) HCl, MeOH, rt, white solid, 2.8 g, 92% o. th., and (vi) CDI, DIPEA, DMF, 49% o. th.

Scheme 1 shows a synthesis of (S)-4-(difluoromethyl)oxazolidin-2-one (10-2). Hemiacetal, 1-ethoxy-2,2-difluoroethan-1-ol 2 was obtained by partial reduction of ethyl 2,2-difluoroacetate **1** using sodium bis(2-methoxyethoxy)aluminium hydride (Red-Al) at 0°C. Hemiacetal, 1-ethoxy-2,2-difluoroethan-1-ol **2** was obtained in a solution in tert-butyl methyl ether TBME. Hemiacetal, 1-ethoxy-2,2-difluoroethan-1-ol **2** and (*S*)-tertbutylsulfinamide **3** were reacted in the presence of titanium ethoxide. N,O-acetal, (S)-N-(1-ethoxy-2,2-difluoroethyl)-2-methylpropane-2-sulfinamide **4** was obtained as a mixture of diastereomers in 60% yield. An excess of Grignard reagent ((isopropoxydimethylsilyl)methyl)magnesium chloride **5** was reacted with **4** in tetrahydrofuran (THF). The first equivalent of Grignard reagent **5** eliminates ethanol from **4** to release the corresponding imine. According to Ellman and co-workers (J. Am. Chem. Soc. 1997, 119, 9913-9914.) the reaction proceeds through a six membered transition state which induces high stereocontrol. The reaction was performed at -25°C, which gave (S)-N-((R)-1,1-difluoro-3-(isopropoxydimethylsilyl)propan-2-yl)-2-methylpropane-2-sulfinamide **7** with a diastereomeric ratio of 97:3. When the reaction was repeated at 10°C, **7** was obtained in 75% o. th. and a diastereomeric ratio of 93:7.

Crude (S)-N-((R)-1,1-difluoro-3-(isopropoxydimethylsilyl)propan-2-yl)-2-methylpropane-2-sulfinamide **7** was directly summited to Tamao oxidation conditions using potassium fluoride, potassium bicarbonate, and hydrogen peroxide in methanol. Alcohol (S)-N-((S)-1,1-difluoro-3-hydroxypropan-2-yl)-2-methylpropane-2-sulfinamide **8-2** could be obtained as white solid in 61% yield (Org. Process Res. Dev. 2014, 18, 66-81 described the Tamao oxidation of a related isopropyloxy dimethyl silane but without a sulfinamide moiety).

Tertbutyl sulfinamide **8-2** was hydrolyzed using hydrochloric acid in methanol to obtain **9-2** in good yield. The latter was treated with N,N-diisopropylethylamine (DIPEA) to release the free amine followed by addition of carbonyl diimidazole (CDI). (*S*)-4-(difluoromethyl)oxazolidin-2-one **(10-2)** was isolated in 48% o. th. with an enantiomeric ratio of 97:3.

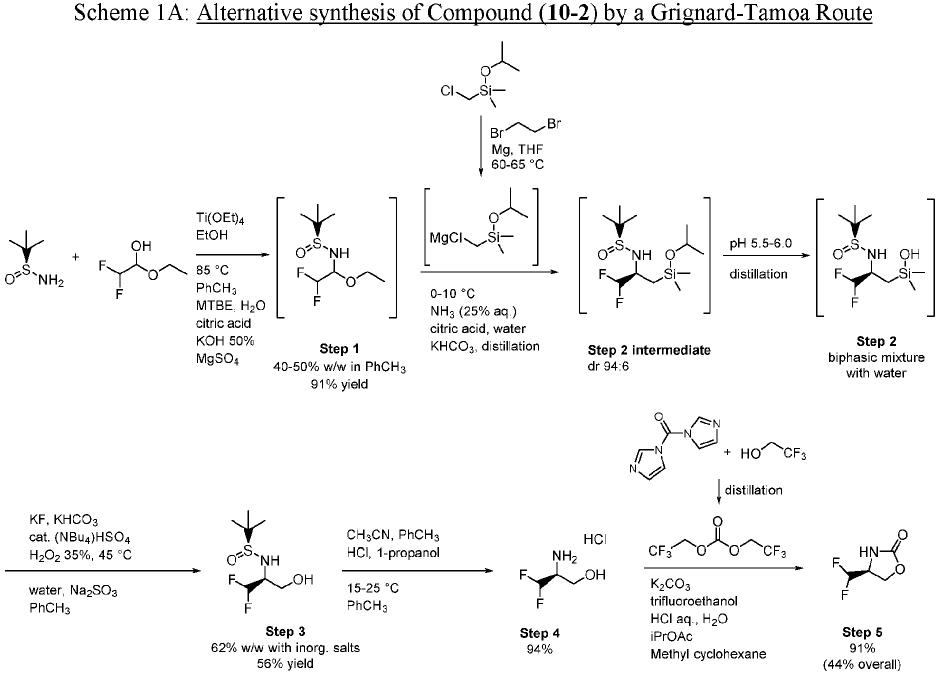

Scheme 1A shows an alternative synthesis of (*S*)-4-(difluoromethyl)oxazolidin-2-one **(10-2)**. Further details of process are provided in the Examples below. 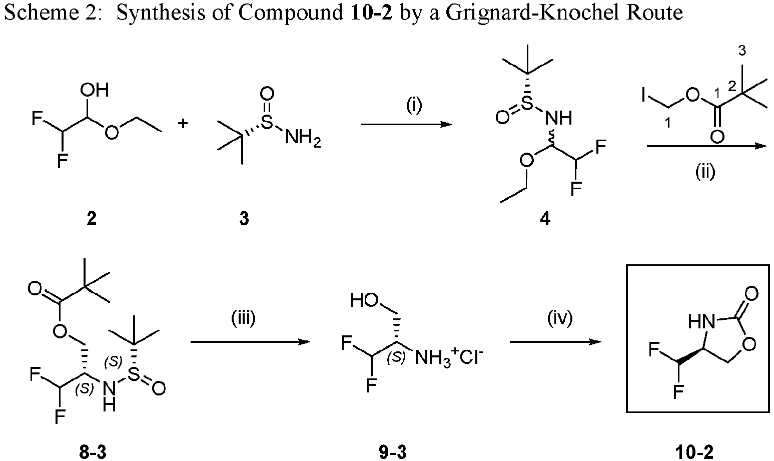 (i) Ti(OEt)₄, neat, 60°C, 42% o. th., (ii) iodomethyl pivalate, iPrMgCl, THF/NMP, -65°C, 75% o. th.; (iii) HCl, 22%, 80°C, 2 hours, 97%, o. th.; and (iv) Et₃N, CDI, ACN, r.t., 55%, o.th.

Scheme 2 shows the synthesis of intermediate 9-3. Magnesation of iodomethyl pivalate was done according to the protocol reported by Knochel at -78°C (Synlett, (11), 1820-1822; 1999). 2.2 equivalent of iodomethyl pivalate and isopropylmagnesium chloride was used in order to eliminated ethanol from *N*,*O*-acetal **4** to generate the imine in-situ, which reacts further to **8-3.** The reaction worked very well on small scale and **8-3** was isolated 75% o. th. after purification by column chromatography. No minor isomer could be detected (crude ¹H-NMR). The reaction was repeated on 20 g scale. In this case, gummy balls were formed after magnesation of iodomethyl pivalate at -60 to 78°C. Knochel describes the Grignard reagent to be stable for only few hours. Ester **8**-3 was hydrolyzed using HCl at 80°C to obtain aminoalcohol hydrochloride **9-3** in quantitative yield. Amino alcohol hydrochloride 9-3 was mixed with triethylamine and ACN at room temperature. CDI was added at room temperature in one portion. Full conversion of **9-3** was obtained after 2 h. The volatiles were evaporated and the crude product was purified by column chromatography. (*S*)-4-(difluoromethyl)oxazolidin-2-one **(10-2)** was obtained as pale yellow oil (308 mg, 55% o. th., single enantiomer). 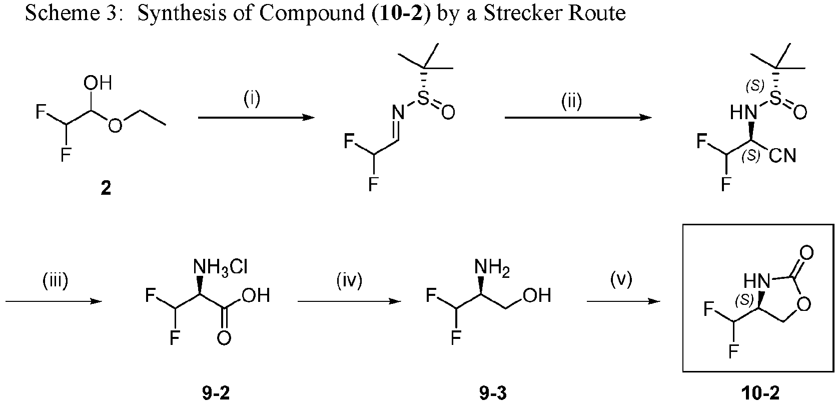 (i) (S)-Ellmans aux, toluene reflux 18% o. th.; (ii) TMSCN (2.0 eq), cat. Y(OTf₃), 10 V DCM, rt, 73% o. th., dr = 5:1, isol. dr =89:11; (iii) HCl, 33%, 80°C; (iv) BH₃, THF, 0 - 45°C, 30% o. th.; and (v) Et₃N, CDI, IPAc, rt, 45-50% o. th.

Scheme 3 shows the synthesis of (*S*)-4-(difluoromethyl)oxazolidin-2-one **(10-2).** Hemiacetal 2 and (S)-tertbutylsulfinamide in toluene was refluxed under Dean-Stark conditions. The desired imine was obtained by vacuum distillation in low yield. Severe corrosion on our laboratory glass equipment was observed. This indicates hydrofluoric acid formation due to decomposition. Strecker reaction of the (S)-tertbutylsulfinamide using TMS-CN and Lewis acid gave the desired aminonitrile. Diastereoselectivity of the Strecker reaction was found to depend strongly depends on the Lewis (scandium triflate, Yttrium triflate and trimethylsilyl triflate). Reaction was performed on larger scale using yttrium triflate. The crude product was purified by column chromatography to obtain the aminonitrile in 73% o. th. and dr of 89:11. Aminonitrile was hydrolyzed and auxiliary was cleaved using aq. HCl to obtain alanine hydrochloride derivative **9-2.** Reduction of carboxylic acid **9-2** using borane THF complex led to aminoalcohol **9-3** in low yield. The sequence was completed using CDI to obtain oxazolidinone **10-2.** Based on chiral GC analysis we obtained the enantiomer of **10-2** with de of 89:11. 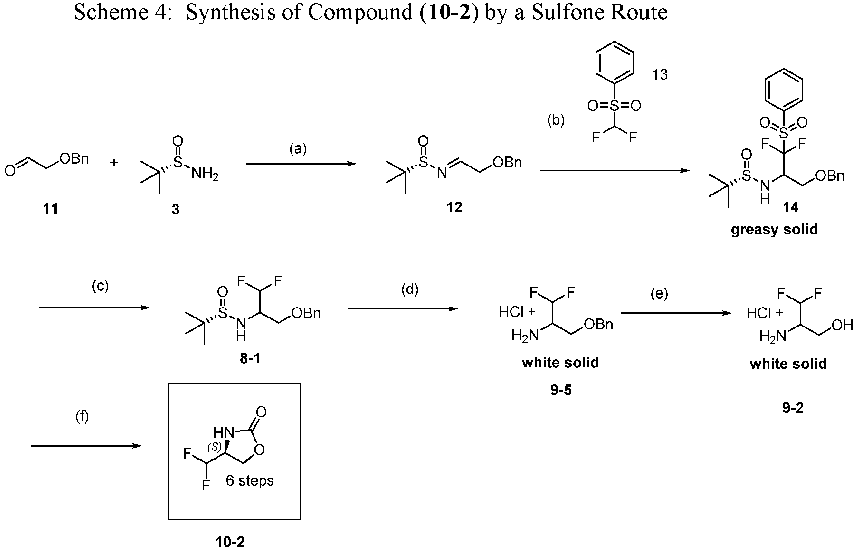 (a) CuSO₄, DCM; (b) NaHMDS, THF, -78°C, quant yield d.r. > 99:1 (NMR); (c) Mg, AcOH/NaOAc, DMF, rt 48% o. th.; (d) HCl, 37%, MeOH, rt, 79% o. th.; (e) H2, Pd/C, MeOH, rt, 78% o. th.; and (f) DIPEA, CDI, THF, rt, 44% o. th.

Scheme 4 shows the synthesis of (*S*)-4-(difluoromethyl)oxazolidin-2-one **(10-2).** Aldehyde 2-(benzyloxy)acetaldehyde **11** and (*S*)-tertbutylsulfinamide **3** were stirred in the presence of copper sulfate in dichloromethane (DCM). Full and clean conversion to imine (R,E)-N-(2-(benzyloxy)ethylidene)-2-methylpropane-2-sulfinamide **12** was obtained overnight. Running the reaction at reflux in toluene led to severe drop of yield (< 50% o. th.). Imine **12** and difluoromethyl phenyl sulfone **13** were treated with sodium bis(trimethylsilyl)amide (NaHMDS) at -70°C as a solution in tetrahydrofuran (THF) to obtain the desired product **14.** The reaction profile was very clean (TLC only one spot) and no minor diastereomer was detected by NMR. Phenylsulfone (R)-N-((S)-3-(benzyloxy)-1,1-difluoro-1-(phenylsulfonyl)propan-2-yl)-2-methylpropane-2-sulfinamide **14** was deprotected using elemental magnesium turnings in dimethylformamide (DMF)/acetate buffer to obtain key intermediate (S)-N-((S)-3-(benzyloxy)-1,1-difluoropropan-2-yl)-2-methylpropane-2-sulfinamide **8-1** as the single product in 48% o. th. (not optimized). Alternative methods to remove the sulfone such as hydrogenation by Raney nickel didn't give any conversion to **8-1.** The auxiliary was cleaved using aq. HCl in methanol to obtain the ammonium hydrochloride of 3-(benzyloxy)-1,1-difluoropropan-2-amine **9-5** in good yield as a white crystalline solid. Benzyl group of **9-5** was removed by hydrogenation with Pd/C to obtain the ammonium hydrochloride of 2-amino-3,3-difluoropropan-1-ol **9-2** as a white solid. (*S*)-4-(difluoromethyl)oxazolidin-2-one **(10-2)** was obtained in moderate yield by treating **9-2** with N,N-diisopropylethylamine (DIPEA) to release the free amine followed by addition of carbonyl diimidazole (CDI). Based on chiral GC analysis we obtained the enantiomer of (*S*)-4-(difluoromethyl)oxazolidin-2-one **(10-2)** with > 99.9% ee. 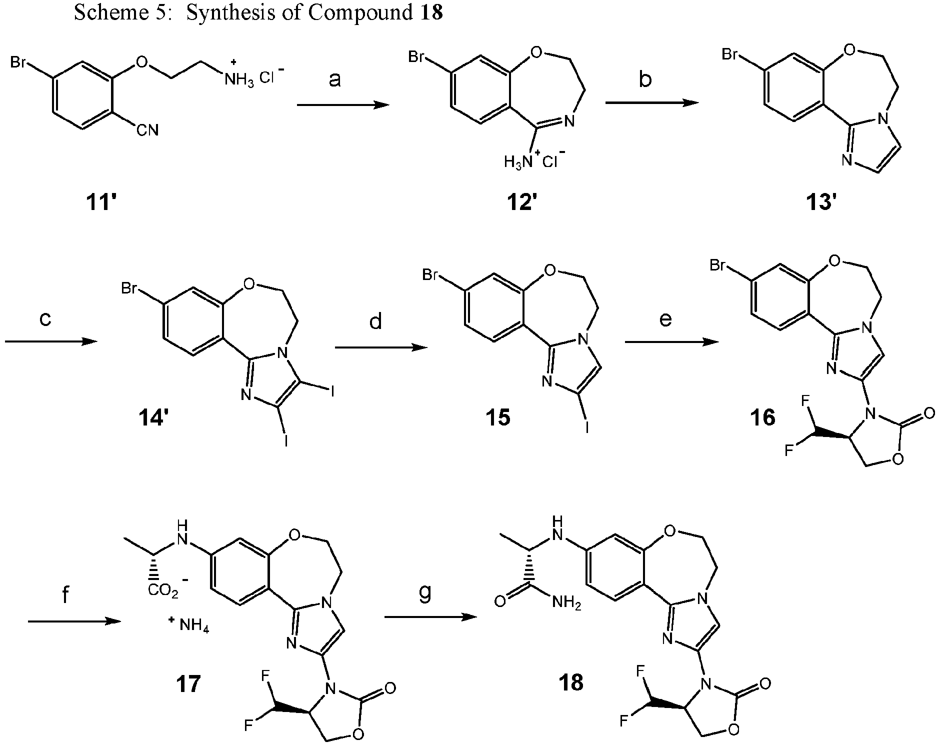 a: i) Mg(OEt)₂, MeOH, MeTHF, ii) HCl, n-PrOH; b: ClCHCHO, KHCO₃, MeTHF, H₂O, c: NIS, DMF; d: EtMgBr, THF; e: **(10-2)**, Cu(OAc)₂, trans-N,N'-dimethylcyclohexane-1,2-diamine, Cs₂CO₃, MeTHF; f: i) (*S*)-2-aminopropanoic acid, Cu₂O, K₃PO₄, DMSO, ii) NH₃, MeOH, THF; g: i) NH₃, HOSu, EDC, THF, ⁱPrOH, ii) EtOH, H₂O.

Scheme 5 shows the preparation of (S)-2-((2-((S)-4-(difluoromethyl)-2-oxooxazolidin-3-yl)-5,6-dihydrobenzo[flimidazo[1,2-d][1,4]oxazepin-9-yl)amino)propanamide **18.** 2-(5-Bromo-2-cyanophenoxy)ethan-1-aminium chloride **11'** cyclized with magnesium ethoxide, Mg(OEt)₂ in methanol, and acidification with a solution of hydrogen chloride in *n*-propanol to give of 8-bromo-2,3-dihydrobenzo[*f*][1,4]oxazepin-5-amine hydrochloride **12'.** Cyclization of **12'** to form the imidazole ring with aqueous chloroacetaldehyde in the presence of potassium bicarbonate as base gave 9-bromo-5,6-dihydrobenzo[*f*]imidazo[1,2-*d*][1,4]oxazepine **13'.** Bis-iodination of the imidazole **13'** with *N*-iodosuccinimide (NIS) or other iodinating reagents such as iodine or iodine monochloride gave 9-bromo-2,3-diiodo-5,6-dihydrobenzo[*f*]imidazo[1,2-*d*][1,4]oxazepine **14'.** Selective reduction of **14'** via an iodo-metal exchange using a Grignard reagent such as ethylmagnesium bromide or isopropylmagnesium chloride gave 9-bromo-2-iodo-5,6-dihydrobenzo[*f*]imidazo[1,2-*d*][1,4]oxazepine **15.** Chemoselective replacement of iodide from **15** with (*S*)-4-(difluoromethyl)oxazolidin-2-one **(10-2)** in the presence of a copper catalyst such as copper (II) acetate or copper (I) iodide, a ligand such as trans-*N,N'*-dimethylcyclohexane-1,2-diamine, 1,10-phenanthroline, or 3,4,7,8-tetramethyl-1,10-phenanthroline, an inorganic base such as cesium carbonate or tripotassium phosphate, and 2-methyltetrahydrofuran or acetonitrile as the solvent gave (*S*)-3-(9-bromo-5,6-dihydrobenzo[j]imidazo[1,2-d][1,4]oxazepin-2-yl)-4-(difluoromethyl)oxazolidin-2-one **16.** Replacement of bromide from **16** with (*S*)-2-aminopropanoic acid in the presence of a copper catalyst such as copper (I) oxide, an inorganic base such as tripotassium phosphate, and DMSO as the solvent followed by the ammonium salt formation in THF using a solution of ammonia in methanol as the ammonia source gave ammonium (*S*)-2-((2-((*S*)-4-(difluoromethyl)-2-oxooxazolidin-3-yl)-5,6-dihydrobenzo[*f*]imidazo[1,2-*d*][1,4]oxazepin-9-yl)amino)propionate **17**. Conversion of the carboxylate salt **17** to the carboxamide was effected with a solution of ammonia in 2-propanol, an additive such as *N*-hydroxysuccinimide (HOSu) or 1-hydroxybenzotriazole (HOBt), and a dehydrating reagent such as *N*-(3-dimethylaminopropyl)-*N*'-ethylcarbodiimide hydrochloride (EDC) or *N*,*N*'-diisopropylcarbodiimide (DIC) in THF to give **18.**

### EXAMPLES

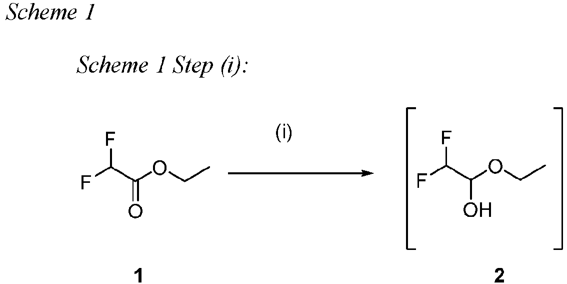

Hemiacetal, 1-ethoxy-2,2-difluoroethan-1-ol **2** was obtained by partial reduction of ethyl 2,2-difluoroacetate **1** using sodium bis(2-methoxyethoxy)aluminium hydride (Red-Al) at 0°C. Hemiacetal, 1-ethoxy-2,2-difluoroethan-1-ol **2** was obtained in a solution in tert-butyl methyl ether TBME.

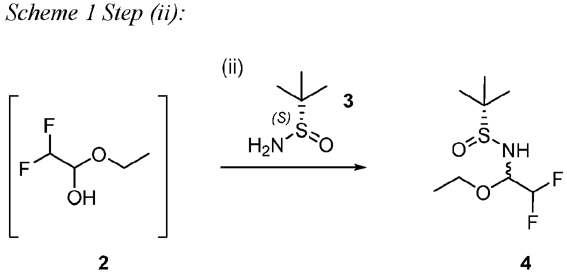

Difluoroacetaldehyde ethyl hemiacetal **2** (60.7 g; 90% w/w: 10% w/w ethanol) was placed in a 500 mL double glass jacked reactor equipped with a mechanical stirrer, thermometer, funnel, and nitrogen supply. (S)-tert-Butylsulfinamide **3** (50.0 g) and Titanium(IV)ethoxide (99.0 g) were added at a temperature less than 20°C. The suspension was heated to 80-90°C for at least 3 hours. The reaction mixture was stirred at this temperature for 4 hours until an orange solution is formed. This solution was cooled to 70-80°C, and the amount of (S)-*tert*-Butylsulfinamide **3** was determined. The reaction mixture was cooled to 15-25°C and aged for at least 2 hours.

In a separate 100 mL double glass jacked reactor equipped with a mechanical stirrer, thermometer, funnel, and nitrogen supply was added 200 mL pharmaceutical grade water and citric acid (79.4 g) at a temperature of 50°C. Potassium hydroxide (58.9 g, 50%) was added and the temperature was lowered to 15-20°C. The reaction mixture (204 g, 190 mL) prepared above was added adiabatically at a temperature less than 45°C. The orange solution was stirred for at least 60 minutes at a temperature between 30-40°C. The phases are separated into aqueous and organic phases. *tert*-Butylmethylether was added to the aqueous phase, and the mixture was stirred for at least 5 minutes at 30-40°C, which was followed by another phase separation. The two organic phases are combined at a temperature less than 30°C. Pharmaceutical grade toluene (100 mL) was added, and the mixture was stirred for at least 5 minutes at a temperature between 15-25°C. The phases are separated for at least 10 minutes.

Magnesium sulfate (anhydrous, 35 g) was suspended in pharmaceutical grade toluene (80 mL), added to the organic phase, and stirred for at least 30 minutes at a temperature less than 30°C. The suspension is filtered. The filtrate contains the desired product (443 mL; 398 g). The filtrate is heated to a temperature between 35-45°C and distilled at reduce pressure (90-22- mBar) to collect the Distillate 1 (312 mL, 253 g). Distillation is continued by additions of pharmaceutical grade toluene (150 mL) to collect Distillate 2 (160 mL, 136 g). (S)-N-(1-ethoxy-2,2-difluoroethyl)-2-methylpropane-2-sulfinamide **4** (170 mL, 171.2 g) was obtained as a pale yellow solution in toluene by filtering the combined distillates with pharmaceutical grade toluene (50 mL). The yield was 89.2% with a purity of 98.1%.

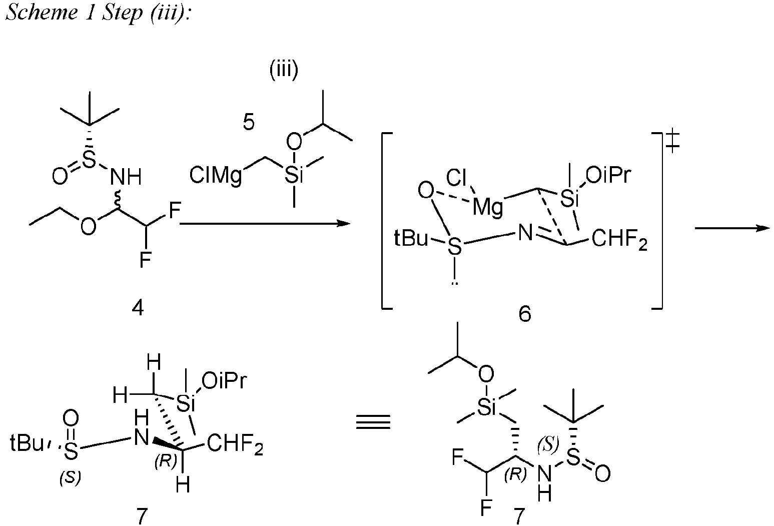

Tetrahydrofuran (stabilized, 700 mL) was placed in a 1500 ml double glass jacked reactor equipped with a mechanical stirrer, a thermometer, addition funnel and nitrogen supply at less than 30°C. Magnesium turnings (23.9 g) were added at less than 30°C. The suspension was warmed to 55-65°C. 1,2-dibromoethane (6.4 g) was added over 15 minutes, keeping the temperature between 55-65°C. (Chloromethyl)dimethyl isopropyloxysilane (5.7 g) was added over at least 20 minutes keeping the temperature between 55-65°C . The suspension was stirred for at least 15 minutes. (Chloromethyl)dimethyl isopropyloxysilane (164.0 g) was added over at least 120 minutes keeping the temperature between 55-65°C. The black mixture was stirred for at least 60 minutes a temperature between 55-65°C and then cooled to between 45-55°C. The mixture was cooled to 0-10°C. ((isopropoxydimethylsilyl)methyl)magnesium chloride 5 was obtained in solution at a purity between 85-90%. (S)-N-(1-ethoxy-2,2-difluoroethyl)-2-methylpropane-2-sulfinamide **4** (160 g) in toluene was added over a period of 2 hours.

A solution was prepared by combining pharmaceutical grade water (168 g), citric acid (117.3 g) and ammonia solution (122.4 g, 25%) and mixing at a temperature between 15-20°C. The mixture (1050 mL) of ((isopropoxydimethylsilyl)methyl)magnesium chloride **5** and (S)-N-(1-ethoxy-2,2-difluoroethyl)-2-methylpropane-2-sulfinamide **4** was added to the ammonium citrate solution over 5 minutes. The biphasic mixture was stirred at a temperature between 35-45°C for at least 10 minutes, and the phases were separated for at least 15 minutes. The lower, aqueous phase (350 mL, 438 g) was drained, leaving a light brown, clear organic phase (1050 mL, 940 g). Potassium hydrogen carbonate (1.7 g) and pharmaceutical grade water (34 mL) was added to the organic phase. The organic phase was distilled at temperature between 35-50°C at reduced pressure (90-300 mBar). The collected distillate (420-450 mL) was combined with pharmaceutical grade water (600 mL) and distilled again until between 1000-1050 mL distillate was collected. The distillate contained (S)-N-((R)-1,1-difluoro-3-(isopropoxydimethylsilyl)propan-2-yl)-2-methylpropane-2-sulfinamide 7 (887 g). The pH of the distillate was adjusted to between pH 5.2-5.7 using citric acid (10% solution) and distilled again at a temperature between 35-55°C and reduced pressure between 80-120 mBar to collect 480-520 mL distillate. This distillate contained 492 g of (S)-N-((R)-1,1-difluoro-3-(isopropoxydimethylsilyl)propan-2-yl)-2-methylpropane-2-sulfinamide 7.

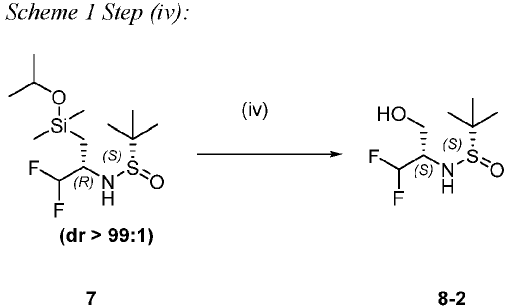

The distillate (196 g) of step (iii) containing (S)-N-((R)-1,1-difluoro-3-(isopropoxydimethylsilyl)propan-2-yl)-2-methylpropane-2-sulfinamide 7 was placed in a 1000 mL double glass jacked reactor equipped with a mechanical stirrer, thermometer, funnel, and nitrogen supply. The distillate was heated to between 40-50°C and potassium hydrogen carbonate (33.9 g), potassium fluoride (39.4 g), and tertbutylammonium hydrogen sulfate (5.9 g) were added. Hydrogen peroxide (49.58 g 35%) was dosed over at least 180 minutes. The pale yellow emulsion was aged for at least 30 minutes at a temperature between 40-55°C.

The biphasic mixture was cooled to 15-25°C, and sodium sulfite (4.27 g) was added over 30 minutes at temperature between 15-30°C. The reaction vessel was flushed with nitrogen to purge oxygen, and anhydrous acetonitrile (150 mL) was added with Celite 545 AW (15 g). The suspension was stirred for at least 30 minutes. The suspension was filtered. The filtrate was washed with anhydrous acetonitrile twice (35 mL).

The resulting triphasic mixture was allowed to separate for at least 15 minutes at 20-30°C. The lowest aqueous phase was drained, and the biphasic mixture was allowed to separate for 15 minutes. The oily middle phase was drained. The upper organic phase (290 mL, 271 g) was distilled at 40-50°C and reduced pressure between 90-240 mBar. Toluene (300 mL) was added during distillation. The collected distillate was 390 mL, weighing 324 g. Anhydrous acetonitrile (40 mL) was added to the distillate, and the mixture was warmed to 60-70°C. Product crystallized upon cooling to 35-40°C. The solution was filtered, and the solids rinsed with anhydrous acetonitrile. Toluene (100 mL) was added, and the mixture was distilled at 40-50°C at reduced pressure of 120-240 mBar. The collected distillate was 90-110 mL, weighing 127 g. Toluene (50 mL) was added and distillation continued. The distillate was cooled to 0-10°C over at least 120 minutes. The distillate was filtered. The solid filter cake was washed with toluene twice (50 mL, 25 mL), and crude (S)-N-((S)-1,1-difluoro-3-hydroxypropan-2-yl)-2-methylpropane-2-sulfinamide **8-2** (49.06 g) was obtained and dried at 40-50°C at 20 mBar. Pure (S)-N-((S)-1,1-difluoro-3-hydroxypropan-2-yl)-2-methylpropane-2-sulfinamide **8-2** (46.0 g) was obtained at 63% yield. ¹H NMR (400 MHz, DMSO-d6) δ 6.04 (td, *J =* 55.6, 3.3 Hz, 1H), 5.53 (d, *J =* 9.1 Hz, 1H), 4.96 (s, 1H), 3.54(dd, *J =* 6.3, 3.6 Hz, 2H), 3.43 (ddqd, *J* = 18.6, 9.4, 6.0, 3.0 Hz, 1H). ¹³C NMR (101 MHz, DMSO-d6) δ 115.90 (t, *J* = 242.5 Hz), 60.44, 59.02 (t, *J =* 20.5 Hz), 56.34, 22.86.

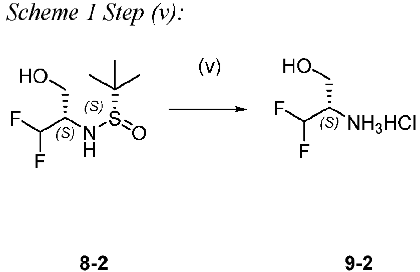

1-Propanol (32.7 g) was placed in a 200 ml double glass jacked reactor equipped with a mechanical stirrer, a thermometer, addition funnel and nitrogen supply at less than 20°C. Hydrochloric acid (gas, 9.0 g) was charged at less than 20°C below solvent niveau. Dry (S)-N-((S)-1,1-difluoro-3-hydroxypropan-2-yl)-2-methylpropane-2-sulfinamide **8-2** (45.0 g) was added in portions over 90 minutes, and the suspension was stirred for 30 minutes at 15-25°C. Crystallization spontaneously occurred. Toluene (20 mL) was added over 30 minutes, and the suspension was stirred for at least 30 minutes. The suspension was filtered. The filter cake was washed three times with toluene (total of 60 mL). The hydrochloride salt of (S)-N-((S)-1,1-difluoro-3-hydroxypropan-2-yl)-2-methylpropane-2-sulfinamide **9-2** was obtained at a mass of 31.6 g, which was then dried under vacuum (20 mBar) at 45°C. Dry hydrochloride salt of (S)-N-((S)-1,1-difluoro-3-hydroxypropan-2-yl)-2-methylpropane-2-sulfinamide **9-2** was obtained at a mass of 29.5G, with 99.7% purity and 96% yield. ¹H NMR (400 MHz, DMSO-d6) δ 8.78 (s, 3H), 6.31 (td, *J =* 54.3, 3.9 Hz, 1H), 5.63 (s, 1H), 3.88 - 3.66 (m, 2H), 3.57 (ddq, *J =* 14.6, 9.4, 4.8 Hz, 1H). ¹³C NMR (101 MHz, DMSO-*d*6) δ 114.20 (t, *J* = 238.9Hz), 63.56 (t, *J =* 4.6 Hz), 53.2 (dd, *J* = 24.2, 23.6 Hz).

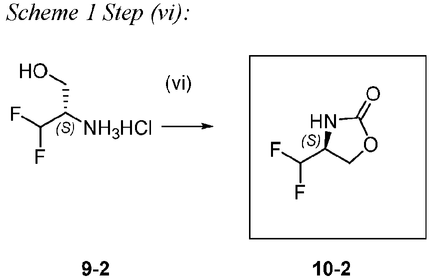

2,2,2-Trifluoroethanol (850 g) was placed in a 3500 ml double glass jacked reactor quipped with a mechanical stirrer, a thermometer, addition funnel and nitrogen supply at less than 30°C. 1,1'-Carbonyldiimidazole (652 g) was dosed in portions at temperature between 10-35°C over at least 60 minutes. The light brown suspension was warmed up to between 80-140°C (start: 90°C, end: 130°C) and distillation was performed at 200-270 mbar. 919 g, 620 ml distillate 1 is collected. The distillate was cooled to 80-100°C, and pharmaceutical grade water (15 g) was added over 60 minutes, followed by another 480 g over 15 minutes, followed by cooling the distillate to less than 30°C. The solution is Distillate 1.

2,2,2-Trifluoroethanol (911 g) was placed in a 3500 ml double glass jacked reactor quipped with a mechanical stirrer, a thermometer, addition funnel and nitrogen supply at less than 30°C. Dry hydrochloride salt of (S)-N-((S)-1,1-difluoro-3-hydroxypropan-2-yl)-2-methylpropane-2-sulfinamide **9-2** (330 g) was added, and the suspension was warmed to 40-55°C. Potassium carbonate (401 g) was added over 30 minutes, and the addition funnel was rinsed with 2,2,2-Trifluoroethanol (137 g).

Distillate 1 (833 g) was added over 60 minutes at 40-55°C, followed by stirring for at least 60 minutes. The suspension was cooled to 15-25°C and pharmaceutical grade water (990 g), hydrochloric acid (382 g, 33%) was added, and the pH was adjusted to between 5.8 to 6.2 with additional hydrochloric acid. The suspension was warmed to 40-55°C and distilled at 220-270 mBar. 1400-1600 mL of distillate was collected. The solution was cooled to 15-30°C (target: 25°C), and the pH was adjusted with hydrochloride acid. Water (150 g) and Isopropyl acetate (990 mL) were added and the biphasic colorless mixture was stirred for at least 15 minutes. Phases were separated for at least 5 minutes. The aqueous phase was extracted eleven times with Isopropyl acetate (each 330 ml) at 15-30°C. The mixture was stirred for at least 15 minutes for each extraction. Phases were separated for at least 5 minutes. Pure water (20 mL) was added when salt is precipitating. All organic extracts (4442 g, 4940 mL) were collected and combined. The organic layer was distilled at 35-55°C and pressure between 170-250 mBar. The distillate was filtered and washed with isopropyl acetate (200 mL). Seed crystals (100 mg) were added, if needed. The suspension was cooled to 0 to 10°C, and methyl cyclohexane (1815 mL) was added over 60 minutes. The suspension was aged for at least 30 minutes, followed by filtering twice with methyl cyclohexane (660 mL total). Wet (S)-4-(difluoromethyl)oxazolidin-2-one **10-2** (291 g) was dried at 25-35°C and 10 mBar pressure. The dried product was 283 g, with a purity near 100% and 92% yield. ¹H NMR (400 MHz, DMSO-*d*6) δ 8.26 (s, 1H), 6.09 (td, *J =* 55.3, 3.3 Hz, 1H), 4.41 (tt, *J* = 9.3, 1.1 Hz, 1H), 4.25 (dd, *J =* 9.3, 4.2 Hz, 1H), 4.22 - 4.08 (m, 1H). ¹³C NMR (101 MHz, DMSO-*d*6) δ159.07, 115.45 (t, *J =* 251.5 Hz), 63.56 (t, *J =* 4.7 Hz), 53.2 (t, *J* = 24.3 Hz).

### Scheme 1A

### Scheme 1A Step 1:

Difluoroacetaldehyde-ethylhemiacetal (72.8 kg, 1.05 eq), Ethanol (2 kg) and (S)-tert-butylsulfinamide (60.0 kg, 1.0 eq) were charged and the temperature was set to ≤ 25°C. Titanium(IV)ethoxide (119 kg, 1.0 eq) ad Ethanol (5 kg) was added and the temperature was increased to 80-90°C over a period of at least 90 minutes. The reaction mixture was stirred for at least 4 h at 80-90°C and the conversion was checked by LC. When the IPC limit was fulfilled ((S)-tertbutylsulfinamide ≤ 1.0%-a/a), the reaction mixture was cooled to 15-25°C and aged for at least two hours. Disappearance of hydrate impurity was checked by LC. When the IPC limit was fulfilled (hydrate step 1 ≤ 1.0%-a/a), the reaction mixture was quenched under adiabatic conditions on potassium citrate solution (95 kg, 1.0 eq citric acid; 71 kg, 1.27 eq KOH 50%; 240 kg water) at 30-40°C. The reactor vessel was rinsed with TBME (60 L). The quenched mixture was stirred for 60 minutes and the phases were separated. The upper organic phase was kept in a separate vessel and the lower aqueous phase was extracted once with TBME (60 L). The lower aqueous phase was drained and the two organic phases were combined. Toluene (120 L) was added to the combined organic phase and the mixture was stirred for 15 minutes. The newly formed aqueous layer was separated for 15 minutes and drained. Magnesium sulfate (42 kg, 0.71 eq) was added as suspension in toluene (72 L). Residual water was controlled by Karl-Fischer titration. When the IPC limit was fulfilled (water ≤ 2.0%-w/w), the suspension was filtered off and the filter cake was washed with toluene (2 x 36 L). Solvents were partially distilled off under reduced pressure at 30-45°C. Feed distillation with toluene (180 L) was performed to remove ethanol. Content of ethanol was checked by GC-HS (Ethanol ≤ 1.0%-w/w). Step 1 solution was diluted with toluene (60 L) and discharged over a filter cartridge and directly telescoped into the subsequent step.

### Scheme 1A Step 2:

Magnesium turnings (9.8 kg, 2.9 eq) and THF (255 kg) were charged and the suspension was warmed up to 50-65°C. 1,2-Dibromoethane (0.8 kg, 0.1 eq) was added and the mixture was stirred for at least 10 minutes while ethylene gas was formed. (Chloromethyl)dimethyl isopropyloxy silane (3.6 kg, 0.1 eq) was added over at least 20 minutes at 50-65°C (target: 60°C). Initiation of the reaction was checked by observation of heat formation. In case the increase of temperature is not clearly noticeable, the initiation of the reaction can be checked by GC ((chloromethyl)dimethyl isopropyloxy silane ≤ 5.0%-a/a). When the IPC limit was fulfilled (increase if internal temperature ≥ 3°C or conversion), dosage of the remaining (chloromethyl)dimethyl isopropyloxy silane (4 x 16.5 kg, 2.9 eq) was completed over at least four hours. The reaction mixture was aged for at least 60 minutes at 50-65°C. Complete consumption of magnesium turnings was checked by GC ((chloromethyl)dimethyl isopropyloxy silane ≥ 3.0%-a/a). If the IPC criteria is reached, the reaction mixture is cooled down to 0-10°C. The step 1 solution in toluene (74 kg, 1.0 eq) was added over at least 120 minutes at 0-10°C. An informative IPC was measured to check the reaction profile. In a second reactor the ammoniumcitrate solution was prepared and precooled to 10-20°C (48 kg, 1.8 eq citric acid; 50 kg, 5.3 eq ammonia 25%; 69 kg water). The reaction mixture from the first reactor was poured under adiabatic conditions on the ammoniumcitrate solution. The temperature of the the quench mixture reaches 34-45°C. THF was added (10 L). The lower aqueous layer was separated and drained. A solution of potassium hydrogen carbonate 5% (0.7 kg, 0.05 eq) in water (14 kg) was added. Most of the solvent was removed at 35-45°C and 100-250 mbar followed by a feed distillation with water (250 kg) to remove THF and volatile siloxane residues. Removal of THF was checked by GC-HS (THF ≤ 0.50%-w/w). A solution of citric acid (10 L, 10% in water) was added to adjust the pH to 5.5 at 35-45°C. The distillation was continued at 40-55°C and 50-150 mbar to remove isopropanol/water. Conversion of step 2 intermediate and removal of solvents was checked by LC and GC-HS (THF ≤ 0.50%-w/w, isopropanol ≤ 0.50%-w/w, step 2 intermediate ≤ 10%-a/a). If the IPC criteria is fullfilled the mixture is discharged over a filter cartridge to obtain step 2 as biphasic mixture with water. This mixture was directly telescoped into the subsequent step.

### Scheme 1A Step 3:

Step 2 (12.14 kg, 1.0 eq) as biphasic mixture with water was charged together with potassium hydrogen carbonate (2.17 kg, 1.0 eq), potassium fluoride (2.52 kg, 2.0 eq) and tetrabutylammonium hydrogen sulfate (0.37 kg, 0.05 eq). The mixture was warmed up to 40-50°C and hydrogen peroxide 35% (3.16 kg, 1.5 eq) was added over at least 180 minutes. The mixture was aged for at least 60 minutes. The conversion was checked with LC. When the IPC limit was fulfilled (step 2 and step 2 dimer < 5.0%-a/a), the reaction mixture was quenched with sodium sulfite (0.27 kg, 0.1 eq) at 40-50°C. The mixture was diluted with toluene (7.8 kg, 0.7 V) at 40-50°C. The biphasic turbid emulsion was cooled down to 35-45°C (target: 40°C) and the mixture was aged for at least 60 minutes to initiate the crystallization spontaneously. The suspension was cooled to 0-10°C over at least 180 min and stirred for at least 30 minutes. The product was isolated by filtration and the filter cake was washed with toluene (10 L, 0.6 V). Step 3, crude, wet was dried at 40-50°C under reduced pressure until the water content was < 1.0%-w/w. Step 3, crude, dry was obtained as off-white to orange solid with off-white inorganic salts (4.75 kg, 56% o. th., 99.2%-a/a and 62%-w/w).

### Scheme 1A Step 4:

Step 3 crude dry (4.75 kg, 1.0 eq, 62%-w/w) mixture with salts, acetonitrile (11.7 kg, 2.8 V) and toluene (1.3 kg, 0.27 V) were charged, and the mixture was warmed up to 40-50°C. The suspension was filtered to a second reactor and the filter cake was washed with acetonitrile (3.9 kg, 0.53 V). The solution was concentrated at 40- 50°C under reduced pressure (2 V distillate). The distillation was continued at 40-50°C under reduced pressure by feeding toluene (15.5 kg, 3.3 V) at a constant reactor level while a suspension was formed. The suspension was cooled down to 15-25°C and 1-propanol (2.47 kg, 3.0 eq) was added. Hydrogen chloride gas (0.55 kg, 1.1 eq.) was passed in at 15-25°C over at least 1 h and the suspension was aged for at least 30 minutes. The conversion was checked with GC. When the IPC limit was fulfilled (step 3 ^ 0.5%-a/a), the product was isolated by filtration and the filter cake was washed with toluene (5.7 kg, 1.2 V displacement). Step 4, pure, wet was dried at 40-50°C under reduced pressure until LOD < 0.40%-w/w and 1- propanol < 500 ppm was reached. Step 4, pure, dry was obtained as white to off-white solid (1.88 kg, 94% o. t. and 99.9%-a/a purity).

### Scheme 1A Step 5:

The reagent (bis(2,2,2-trifluoroethyl) carbonate was prepared according to the following procedure: 2,2,2-Trifluoroethanol (104 kg, 2.1 eq) was charged at JT ≤ 30°C. 1,1'-Carbonyldiimidazole (80 kg, 1.0 eq) was dosed in portions over at least 60 minutes at IT = 10-55°C. The thick suspension was heated up to IT = 80- 130°C and bis(2,2,2-trifluoroethyl) carbonate (BTFEC) was distilled off under reduced pressure (150- 300 mbar). The purity of BTFEC in the distillate was checked by GC (typically 90-93%-ala). The distillation residue was quenched by a small portion of water (1.8 kg, 1.8 L). Complete hydrolysis of the remaining bis(2,2,2-trifluoroethyl) carbonate in the distillation residue was controlled by GC (IPC bis(2,2,2-trifluoroethyl) carbonate ≤ 0.1%-a/a). When the IPC criterial was fulfilled, the quenched residue was diluted with water (57 kg, 57 L) and disposed.

The process for the oxazolidinone formation was performed according to the following procedure: Step 4, pure dry (15.0 kg, 1.0 eq) was suspended in 2,2,2-trifluoroethanol (45 kg, 32 L) at IT ≤ 30°C. The suspension was warmed up to IT = 40-55°C. Potassium carbonate powder (18.2 kg, 1.3 eq) was added in portions over at least 30 minutes at IT = 40-55°C. The addition funnel was rinsed with a small amount of 2,2,2-trifluoroethanol (2 L). Bis(2,2,2-trifluoroethyl) carbonate (34.4 kg, 1.5 eq) was dosed over at least 60 minutes at IT = 40-55°e. The suspension was additionally aged for at least 60 minutes. Conversion was checked by GC (Step 4 ≤ 0.5%-a/a). When the IPC limit was fulfilled, the mixture was cooled down to IT = 15-30°C and the reaction mixture quenched by the addition of water (44 kg, 44 L) at IT ≤ 30°C. The pH was adjusted to 5.5-6.5 with hydrochloric acid 33% (19.4 kg, ~1.6 eq). A fraction of solvent was distilled off at IT = 40-55°C under reduced pressure. The mixture was cooled down to IT = 15-30°C and the pH was checked and readjusted to 5.5-6.5 with a small amount of hydrochloric acid 33%. The aqueous product solution was extracted twelve times with isopropyl acetate (170 kg, 195 L). The combined organic layers were concentrated at IT = 40-55°C under reduced pressure. The concentrate was transferred to a second reactor over a filter cartridge. The filter cartridge was rinsed with a small amount of isopropyl acetate (8 L). The product was further concentrated at IT = 40-55°C under reduced pressure. The product solution was cooled down to IT = 35-40°C. The mixture was seeded if the crystallization has not initiated spontaneously. Initiation of the crystallization was controlled by visual inspection. After the crystallization has been initiated, the suspension was cooled down to IT 0-10°C over a period of at least 120 minutes. Then, methyl cyclohexane (69 kg, 89 L) was added over a period of at least 60 minutes. The suspension was aged for at least 30 minutes to complete the crystallization process. The product was isolated by centrifugation. The pure, wet was dried at 25-35°C under reduced pressure to afford the pure, dry as a white to off-white solid (23.4 kg, 89% o. th., 100%-a/a purity).

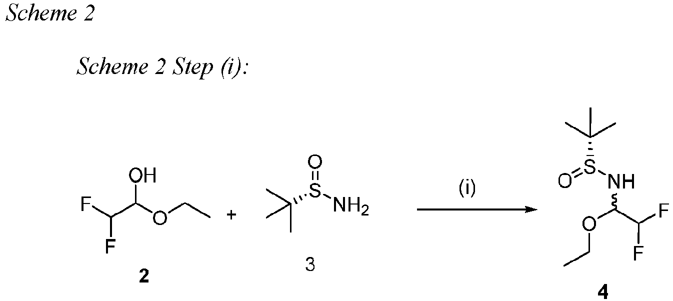

### (i) Ti(OEt)₄, neat, 60°C, 42% o. th.

(S)-*tert*-Butylsulfinamide **2** (10 g, 82 mmol, 1.0 eq), hemiacetal **3** (14.8 g, 116 mmol, 1.4 eq) and titanium ethoxide (26.3 g, 116 mmol, 1.4 eq) were mixed and heated to 60°C. Full conversion of **3** was obtained after 16 h (TLC). The solution was quenched on saturated brine (50 ml) and EtOAc (200 ml). The slurry was filtered over Celite (10 g). The phases were separated and the organic layer was dried over MgSO₄. The solvent was evaporated and the crude product was purified by column chromatography (EtOAc Cyclohexane 2:1). N,O-Acetal **4** was obtained as colorless solid (7.2 g, 42% o. th.). ¹H NMR (300 MHz, DMSO-*d*₆) δ 6.45 (d, *J =* 10.0 Hz, 1H), 5.83 (td, *J =* 55.4, 4.4 Hz, 1H), 4.65 - 4.41 (m, 1H), 3.88 (dq, *J =* 9.5, 7.1 Hz, 1H), 3.50 (dq, *J =* 9.5, 6.9 Hz, 1H), 1.17- 1.10 (m, 12H tBu).

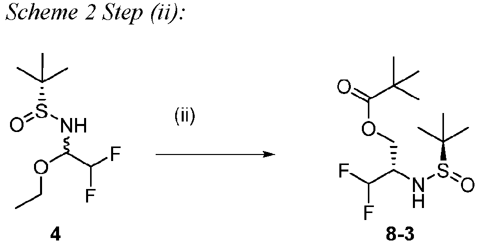

Iodomethyl pivalate (8.0 g, 33 mmol, 3.0 eq) was dissolved in a mixture of THF (50 ml) and NMP (10 ml). The solution was cooled down to -65°C. *i*PrMgCl 2.0 M solution in THF (19.0 ml, 38 mmol, 3.5 eq) was added at IT = -65°C over 30 min. Subsequently, the N,O-acetal **4** (2.5 g, 11 mmol, 1.0 eq) dissolved in THF (5 ml) was added over 30 min at - 65°C. The diastereoselectivity was 94:6 (NMR). The mixture was quenched on saturated aquouse NH₄Cl solution (50 ml). The aquouse layer was extracted with TBME. The organic layers were combined and dried over MgSO₄. The crude product was purifed by column chromatography to give pivalate **8-3** as greazy solid (2.46 g, 75% o. th.). ¹H NMR (300 MHz, DMSO-*d*₆) δ 6.09 (td, *J =* 55.1, 3.6 Hz, 1H), 5.81 (d, *J =* 9.3 Hz, 1H), 4.18 (dd, *J=* 11.5, 5.1 Hz, 1H), 4.08 (ddd, *J* = 11.5, 6.4, 1.1 Hz, 1H), 3.88 - 3.62 (m, 1H), 1.15 (s, 9H), 1.14 (s, 9H).

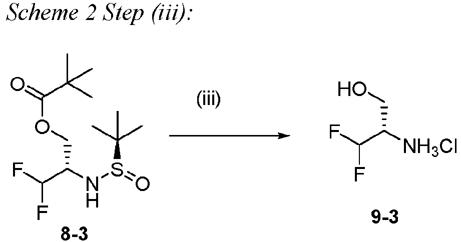

Pivalate **8-3** (1.0 g, 4.4 mmol, 1.0 eq) was mixed in HCl 33% (4 ml). The reaction mixture was heated to 80°C. Full conversion of pivalate **8-3** was obtained after 2 h (TLC: EtOAc). The mixture was concentrated and co-evaporated with MeOH, ACN and toluene. The remaining solid was suspended in TBME (5 ml) and filtered off. The amino alcohol hydrochloride **9-3** was obtained as greazy solid (0.64 g, 97% o. th. ¹H NMR (300 MHz, DMSO-*d*₆) δ 8.78 (s, 3H), 6.31 (td, 1H, *J =* 54.3 Hz, *J =* 3.9 Hz, H3), 5.63 (s, 1H, OH), 3.88 - 3.66 (m, 2H, H1), 3.65-3.50 (m, 1H, H2).

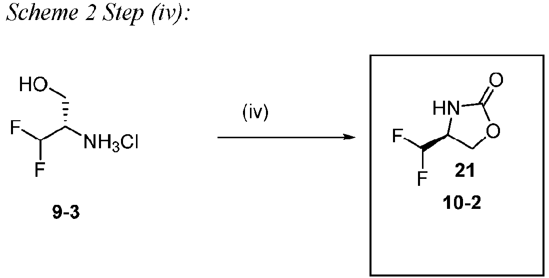

The amino alcohol hydrochloride **9-3** (0.60 g, 4.1 mmol, 1.0 eq) was mixed with triethylamine (1.2 ml, 8.2 mmol, 2.0 eq) and ACN (5 ml, 8 V) at room temperature. CDI (725 mg 4.5 mmol, 1.1 eq) was added at room temperature in one portion. Full conversion of **9-3** was obtained after 2 h (IPC: TLC BuOH, AcOH, water 5:1:1). The volatiles were evaporated and the crude product was purified by column chromatography. (S)-4-(difluoromethyl)oxazolidin-2-one **10-2** was obtained as pale yellow oil (308 mg, 55% o. th., single enantiomer). ¹H NMR (300 MHz, DMSO-*d*₆) δ = 8.26 (s, 1H), 6.09 (td, 1H, *J =* 55.3 Hz, *J =* 3.3 Hz, H4), 4.41 (tt, *J =* 9.3 Hz, *J =* 1.1 Hz, 1H), 4.25 (dd, *J* = 9.3 Hz, *J =* 4.2 Hz, H3), 4.22 - 4.08 (m, 1H, H2).

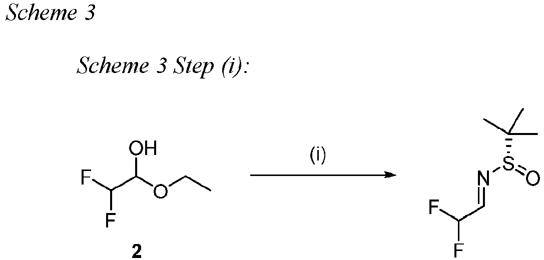

Hemiacetal **2** (5.0 g, 40 mmol, 1.0 eq) and (S)-tert-butylsulfinamide (4.8 g, 40 mmol, 1.0 eq) was dissolved in toluene (25 ml, 5 V). The mixture was refluxed with a Dean-Stark trap for 5 h. The solvent was distilled off. The crude product was purified by distillation under reduced pressure at 100°C. (*S*,*E*)-N-(2,2-difluoroethylidene)-2-methylpropane-2-sulfinamide was obtained as colorless liquid (1.1 g, 18% yield o. th). ¹H NMR (300 MHz, Chloroform-d) δ 8.07 (dt, *J =* 4.7, 3.1 Hz, 1H), 6.28 (td, *J =* 54.6, 4.7 Hz, 1H), 1.27 (s, 9H).

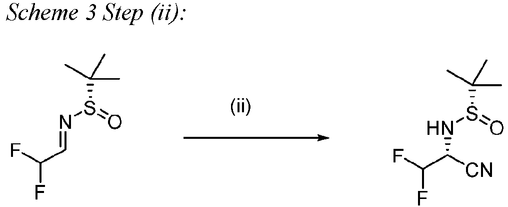

(S,E)-N-(2,2-difluoroethylidene)-2-methylpropane-2-sulfinamide (13 g, 71 mmol, 1.0 eq) was dissolved in DCM (130 ml, 10 V). Y(OTf)₃ (3.8 g, 7.1 mmol, 10 mol%) was added and the suspension was stirred for 15 min. TMSCN (18 ml, 142 mmol, 2.0 eq) was added over 30 min at room temperature. The Reaction mixture was stirred for 4 h until full conversion of (S,E)-N-(2,2-difluoroethylidene)-2-methylpropane-2-sulfinamide was reached (TLC: EtOAc). The reaction was quenched by the addition of water (50 ml). The organic layer was washed 2 x with water 50 ml and the solvent was distilled off. The diastereomeric ratio of the crude product was 5:1 (NMR). The crude product was purified by column chromatography (EtOAc cyclohexane 1:3 to 1:1). The diastereomerically pure (S)-N-((R)-1-cyano-2,2-difluoroethyl)-2-methylpropane-2-sulfinamide was obtained as light brown solid (11 g, 73% o. th). ¹H NMR (300 MHz, Chloroform-d) δ 5.95 (ddd, *J=* 55.0, 54.3, 3.2 Hz, 1H), 4.61 (dddd, *J* = 14.2, 9.2, 8.4, 3.2 Hz, 1H), 1.29 (s, 9H).

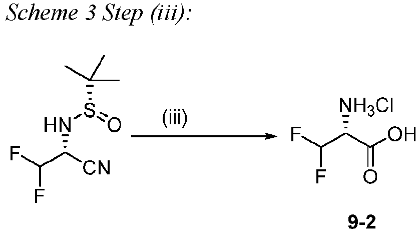

(S)-N-((R)-1-cyano-2,2-difluoroethyl)-2-methylpropane-2-sulfinamide (6.0 g, 29 mmol, 1.0 eq) was dissolved in 33% HCl (30 ml, 5 V). The mixture was heated gently to 80°C and stirred for 4 h until full conversion of the (S)-N-((R)-1-cyano-2,2-difluoroethyl)-2-methylpropane-2-sulfinamide(TLC: EtOAc). The volatiles were removed under reduced pressure. Methanol (10 V) was added and the suspension was stirred for 30 min at room temperature. The solid (NH₄Cl) was filtered off and the filter cake was washed with methanol (1.0 V). Methanol was removed and the residue was suspended in TBME (10 V) and stirred for 30 min at room temperature. The suspension was filtered off and the wet product was dried under reduced pressure. Amino acid hydrochloride **9-2** was obtained as light brown solid (4.3 g, 93% o. th.). ¹H NMR (300 MHz, Deuterium Oxide) δ 6.46 (td, *J =* 52.8, 1.9 Hz, 1H), 4.70 (s, 4H), 4.40 (dt, *J =* 25.8, 1.9 Hz, 1H).

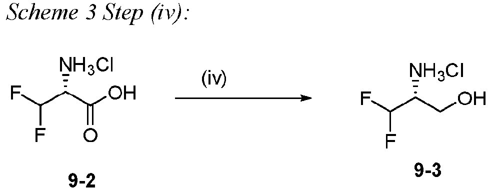

Amino acid hydrochloride **9-2** (1.0 g, 6.6 mmol, 1.0 eq) was suspended in THF (5 ml, 5 V). BH₃ 1.0 M in THF (20 ml, 3.0 eq) was added at 0°C over 30 min. Vigorous gas evolution was observed. The suspension was heated to 45°C for 2 h. Full conversion of the starting material was obtained after 2 h (IPC: TLC BuOH, AcOH, water 5:1:1). The mixture was quenched with methanol (5 ml, 5 V) and acetic acid (5 ml, 5 V) at room temperature. The volatiles were evaporated and the residue was taken up in HCl 33% (1.5 ml, 1.5 V). The volatiles were evaporated and the solid was dried under reduced pressure. The crude amino alcohol hydrochloride **9-3** was directly used for the next step. ¹H NMR (300 MHz, DMSO-*d*₆) δ 8.78 (s, 3H), 6.31 (td, 1H, *J =* 54.3 Hz, *J =* 3.9 Hz, H3), 5.63 (s, 1H, OH), 3.88 - 3.66 (m, 2H, H1), 3.65-3.50 (m, 1H, H2).

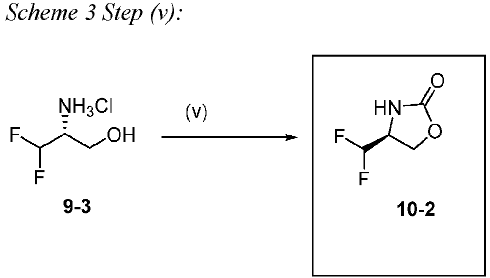

The crude mixture from amino alcohol hydrochloride **9-3** was mixed with triethylamine (0.92 ml, 18 mmol, 3.0 eq) and IPAc (5 ml, 5 V) at room temperature. CDI (1.24 g, 2.0 eq) was added at room temperature in one portion. Full conversion of amino alcohol **9-3** was obtained after 2 h (IPC: TLC 1-BuOH, AcOH, water 5:1:1). The volatiles were evaporated and the crude product was purified by column chromatography. (S)-4-(difluoromethyl)oxazolidin-2-one **10-2** was obtained as pale yellow oil (450 mg, 50% o. th. over two steps). The ratio of the enantiomers was 89:11. ¹H NMR (300 MHz, Chloroform-d) δ 6.33 (s, 1H), 5.71 (td, *J =* 55.3, 4.5 Hz, 1H), 4.46 (td, *J* = 9.2, 1.3 Hz, 1H), 4.35 (dd, *J =* 9.6, 4.5 Hz, 1H), 4.04 (ddq, *J* = 13.9, 9.3, 4.5 Hz, 1H).

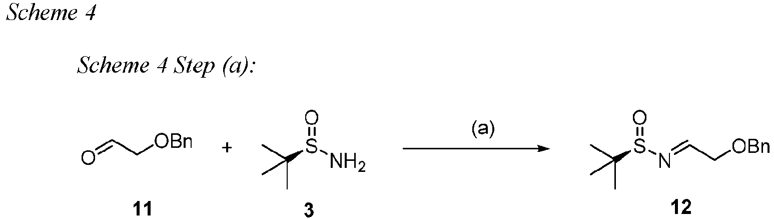

Benzyloxy acetaldehyde 11 (2.50 g, 16.7 mmol, 1.0 eq) and (R)-*tert*-butylsulfinamide 3 (2.15 g, 18.3mmol, 1.1 eq) were dissolved in DCM (25 ml, 10 V). Copper sulfate (6.44 g, 41.8 mmol, 2.5 eq) was added and the suspension was stirred for 16 h at 25°C until full conversion of **11** was reached (TLC: EtOAc heptane 1:1). Celite (10 g) was added and the suspension was filtered over silica (10 g). The filter cake was rinsed with DCM (50 ml, 20 V) and the solvents were evaporated. Sulfinimide **12** was obtained as yellow oil (4.05 g, quantitative yield). ¹H NMR (300 MHz, Chloroform-d) δ 8.06 (t, *J =* 3.3 Hz, 1H), 7.34 - 7.20 (m, 5H), 4.56 (s, 2H), 4.33 (dd, *J* = 3.2, 1.0 Hz, 2H), 1.14 (s, 9H).

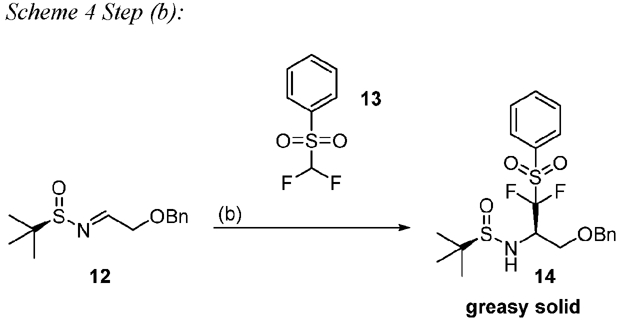

Sulfinimide **12** (600 mg, 2.4 mmol, 1.0 eq) and difluoromethylphenylsulfone **13** (500 mg, 2.6 mmol, 1.1 eq) were dissolved in THF (12 ml, 20 V). The solution was cooled down to -78°C. NaHMDS 40% in THF (1.3 g, 2.8 mmol, 1.2 eq) was added over 5 min at -78°C. The purple solution was stirred for 15 min. Full conversion was reached (TLC, EtOAc/heptane 1:1). The reaction mixture was quenched with saturated NaHCO₃ solution (20 ml). The aqueous layer was extracted with EtOAc (50 ml). The organic layers were dried over MgSO₄ and the solvent was evaporated. Sulfone **14** was obtained as a light brown oil (1.05 g, quantitative yield). ¹H NMR (300 MHz, Chloroform-d) δ 7.81 (d, *J =* 7.4 Hz, 2H), 7.65 - 7.55 (m, 1H), 7.46 (t, *J =* 7.7 Hz, 2H), 7.25 - 7.11 (m, 5H), 4.49 (d, *J =* 11.8 Hz, 1H), 4.41 (d, *J =* 11.8 Hz, 1H), 4.25 (ddddd, *J =* 15.2, 10.8, 9.2, 4.4, 3.3 Hz, 1H), 4.06 (d, *J =* 9.2 Hz, 1H), 3.90 (ddd, *J =* 10.5, 3.3, 1.4 Hz, 1H), 3.81 (dd, *J =* 10.5, 4.4 Hz, 1H), 1.12 (s, 9H).

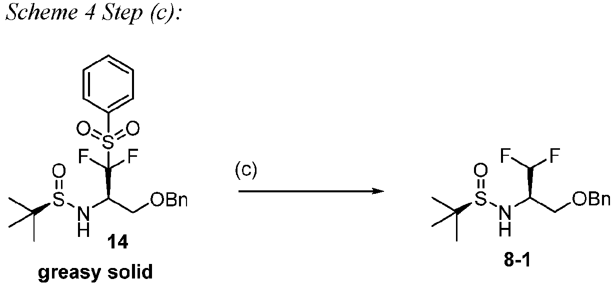

Sulfone **14** (7.03 g, 15.7 mmol, 1.0 eq) was dissolved in DMF (105 ml, 15 V) and acetate buffer (5.0 g acetic acid, 6.4 g NaOAc, 13 g water). Magnesium tunings (5.67 g, 23.5 mmol, 15 eq) were added in one portion and the suspension was stirred for 3 h at 30°C until full conversion of sulfone **14** was reached (TLC: EtOAc). Remaining magnesium tunings were filtered off and the reaction mixture was quenched on MTBE/water (2.0 V). The aqueous layer was extracted three times with MTBE (150 ml). The organic layers were combined and washed with water (50 ml). The volatiles were distilled off and the crude product was purified by column chromatography (EtOAc heptane 1:1 to 2:1). The sulfinamide **8-1** was obtained as single diasteromer (2.3 g, 48% o. t.). ¹H NMR (300 MHz, Chloroform-*d*) δ 7.40 - 7.14 (m, 5H), 5.75 (ddd, *J =* 56.4, 55.5, 4.7 Hz, 1H), 4.51 (d, *J* = 11.7 Hz, 1H), 4.43 (d, *J =* 11.8 Hz, 1H), 3.76 (ddd, *J =* 9.7, 3.6, 2.2 Hz, 2H), 3.69 - 3.61 (m, 1H), 3.55 (dddd, *J =* 11.9, 8.3, 4.9, 2.6 Hz, 1H), 1.16 (s, 9H).

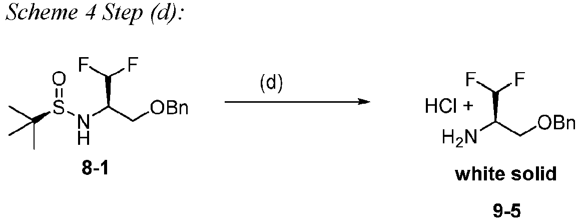

Sulfinamide **8-1** (2.00 g, 6.6 mmol, 1.0 eq) was dissolved in methanol (10 ml, 5.0 V). Hydrochloric acid 37% (0.65 ml, 7.9 mmol, 1.2 eq) was added at room temperature and the reaction mixture was stirred for 3 h until full conversion was reached (TLC: EtOAc heptane 1:1). The volatiles were distilled off. The residue was suspended in MTBE (20 ml, 10 V). The solid was filtered off and dried under vacuum. Benzyl ether **9-5** was obtained as white solid (1.23 g, 79% o. t.). ¹H NMR (300 MHz, DMSO-*d*₆) δ 8.93 (s, 3H), 7.45 - 7.27 (m, 5H), 6.37 (td, *J=* 54.1, 3.7 Hz, 1H), 4.57 (d, *J* = 2.7 Hz, 2H), 3.96 - 3.82 (m, 1H), 3.82 - 3.65 (m, 2H).

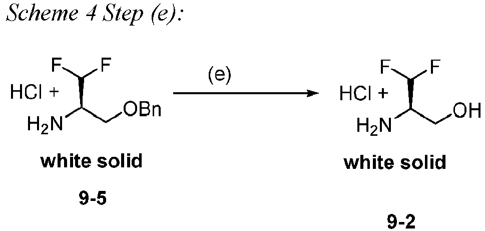

Benzyl ether **9-5** (1.1 g, 4.6 mmol, 1.0 eq) was dissolved in methanol (10 ml, 5.0 V) and Pd/C 5.0% (200 mg) was added. The tube was purged 3 x with hydrogen. The reaction mixture was stirred for 5 h under 20 bar hydrogen at room temperature until full conversion of benzyl ether **9-5** was obtained (TLC: DCM MeOH 20:1). The catalyst was filtered off and the solvent was distilled off. The residue was suspended in MTBE (20 ml, V). The solid was filtered off and dried under vacuum. The amino alcohol hydrochloride **9-2** was obtained as white solid (0.53 g, 78% yield o. th.). ¹H NMR (300 MHz, DMSO-*d*₆) δ 8.78 (s, 3H), 6.31 (td, 1H, *J =* 54.3 Hz, *J =* 3.9 Hz, H3), 5.63 (s, 1H, OH), 3.88 - 3.66 (m, 2H, H1), 3.65-3.50 (m, 1H, H2).

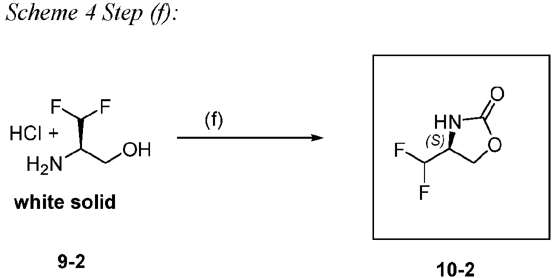

Amino alcohol hydrochloride **9-2** (0.44 g, 3.0 mmol, 1.0 eq) was suspended in THF (5 ml, 11V). DIPEA (1.15 ml, 9.0 mmol, 2.0 eq) was added and the suspension was stirred for 30 min at room temperature. CDI (0.72 g, 4.4 mmol, 1.5 eq) was added and the reaction mixture was stirred for 16 h at room temperature until full conversion of 8 was reached. The reaction mixture was absorbed on silica and purified by column chromatography. The enantiomer of (S)-4-(difluoromethyl)oxazolidin-2-one **10-2** was obtained as colorless oil (180 mg, 44% o. th., single enantiomer). ¹H NMR (300 MHz, Chloroform-d) δ 6.33 (s, 1H), 5.71 (td, *J =* 55.3, 4.5 Hz, 1H), 4.46 (td, *J* = 9.2, 1.3 Hz, 1H), 4.35 (dd, *J =* 9.6, 4.5 Hz, 1H), 4.04 (ddq, *J* = 13.9, 9.3, 4.5 Hz, 1H).

### Scheme 5

### Scheme 5 Step a:

Into a suspension of 2-(5-bromo-2-cyanophenoxy)ethan-1-aminium chloride **11'** (20.4 kg, 97.8 wt %, 71.9 mol, 100 mol %) in MeOH (64.0 kg), solid magnesium ethoxide, Mg(OEt)₂ (17.9 kg, 219 mol %) was charged. The mixture was agitated at 25 °C for 30 min and followed by the addition of 2-methyltetrahydrofuran, 2-MeTHF (140 kg), the reaction mixture was heated to reflux and stirred for 40 h. After the reaction was completed, the batch was concentrated to approximately 50 L under reduced pressure below 40 °C. Followed by the addition of 2-MeTHF (172 kg), a solution of hydrogen chloride in *n*-propanol (83.0 kg, 5.00 M) was added below 15 °C. The suspension was stirred at 15 °C for 4 h and filtered. The resulting solid was washed with 2-MeTHF (10 kg) and dried under reduced pressure at 50 °C to afford 8-bromo-2,3-dihydrobenzo[*f*][1,4]oxazepin-5-amine hydrochloride **12'** (17.6 kg, 88% yield) as a hygroscopic solid that was used as is for the next step. ¹H NMR (500 MHz, DMSO-*d*₆) δ 8.32 (s, 3H), 7.74 (d, *J =* 8.3 Hz, 1H), 7.61 (d, *J =* 1.5 Hz, 1H), 7.38 (dd, *J* = 8.3*,* 1.5 Hz, 1H), 4.44 (t, *J=* 5.2 Hz, 2H), 3.24 (t, *J* = 5.2 Hz, 2H).

### Scheme 5 Step b:

Into a mixture of 8-bromo-2,3-dihydrobenzo[*f*][1,4]oxazepin-5-amine hydrochloride **12'** (17.6 kg, 63.4 mol, 100 mol %) and 2-MeTHF (122 kg) were charged a 40% chloroacetaldehyde aqueous solution (16.4 kg, 132 mol %) and water (10 kg). The mixture was heated to 40 °C and aqueous potassium bicarbonate solution was charged. The reaction mixture was stirred at 45 °C for 21 h. After the reaction was complete, the reaction mixture was cooled to 20 °C, stirred for 30 min, and filtered. The resulting cake was rinsed with 2-MeTHF (33.0 kg) and the combined filtrates were allowed to settle. The resulting organic layer was washed with aqueous sodium bisulfite solution (30 kg), concentrated to approximately 26 L under reduced pressure below 45 °C. After the addition of DMF (25 kg), the mixture was concentrated to approximately 26 L under reduced pressure below 45 °C. Water (154 kg) was charged at 40 °C followed by the seed of 9-bromo-5,6-dihydrobenzo[*f*]imidazo[1,2-*d*][1,4]oxazepine **13'** (1.20 kg). The mixture was stirred at 40 °C for another 1.5 h and cooled to 20 °C. After stirring for 10 h at 20 °C, the suspension was filtered. The resulting solid was washed with water twice (25 kg × 2) and dried under reduced pressure at 45 °C to afford 9-bromo-5,6-dihydrobenzo[*f*]imidazo[1,2-d][1,4]oxazepine **13'** (16.3 kg, 97.5 wt %, 95% yield). ¹H NMR (500 MHz, DMSO-*d*₆) δ 8.33 (d, *J* = 8.6 Hz, 1H), 7.35 (s, 1H), 7.31-7.22 (m, 2H), 7.06 (s, 1H), 4.45 (q, *J* = 5.3 Hz, 4 H); HRMS calcd. For C₁₁H₁BrN₂O [M+H]⁺: 264.9971, found 264.9976.

### Scheme 5 Step c:

Into a solution of 9-bromo-5,6-dihydrobenzo[*f*]imidazo[1,2-*d*][1,4]oxazepine **13'** (16.3 kg, 97.5 wt %, 59.9 mol, 100 mol %) in DMF (78.0 kg) was added N-iodosuccinimide (NIS) (29.0 kg, 215 mol %) at 40 °C. The reaction mixture was slowly heated to 70 °C and stirred for 6 h. After the reaction was complete, 10% aqueous sodium sulfite solution (78.0 kg) was charged at 45 °C followed by water (154 kg). The resulting suspension was stirred at 45 °C for 1 h and cooled to 20 °C. After stirring at 20 °C for 8 h, the suspension was filtered. The resulting solid was washed with water (160 kg) and dried under reduced pressure at 45 °C to afford 9-bromo-2,3-diiodo-5,6-dihydrobenzo[*f*]imidazo[1,2-*d*][1,4]oxazepine **14'** (29.7 kg, 100 wt %, 96% yield) as an off-white solid. ¹H NMR (500 MHz, DMSO-*d*₆) δ 8.21 (d, *J =* 8.6 Hz, 1H), 7.32-7.24 (m, 2H), 4.51-4.45 (m, 2H), 4.39-4.34 (m, 2H); HRMS calcd. For C₁₁H₈BrI₂N₂O [M+H]⁺: 516.7904, found 516.7911.

### Scheme 5 Step d:

Into a solution of 9-bromo-2,3-diiodo-5,6-dihydrobenzo[*f*]imidazo[1,2-*d*][1,4]oxazepine **14'** (39.4 kg, 76.2 mol, 100 mol %) in tetrahydrofuran, THF (180 kg) was added a solution of 2.0 M ethylmagnesium bromide in 2-methyltetrahydrofuran (44.0 kg, 120 mol %) at 10 °C. The reaction mixture was stirred at 10 °C for 2 h. After the reaction was complete, 5% acetic acid (133 kg) was charged while maintaining the batch temperature below 30 °C. Ethyl acetate (168 kg) was charged and the resulting mixture was stirred at 20 °C for 1 h. The layers were separated and the aqueous layer was extracted with ethyl acetate (77.8 kg). The combined organic layers were washed with water (76.0 kg) and filtered through a pad of silica gel (19.8 kg). The silica gel pad was rinsed with ethyl acetate (69.6 kg). The combined filtrates were concentrated to approximately 100 L under reduced pressure below 50 °C and THF (146 kg) was added. The resulting mixture was heated to 60 °C until a clear solution was obtained before it was concentrated to approximately 100 L under reduced pressure below 50 °C and then cooled to 30 °C. *n*-Heptane was charged (86.8 kg) and the resulting mixture was stirred at 30 °C for 2 h. The batch was solvent-switched to n-heptane by three cycles of batch concentration under reduced pressure below 35 °C to approximately 180 L and n-heptane addition (47.6 kg × 3). The resulting suspension was cooled to 20 °C, stirred for 12 h, and filtered. The resulting solid was washed with *n*-heptane (64.0 kg) and dried under reduced pressure at 45 °C to afford 9-bromo-2-iodo-5,6-dihydrobenzo[*f*]imidazo[1,2-*d*][1,4]oxazepine **15** (25.3 kg, 98.7 wt %, 84% yield) as a light tan solid.. ¹H NMR (500 MHz, DMSO-*d*₆) δ 8.23 (d, *J =* 8.6 Hz, 1H), 7.55 (s, 1H), 7.32-7.24 (m, 2H), 4.44 (q, *J =* 5.4 Hz, 4H); HRMS calcd. For C₁₁H₉BrIN₂O [M+H]⁺: 390.8937, found 390.8949.

### Scheme 5 Step e:

9-Bromo-2-iodo-5,6-dihydrobenzo[*f*]imidazo[1,2-*d*][1,4]oxazepine **15** (6.90 kg, 98.7 wt %, 17.4 mol, 100 mol %) was charged to a reactor, followed by (S)-4-(difluoromethyl)oxazolidin-2-one **(10-2)** (2.68 kg, 112 mol %), copper (II) acetate (0.653 kg, 20.6 mol %), and Cs₂CO₃ (11.7 kg, 206 mol %). The reactor was evacuated and backfilled with nitrogen three times. 2-Methyltetrahydrofuran (36.0 kg) and *trans*-*N*,*N*-dimethylcyclohexane-1,2-diamine (0.764 kg, 30 mol %) was then charged into the reactor. The reactor was evacuated and backfilled with nitrogen three times. The reaction mixture was heated to 78 °C and stirred for 22 h. After the reaction was complete, a 20 wt % NaHSO₄ aqueous solution (42.0 kg) was slowly added while maintaining the internal temperature between 60-70 °C. The layers were separated at 65 °C and the aqueous layer was removed. The batch was solvent-switched to acetonitrile via a constant volume distillation under reduced pressure at 60-70 °C by adding acetonitrile (62.3 kg). Water (14.1 kg) was added into the reactor while maintaining the batch temperature between 60-70 °C. The suspension was cooled to 20 °C at a rate of 0.5 °C/min, stirred for 18 h, and filtered. The resulting solid was washed with a mixture of acetonitrile and water (50 kg, 44:56, w/w) and dried under reduced pressure at 90 °C to afford (S)-3-(9-bromo-5,6-dihydrobenzo[f]imidazo[1,2-d][1,4]oxazepin-2-yl)-4-(difluoromethyl)oxazolidin-2-one **16** as a tan solid (5.85 kg, 91.9 wt %, 77% yield). ¹H NMR (500 MHz, CDCl₃) δ 8.22 (d, *J =* 8.8 Hz, 1H), 7.31 (s, 1H), 7.28-7.19 (m, 2H), 6.71-6.62 (m, 1H), 4.90 (ddd, *J =* 24.0, 9.3, 3.8 Hz, 1H), 4.75 (dd, *J =* 9.4, 3.9 Hz, 1H), 4.56 (t, *J =* 9.3 Hz, 1H), 4.51-4.44 (m, 2H), 4.41-4.35 (m, 2H); HRMS calcd. For C₁₅H₁₃BrF₂N₃O₃ [M+H]⁺: 400.0103, found 400.0134.

### Scheme 5 Step f :

(*S*)-3-(9-bromo-5,6-dihydrobenzo[*f*]imidazo[1,2-d][1,4]oxazepin-2-yl)-4-(difluoromethyl) oxazolidin-2-one **16** (3.96 kg, 91.9 wt %, 9.19 mol, 100 mol %) was charged to a reactor, followed by (*S*)-2-aminopropanoic acid (L-alanine) (2.49 kg, 307 mol %), K₃PO₄ (5.84 kg, 303 mol %), and DMSO (19.9 kg). The mixture was sparged with nitrogen for 1 h and heated to 95 °C. A slurry of copper (I) oxide (67.1 g, 5.16 mol %) in DMSO (2.21 kg) that was pre-sparged with nitrogen for 30 min was then transferred to the reactor The reaction mixture was stirred at 95 °C for 4 h. After the reaction was complete, the reaction mixture was cooled to 20 °C. DCM (37.3 kg) was added to the reactor, followed by water (24.2 kg). The layers were separated and the organic layer was removed. The aqueous layer was washed with dichloromethane, DCM (26.6 kg) one more time. THF (35.2 kg) and an aqueous sodium bisulfate solution (19 wt %, 20.7 kg) were charged to the reactor sequentially. The layers were separated and the aqueous layer was removed. The organic layer was washed with 15 wt % brine (2 × 12 kg). SiliaMetS^{®} DMT (Silicycle Inc., 1.60 kg) was charged and the batch was stirred at 25 °C for 15 h and filtered to scavenge residual metal. SiliaMetS^{®} DMT is the silica-bound equivalent of 2,4,6-trimercaptotriazine (trithiocyanuric acid, TMT), and a versatile metal scavenger for a variety of metals including ruthenium catalysts and hindered Pd complexes. Tetrahydrofuran, THF (24.8 kg) was used to rinse the filter. The combined filtrates were heated to 50 °C. A 7 N solution of ammonia in methanol (1.02 kg, 100 mol %) was added followed by a slurry of seeds (ammonium (*S*)-2-((2-((S)-4-(difluoromethyl)-2-oxooxazolidin-3-yl)-5,6-dihydrobenzo[*f*]imidazo[1,2-*d*][1,4]oxazepin-9-yl)amino)propionate **17,** 19.5 g) in THF (0.395 kg). The resulting suspension was stirred at 50 °C for 2 h and a constant volume distillation was conducted at 40-60 °C under reduced pressure to remove residual water by adding anhydrous THF (60.1 kg). A 7 N solution of ammonia in methanol (1.02 kg, 100 mol %) was added. The suspension was stirred at 50 °C for 15 h and filtered. The resulting solid was washed with THF (21.8 kg) and dried under reduced pressure at 25 °C to afford ammonium (*S*)-2-((2-((*S*)-4-(difluoromethyl)-2-oxooxazolidin-3-yl)-5,6-dihydrobenzo[*f*]imidazo[1,2-*d*][1,4]oxazepin-9-yl)amino)propionate **17** as a beige solid (3.19 kg, 98.0 wt %, 81% yield). ¹H NMR (DMSO-d₆) δ 7.97 (d, *J* = 8.8Hz, 1H), 7.16 (s, 1H), 6.74 - 6.69 (m, 1H), 6.38 (dd, *J =* 9.0, 2.2 Hz, 1H), 6.07 (d, *J =* 2.2Hz, 1H), 5.02 - 4.91 (m, 1H), 4.64 - 4.52 (m, 2H), 4.40 - 4.30 (m, 4H), 3.63 (q, *J =* 6.1, 5.5Hz, 1H), 1.27 (d, *J* = 6.7Hz, 3H). HRMS calcd. For C₁₈H₁₉F₂N₄O₅ [M+H]⁺: 409.1318, found 409.1318.

### Scheme 5 Step g:

Ammonium (*S*)-2-((2-((*S*)-4-(difluoromethyl)-2-oxooxazolidin-3-yl)-5,6-dihydrobenzo[*f*]imidazo[1,2-*d*][1,4]oxazepin-9-yl)amino)propionate **17** (5.60 kg, 13.2 mol, 100 mol %) was charged to a reactor, followed by *N*-hydroxysuccinimide, HOSu (1.52 kg, 102 mol %) and THF (49.6 kg). The batch was sparged with nitrogen for 40 min and cooled to 10 °C. A 2 N solution of ammonia in 2-propanol (5.05 kg, 101 mol %) and *N*-(3-dimethylaminopropyl)-*N*'-ethylcarbodiimide hydrochloride, EDC (5.20 kg, 210 mol %) were charged sequentially to the reactor. The reaction mixture was stirred at 10 °C for 20 h. After the reaction was complete, the mixture was warmed up to 20 °C and 15 wt % brine (33.7 kg) was added. The layers were separated at 35 °C and the aqueous layer was removed. The organic layer was washed sequentially with 15 wt % brine (2 × 16.9 kg) and a mixture of 15 wt % brine (8.97 kg) and 28.0-30.0 wt % ammonium hydroxide (7.55 kg) and then filtered through a polishing filter unit. The filter unit was rinsed with THF (5.05 kg). The combined filtrates were distilled under reduced pressure at 50 °C to approximately half of its original volume. Ethanol (8.90 kg) was charged at 50 °C, followed by a slurry of seeds ((*S*)-2-((2-((*S*)-4-(difluoromethyl)-2-oxooxazolidin-3-yl)-5,6-dihydrobenzo[*f*]imidazo[1,2-d][1,4]oxazepin-9-yl)amino)propanamide **18**, 27.1 g) in ethanol (0.340 kg). The resulting suspension was stirred at 50 °C for 30 min and solvent-switched to ethanol via a constant volume distillation under reduced pressure at 40-60 °C by adding ethanol (39.9 kg). Water (0.379 kg) was added at 50 °C. The suspension was cooled to 20 °C, stirred for 23 h, and filtered. The resulting solid was washed with a 90:10 (w/w) mixture of ethanol and water (27.9 kg) and dried under reduced pressure at 80 °C to afford (*S*)-2-((2-((*S*)-4-(difluoromethyl)-2-oxooxazolidin-3-yl)-5,6-dihydrobenzo[*f*]imidazo[1,2-*d*][1,4]oxazepin-9-yl)amino)propanamide **18** as a light pink solid (4.37 kg, 99.7 wt %, 83% yield). ¹H NMR (600 MHz, CD₃CN) δ 8.08 (d, *J* = 8.8 Hz, 1H), 7.11 (s, 1H), 6.86-6.50 (m, 1H), 6.41 (dd, *J =* 8.8, 2.3 Hz, 1H), 6.12 (d, *J =* 2.4 Hz, 1H), 4.87 (dd, *J =* 23.8, 8.8 Hz, 1H), 4.67-4.50 (m, 2H), 4.43-4.33 (m, 2H), 4.33-4.26 (m, 2H), 3.82 (q, *J* = 7.0 Hz, 1H), 1.41 (d, *J* = 7.0 Hz, 3H) (Note: N-H protons were omitted for clarity); ¹³C NMR (151 MHz, CD₃CN) δ 178.2, 157.0, 155.1, 149.1, 141.6, 135.4, 130.8, 113.3, 108.9, 108.1, 107.7, 102.1, 68.5, 61.7, 56.1, 53.1, 49.6, 18.2; HRMS calcd. For C₁₈H₂₀F₂N₅O₄ [M+H]⁺: 408.1478, found 408.1473.

### Scheme 5 (Alternatives)

### Scheme 5 Step b (Alternative)

Into a mixture of 8-bromo-2,3-dihydrobenzo[*f*][1,4]oxazepin-5-amine hydrochloride (40.00 g, 144 mmol) and 2-MeTHF (444 g, 520 mL) were charged a 46% chloroacetaldehyde aqueous solution (39.27 g, 231 mmol, 1.6 equiv.) and water (20 mL). The mixture was heated to 65 °C and solution of potassium bicarbonate (45.45 g, 454 mmol, 3.15 equiv) in water (161 mL) was added over 2 h. The reaction mixture was stirred at 65 °C for 0.5 h. The aqueous layer was separated and the resulting organic layer was concentrated to approximately 200 mL under reduced pressure. Ethanol (200 mL, 156 g) was added and the resulting mixture was concentrated to approximately 200 mL under reduced pressure. Ethanol (200 mL, 156 g) was added and the resulting mixture was concentrated to approximately 200 mL under reduced pressure. Ethanol (200 mL, 156 g) was added and the resulting mixture was concentrated to approximately 200 mL under reduced pressure and warmed to 50 °C. To the resulting solution water (200 g, 200 mL) was added over 1.5 h followed by the seed of 9-bromo-5,6-dihydrobenzo[*f*]imidazo[1,2-d][1,4]oxazepine (200 mg). The mixture was stirred at 50 °C for another 1.0 h and cooled to 0 °C over 6 h. After stirring for min. 1 h at 0 °C, the suspension was filtered. The resulting solid was washed with water three times (50 mL x 3) and dried under reduced pressure at 50 °C to afford 9-bromo-5,6-dihydrobenzo[*f*]imidazo[1,2-*d*][1,4]oxazepine (33.9 g, 100.0 wt %, 89% yield).

### Scheme 5 Step c (Alternative)

Into a solution of 9-bromo-5,6-dihydrobenzo[*f*]imidazo[1,2-*d*][1,4]oxazepine (20 g, 75.4 mmol) in MeCN (139 g, 177 mL) was added iodine (19.15 g, 75.4 mmol, 1.0 equiv.), sodium periodate (9.68 g, 45.3 mmol, 0.6 equiv), and MeCN (10 g, 17.2 mL) at 25 °C. Aqueous sulfuric acid 10% (37.00 g, 75.4 mmol, 1.0 equiv) was added over 0.5 h. The reaction mixture was heated to 60 °C over 0.5 h and stirred for 13 h before it was cooled to 30 °C over 0.5 h. A solution of sodium sulfite (18.54 g, 147 mmol, 1.95 equiv.) in water (210 g, 210 mL) was added over 2 h. The resulting suspension was stirred at 30 °C for min. 1 h and filtered. The resulting solid was washed with water twice (2x 40g) and dried under reduced pressure at 50 °C to afford 9-bromo-2,3-diiodo-5,6-dihydrobenzo[*f*]imidazo[1,2-*d*][1,4]oxazepine (36.8 g, 100.0 wt %, 94.4% yield).

### Scheme 5 Step d (Alternative)

Into a mixture of 9-bromo-2,3-diiodo-5,6-dihydrobenzo[*f*]imidazo[1,2-*d*][1,4]oxazepine (1.30 kg, 2.51 mol, 1.0 equiv) and toluene (11.3 kg) was added a solution of 24% ethylmagnesium bromide in 2-methyltetrahydrofuran (2.00 kg, 3.60 mmol, 1.4 equiv.) at -10 °C over 1.5 h. The reaction mixture was stirred at -10°C for 1 h before it was transferred onto a solution of 80% acetic acid (1.04 kg, 13.9 mmol, 5.5 equiv.) in water (7.2 kg) at 15-20 °C over 1 h. The mixture was heated to 60 °C before the aqueous phase was separated and the organic phase was washed with water twice (2 x 7.2 kg). The resulting organic layer was concentrated to approximately 6.5 L under reduced pressure. After the solution was heated to 85 °C, heptane (14.3 kg) was added over 1.5 h. The mixture was cooled to 10 °C over 8 h. After stirring for min. 1 h at 0 °C, the suspension was filtered. The resulting solid was washed with heptane twice (2 x 2.7 kg) and dried under reduced pressure at 50 °C to afford 9-bromo-2-iodo-5,6-dihydrobenzo[*f*]imidazo[1,2-*d*][1,4]oxazepine **15** (0.94 kg, 98.9 wt %, 95.8% yield).

### Scheme 5 Step e (Alternative)

A suspension of 9-bromo-2-iodo-5,6-dihydrobenzo[*f*]imidazo[1,2-*d*][1,4]oxazepine (15.00 g, 38.34 mmol, 1.0 equiv.), (S)-4-(difluoromethyl)oxazolidin-2-one (5.78 g, 42.2 mmol, 1.1 equiv), *trans*-*N*,*N*-dimethylcyclohexane-1,2-diamine (0.818 g, 5.75 mmol, 0.15 equiv), and Cs₂CO₃ (31.2 g, 95.9 mmol, 2.5 equiv) in 2-Methyltetrahydrofuran (120 mL, 102 g) was thoroughly purged with argon. Copper(I) iodide (0.365 g, 1.92 mmol, 0.05 equiv.) was then added and the reaction mixture was heated to 70 °C and stirred for 46 h. The mixture was cooled to 60 °C, and diluted with THF (120 mL) before 5% aqueous solution of NH₄OH (44 mL) was added. The phases were separated and the organic phase was washed with 5% aqueous solution of NH₄OH twice (2 x 44 mL). The resulting organic layer was concentrated to approximately 90 mL under reduced pressure. The distillation was continued with continuous addition of acetonitrile (200 mL) at constant volume. The resulting suspension was heated to 60 °C and water (35 g) was added over 20 min. The mixture was cooled to 20 °C over 1.5 h. After stirring for min. 1 h at 20 °C, the suspension was filtered. The resulting solid was washed with a mixture of acetonitrile (39 g) and water (18 g) in three portions and dried under reduced pressure at 50 °C to afford (*S*)-3-(9-bromo-5,6-dihydrobenzo[*f*]imidazo[1,2-*d*][1,4]oxazepin-2-yl)-4-(difluoromethyl)oxazolidin-2-one (13.79 g, 100.5 wt %, 90% yield).

### Scheme 5 Step f (Alternative)

(*S*)-3-(9-bromo-5,6-dihydrobenzo[*f*]imidazo[1,2-d][1,4]oxazepin-2-yl)-4-(difluoromethyl) oxazolidin-2-one (33 g, 81.1 mmol, 1.0 equiv.) was charged to a reactor, followed by (*S*)-2-aminopropanoic acid (L-alanine) (21.69 g, 243.4 mmol, 3.0 equiv.), Cu(I) oxide (0.290 g, 2.0 mmol, 0.025 equiv), and K₃PO₄ (51.67 g, 243.4 mmol, 3.0 equiv). The reactor was evacuated and backfilled with nitrogen three times. DMSO (167 mL, 183 g) was added and the reactor was evacuated and backfilled with nitrogen three times. The mixture was heated to 95 °C. A slurry of copper (I) oxide (67.1 g, 5.16 mol %) in DMSO (2.21 kg) that was pre-sparged with nitrogen for 30 min was then transferred to the reactor. The reaction mixture was stirred at 95 °C for 6 h. After the reaction was complete, the reaction mixture was cooled to 20 °C. A solution of Ammonium pyrrolidinedithiocarbamate (12 mmol, 0.15 eq.) dissolved in (212 mL) Water and (232 mL) 2-MeTHF was added and the mixture was stirred for 2 h. The lowest of three liquid phases was separated and the mixture was filtered. The upper organic pahse was discarded and the lower aqueous phase was washed with 2-MeTHF (132 mL). The the aquous phase was added 2-MeTHF(660 mL) and a 20% aquous solution of sodium hydrogen sulfate (171.5 g). The mixture was stirred for 20 min and filtered and the filter was rinsed with 2-MeTHF (99 mL). The aqueous phase was separated. To The resulting organic phase was added acetonitrile (99 mL), a solution of ammonia in methanol (7N, 3.4 mL, 24 mmol, 0.3 equiv) and the seed of ammonium (*S*)-2-((2-((*S*)-4-(difluoromethyl)-2-oxooxazolidin-3-yl)-5,6-dihydrobenzo[f]imidazo[1,2-d][1,4]oxazepin-9-yl)amino)propionate (10 mg). The mixture was stirred for 2 h before additional solution of ammonia in methanol (7N, 14.0 mL, 98 mmol, 1.2 equiv) was added over 2 h. The resulting suspension was stirred for min. 12 h and filtered. The resulting solid was washed with 2-MeTHF twice (2 x 200 mL) and dried under reduced pressure at 50 °C to afford ammonium (*S*)-2-((2-((*S*)-4-(difluoromethyl)-2-oxooxazolidin-3-yl)-5,6-dihydrobenzo[*f*]imidazo[1,2-*d*][1,4]oxazepin-9-yl)amino)propionate (29.9 g, 87% yield).

### Scheme 5 Step g (Alternative)

To a suspension of ammonium (*S*)-2-((2-((*S*)-4-(difluoromethyl)-2-oxooxazolidin-3-yl)-5,6-dihydrobenzo[*f*]imidazo[1,2-*d*][1,4]oxazepin-9-yl)amino)propionate (25.0 g, 58.8 mmol, 1.0 equiv.)) in THF (250 mL) was added N-hydroxysuccinimide (1.35 g, 11.8 mmol, 0.2 equiv), ammonium bicarbonate (2.32 g, 29.4 mmol, 0.5 equiv.), *N,N*-Diisopropylcarbodiimid (8.90 g, 10.99 mL, 70.5 mmol, 1.2 equiv.), and N-methylmorpholine (4.16 g, 4.57 mL, 41.1 mmol, 0.7 equiv.). The mixture was stirred for 16 h at 25 °C. A 10% aqueous solution of sodium chloride (150 mL) was added and the mixture was heated to 40 °C. The aqueous phase was separated and the organic layer was washed with a mixture of 10% aqueous solution of sodium chloride (80 mL) and 5% aqueous solution of sodium hydrogen carbonate (40 mL) twice. The organic solution was washed with 10% aqueous solution of sodium chloride (80 mL) and heated to 50 °C, and concentrated to approximately 125 mL under reduced pressure. 1-Propanol (125 mL) was added and the resulting mixture was concentrated to approximately 125 mL under reduced pressure. 1-Propanol (125 mL) was added and the resulting mixture was concentrated to approximately 125 mL under reduced pressure and warmed to 50 °C. The suspension was cooled to 20 °C over 2 h, stirred for 4 h, and filtered. The resulting solid was washed with a 1-propanol (75 mL), water (75 mL), and 1-propanol (75 mL) and dried under reduced pressure at 60 °C to afford (*S*)-2-((2-((*S*)-4-(difluoromethyl)-2-oxooxazolidin-3-yl)-5,6-dihydrobenzo[*f*]imidazo[1,2-*d*][1,4]oxazepin-9-yl)amino)propanamide (20.18 g, 97.8 wt%, 82% yield).

### Scheme 5 Step d - Continuous flow process

A continuous flow process consisted of simultaneous addition of 9-bromo-2,3-diiodo-5,6-dihydrobenzo[*f*]imidazo[1,2-*d*][1,4]oxazepine (compound **14**') (1.00 equiv, 0.223 M in THF) and EtMgBr (1.45 equiv, 40.0 wt% in MeTHF) in pipe reactor 1 (JT 10 °C, Tᵣₑₛ ca. 30 s), followed by aqueous acetic acid (2.25 equiv, 14.5 wt% in water) in pipe reactor 2 (JT 10 °C, Tᵣₑₛ ca. 30 s). The biphasic reaction mixture exiting pipe reactor 2 was directed through a heat exchanger to the receiving tank. The quenched reaction mixture was collected over a specified period of time and yield was calculated based on the flow rate of compound **14'** (mmol/min) and run time. The biphasic reaction mixture from the continuous process was diluted with toluene, extracted with an aqueous solution of NaHCO₃ and water. The organic phase was concentrated, anti-solvent heptane was added and the product 9-bromo-2-iodo-5,6-dihydrobenzo[*f*]imidazo[1,2-*d*][1,4]oxazepine (compound **15**) was filtered and dried under vacuum to yield compound **15** as a pink powder in 92 - 96% yield.

### Analysis of ¹⁴C labeled inavolisib

(2S)-2-[[2-[(4S)-4-(difluoromethyl)-2-keto-oxazolidin-3-yl]-5,6-dihydro[2-¹⁴C]imidazolo[1,2-d][1,4]benzoxazepin-9-yl]amino]propionamide (14.4 mCi, 107.7 mg of light brown, beige solid) was made according to Scheme 6 and analyzed by HPLC.

HPLC Method: Column: XBridge C18; 3.5 µm (3.0 x 100 mm). Mobile Phase A: water/acetonitrile 95:5 + 0.1% Phosphoric acid. Mobile Phase B: acetonitrile. Conditions: 0% B, 0-2 min; 0-15% B, 2-18 min; 15-90% B, 18-26 min; 90% B 26-28 min. Flow rate: 0.8 mL/min. Temperature: 35 °C.

The UV purity (λ: 330 nm) was 98.8% (retention time: 13.4 min) and radiochemical purity (β-Ram detector) was 98.5% [1.5% of diastereoisomer was detected (retention time: 14.60 min)].

The identity and purity of the material were proven by HPLC analysis by co-injection with the non-labeled reference standard.

Mass spectroscopy analysis with in flow injection was performerd. MS (ESI) m/z [¹⁴C-M + H]⁺ 410.15, [¹²C-M + H]⁺ 408.15

The compound shows 89% (by MS measurement) and 87.48% (by gravimetric analysis) of ¹⁴C isotopic enrichment. The specific activity was measured by gravimetric analysis and determined to be 133.35 µCi/mg (4933.95 kBq/mg), 54.6 mCi/mmol.

Although the foregoing invention has been described in some detail by way of illustration and example for purposes of clarity of understanding, the descriptions and examples should not be construed as limiting the scope of the invention. Accordingly, all suitable modifications and equivalents may be considered to fall within the scope of the invention as defined by the claims that follow.

## Claims

1. A compound of Formula (8A): 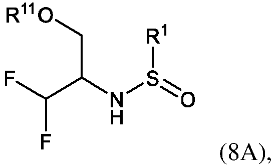 or a salt thereof, wherein:
R¹ is an optionally substituted C₁₋₁₂ alkyl, an optionally substituted C₃₋₁₄ cycloalkyl, or an optionally substituted C₆₋₁₄ aryl; and
R¹¹ is hydrogen or a hydroxyl protecting group, wherein the hydroxyl protecting group is selected from the group consisting of acetyl, trimethylacetyl, benzyl: 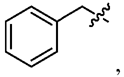 and silyl ethers including trimethylsilyl, triethylsilyl, triiso-propylsilyl, tert-butyldimethylsilyl, *tert-*butyldiphenylsilyl, and di-*tert*-butylmethylsilyl.

2. The compound of claim 1, wherein R¹ is selected from the group consisting of *tert*-butyl, *tert*-pentyl, 3-ethylpentan-3-yl, 1-methylcyclohexyl, 1-adamantyl, phenyl, and naphthyl and wherein R¹¹ is hydrogen or benzyl.

3. The compound of claim 1, wherein the compound is of Formula (8-1) or
Formula (8-2): 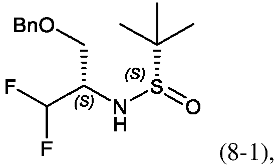 or a salt thereof; or 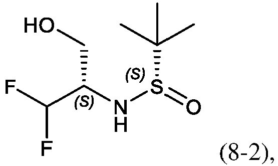 or a salt thereof.

4. A compound of Formula (7A): 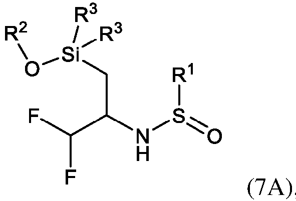 , or a salt thereof,
wherein:
R¹ is an optionally substituted C₁₋₁₂ alkyl, an optionally substituted C₃₋₁₄ cycloalkyl, or an optionally substituted C₆₋₁₄ aryl;
R² is an optionally substituted C₁₋₁₂ alkyl or an optionally substituted C₆₋₁₄ aryl; and
each R³ is independently an optionally substituted C₁₋₁₂ alkyl, an optionally substituted C₆₋₁₄ aryl, or OR².

5. The compound of claim 4, wherein the compound is of the formula (7): 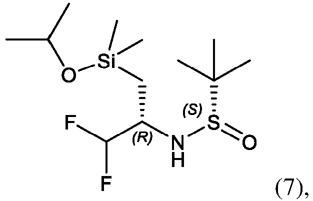 or a salt thereof.

6. A process of preparing a compound of Formula (8C): 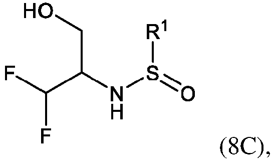 or a salt thereof,
wherein:
R¹ is an optionally substituted C₁₋₁₂ alkyl, an optionally substituted C₃₋₁₄ cycloalkyl, or an optionally substituted C₆₋₁₄ aryl;
the process comprising the steps of:
(iii) reacting a compound of Formula (4A): 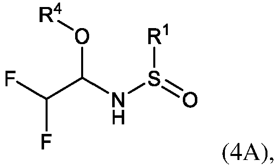
or a salt thereof,
wherein:
R¹ is an optionally substituted C₁₋₁₂ alkyl, an optionally substituted C₃₋₁₄ cycloalkyl, or an optionally substituted C₆₋₁₄ aryl; and
R⁴ is an optionally substituted C₁₋₆ alkyl or hydrogen;
with a Grignard reagent of formula (5A): 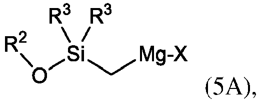 wherein:
R² is an optionally substituted C₁₋₁₂ alkyl or an optionally substituted C₆₋₁₄ aryl;
each R³ is independently an optionally substituted C₁₋₁₂ alkyl, an optionally substituted C₆₋₁₄ aryl, or OR²; and
X is a halide;
to thereby form a compound of Formula (7A): 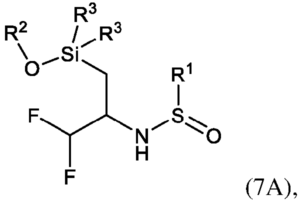 or a salt thereof,
and
(iv) reacting the compound of Formula (7A) with a fluoride salt, a base, and an oxidant to form the compound of Formula (8C).

7. The process of claim 6 further comprising the steps of:
(i) partially reducing a compound of Formula (1A): 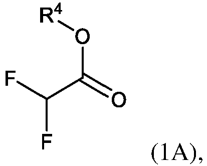 or a salt thereof,
wherein R⁴ is an optionally substituted C₁₋₆ alkyl or hydrogen, to form a compound of Formula (2A): 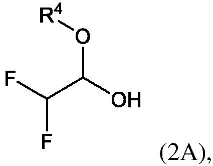 or a salt thereof,
and
(ii) reacting the compound of Formula (2A) with a sulfonamide compound of Formula (3A): 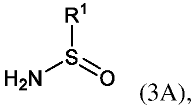 wherein R¹ is an optionally substituted C₁₋₁₂ alkyl, an optionally substituted C₃₋₁₄ cycloalkyl, or an optionally substituted C₆₋₁₄ aryl, in the presence of a dehydrating reagent
to form the compound of Formula (4A): 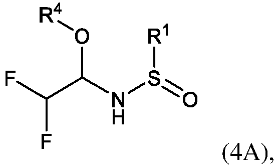 or a salt thereof.

8. The process of claim 6 or claim 7, further comprising the step of:
(v) reacting the compound of Formula (8C): 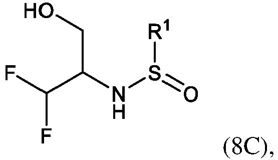 or a salt thereof,
wherein R¹ is as defined in claim 6, with an acid to thereby yield an amine compound of Formula (9-1): 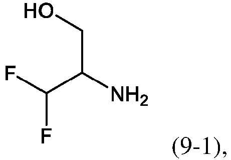 or an acid addition salt thereof.

9. The process of claim 8, further comprising the step of:
(vi) reacting the compound of Formula (9-1), or the acid addition salt thereof, with an acylating reagent to form a compound of Formula (10-1): 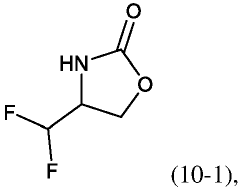 or a salt thereof.

10. The process of any one of claims 6 to 9, wherein R¹ is *tert*-butyl; R² is 2-propyl, each R³ is methyl, X is chloride; and R⁴ is ethyl.

11. The process of any one of claims 6 to 10, comprising the steps of:
(iii) reacting a compound of formula (4): 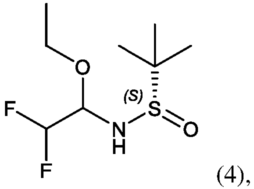 or a salt thereof, with a compound of formula (5): 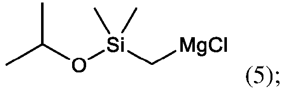 to form a compound of formula (7): 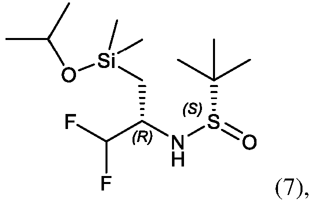 or a salt thereof; and
(iv) reacting the compound of formula (7) with potassium fluoride, potassium bicarbonate, and hydrogen peroxide to form a compound of formula (8-2):

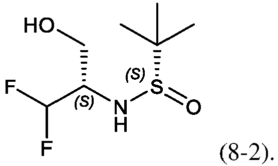

12. The process of any one of claims 6 to 11, wherein the fluoride salt is potassium fluoride and the base is potassium bicarbonate for step (iv).

13. The process of any one of claims 8 to 12, wherein the acid for step (v) is HCl, and the acid addition salt of the compound of Formula (9-1) is a hydrochloride salt having the structure (9-2):

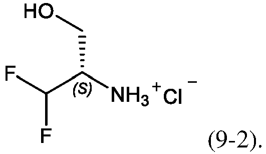

14. A process of making a compound of formula (10-2) according to the following sequence of steps:

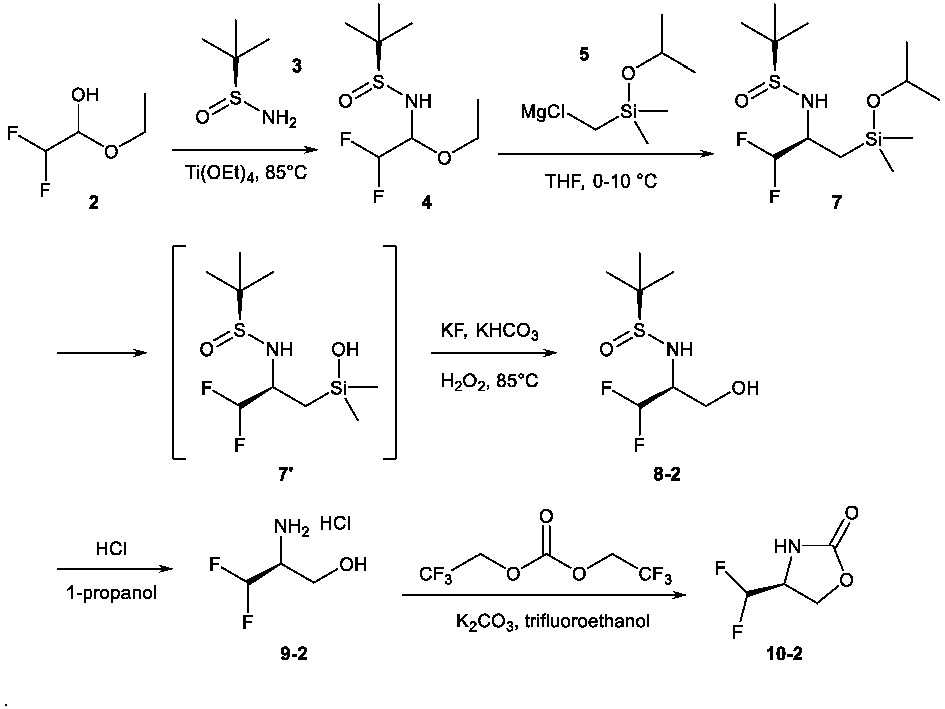

15. A process of preparing a compound of Formula (8A): 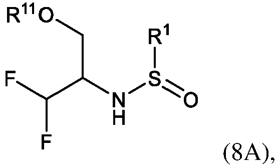 or a salt thereof, wherein:
R¹ is an optionally substituted C₁₋₁₂ alkyl, an optionally substituted C₃₋₁₄ cycloalkyl, or an optionally substituted C₆₋₁₄ aryl; and
R¹¹ is a hydroxyl protecting group, the process comprising the steps of:
(b) reacting a compound of Formula (12A): 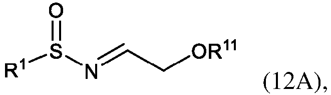 with a compound of Formula (13A): 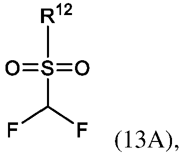 wherein R¹² is optionally substituted C₆₋₁₄ aryl and a base at a temperature below 0°C to form a compound of Formula (14A): 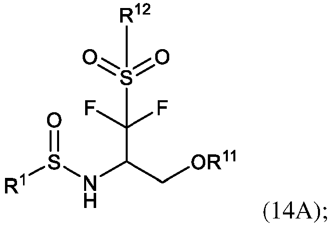 and
(c) reacting the compound of Formula (14A) with magnesium in the presence of an acetate buffer to thereby form the compound of Formula (8A), wherein the hydroxyl protecting group is selected from the group consisting of acetyl, trimethylacetyl, benzyl: 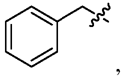 and silyl ethers including trimethylsilyl, triethylsilyl, triiso-propylsilyl, *tert-*butyldimethylsilyl, *tert*-butyldiphenylsilyl, and di-*tert*-butylmethylsilyl.

16. The process of claim 15, further comprising the step of:
(a) reacting a compound of Formula (11A): 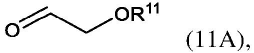 with a sulfonamide compound of Formula (3A) : 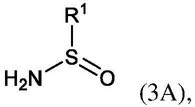 in the presence of a dehydrating reagent to form the compound of Formula (12A): 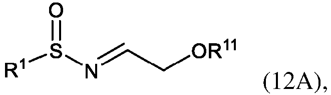 wherein R¹ and R¹¹ are as defined in claim 15.

17. The process of claim 15 or 16, further comprising the step of:
(d) reacting the compound of Formula (8A): 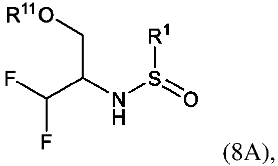 or a salt thereof,
with an acid to yield an amine compound of Formula (9A): 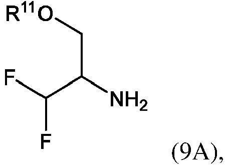 or an acid addition salt thereof,
wherein R¹ and R¹¹ are as defined in claim 15.

18. The process of claim 17, further comprising the steps of:
(e) removing the hydroxyl protecting group of the compound of Formula (9A) to form a compound of Formula (9-1): 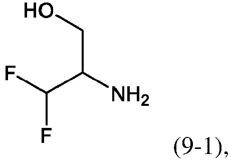 or an acid addition salt thereof,
and
(f) reacting the compound of Formula (9-1), or an acid addition salt thereof, with an acylating reagent to form a compound of Formula (10-1):

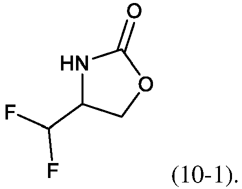

19. The process of any one of claims 15 to 18, wherein the compound of Formula (12A) is of Formula (12B): 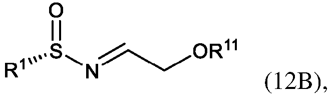 the compound of Formula (14A) is of Formula (14B): 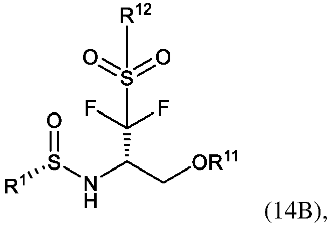 and the compound of Formula (8A) is of Formula (8B): 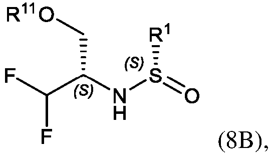 or a salt thereof,
wherein R¹, R¹¹, and R¹² are as defined in claim 15.

20. The process of any one of claims 16 to 19, wherein the compound of Formula (3A) is of Formula (3B): 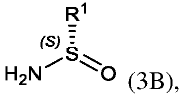 wherein R¹ is as defined in claim 16.

21. The process of any one of claims 17 to 20, wherein the compound of Formula (9A) is of Formula (9B): 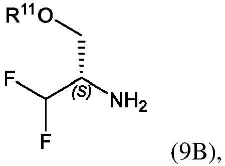 or a salt thereof,
wherein R¹¹ is as defined in claim 17.

22. The process of any one of claims 18 to 21, wherein the acid in step (d) is HCl and the acid addition salt of the compound of Formula (9A) or (9B) is a hydrochloride salt having the structure (9C):

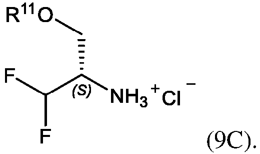

23. The process of any one of claims 18 to 22, wherein the compound of Formula (9-1) is of Formula (9-3): 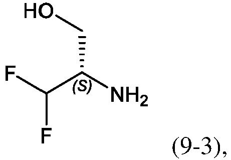 or the acid addition salt thereof;
and the compound of Formula (10-1) is of Formula (10-2):

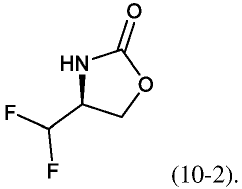

24. The process of any one of claims 10-14 and 18-23, further comprising reacting a compound of Formula (10-1) or compound (10-2) having the structures: 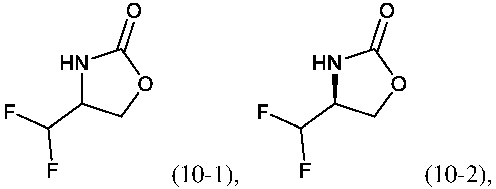 with compound **15**, having the structure: 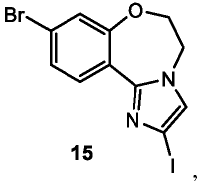 a copper salt and a ligand to form compound **16,** having the structure:

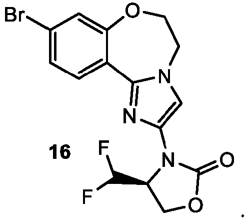

25. The process of claim 24, wherein the copper salt is copper(II) acetate or copper (I) iodide and the ligand is trans-*N*,*N*-dimethylcyclohexane-1,2-diamine.

26. The process of claim 24 or 25, further comprising reacting compound **16** with (S)-2-aminopropanoic acid and a copper (I) catalyst to form compound **17,** having the structure:

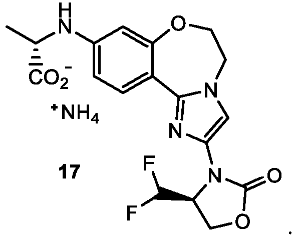

27. The process of claim 26, wherein the copper (I) catalyst is copper(I) oxide.

28. The process of claim 26 or 27, further comprising reacting compound **17** with ammonia or an ammonia equivalent and a peptide coupling reagent to form compound **18**, having the structure:

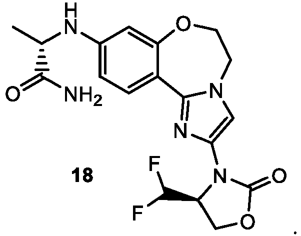

29. A process of preparing a compound of Formula (8A): 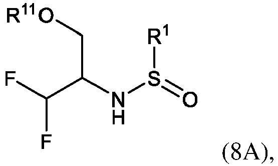 or a salt thereof, wherein:
R¹ is an optionally substituted C₁₋₁₂ alkyl, an optionally substituted C₃₋₁₄ cycloalkyl, or an optionally substituted C₆₋₁₄ aryl; and
R¹¹ is a hydroxyl protecting group, the process comprising the steps of:
(ii) reacting a compound of Formula (4A): 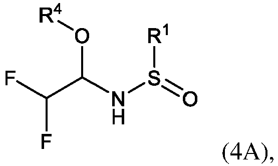 or a salt thereof,
wherein R⁴ is an optionally substituted C₁₋₆ alkyl or hydrogen;
with a Grignard reagent, to thereby prepare the compound having formula (8-A).

30. The process of claim 29, wherein the Grignard reagent is prepared by reacting iodomethyl pivalate with sec-butylmagnesium chloride.

31. The process of claim 29, further comprising:
(iii) hydrolyzing the compound having formula (8-A) an acid to thereby yield an amine compound of Formula (9-1): 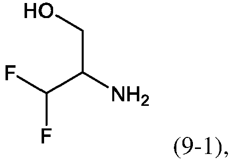 or an acid addition salt thereof.

32. A process for preparing a compound of Formula (9-1): 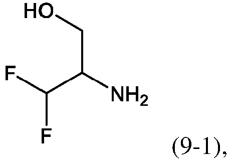 or an acid addition salt thereof;
the process comprising:
(i) reacting a compound of Formula (2A) 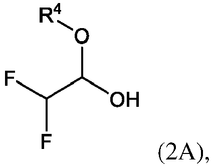 or a salt thereof;
wherein R⁴ is an optionally substituted C₁₋₆ alkyl or hydrogen;
with (S)-2-methylpropane-2-sulfinamide to thereby prepare (S,E)-N-(2,2-difluoroethylidene)-2-methylpropane-2-sulfinamide having structure: 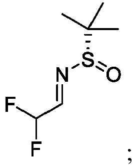
(ii) reacting (S,E)-N-(2,2-difluoroethylidene)-2-methylpropane-2-sulfinamide with trimethylsilyl-cyanide to give the aminonitrile (S)-N-((S)-1-cyano-2,2-difluoroethyl)-2-methylpropane-2-sulfinamide having the structure: 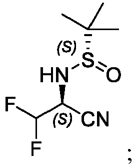
(iii) hydrolyzing (S)-N-((S)-1-cyano-2,2-difluoroethyl)-2-methylpropane-2-sulfinamide in acid to give the product (S)-2-(chloro-λ⁵-azaneyl)-3,3-difluoropropanoic acid: 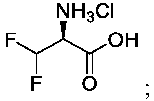 and
(iv) reducing (S)-2-(chloro-λ⁵-azaneyl)-3,3-difluoropropanoic acid to provide the intermediate compound of Formula (9-1) or the acid addition salt thereof.

## Patentansprüche

1. Verbindung der Formel (8A): 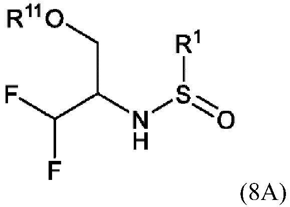 oder ein Salz davon, wobei:
R¹ ein gegebenenfalls substituiertes C₁₋₁₂-Alkyl, ein gegebenenfalls substituiertes C₃₋₁₄-Cycloalkyl oder ein gegebenenfalls substituiertes C₆₋₁₄-Aryl ist; und
R¹¹ Wasserstoff oder eine Hydroxylschutzgruppe ist, wobei die Hydroxylschutzgruppe ausgewählt ist aus der Gruppe bestehend aus Acetyl, Trimethylacetyl, Benzyl: 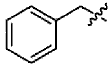 und Silylethern, einschließlich Trimethylsilyl, Triethylsilyl, Triisopropylsilyl, *tert-*Butyldimethylsilyl, *tert*-Butyldiphenylsilyl und Di-*tert*-butylmethylsilyl.

2. Verbindung nach Anspruch 1, wobei R¹ ausgewählt ist aus der Gruppe bestehend aus *tert*-Butyl, *tert*-Pentyl, 3-Ethylpentan-3-yl, 1-Methylcyclohexyl, 1-Adamantyl, Phenyl und Naphthyl und wobei R¹¹ Wasserstoff oder Benzyl ist.

3. Verbindung nach Anspruch 1, wobei die Verbindung die Formel (8-1) oder
Formel (8-2) aufweist: 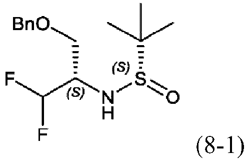 oder ein Salz davon; oder 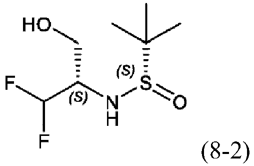 oder ein Salz davon.

4. Verbindung der Formel (7A): 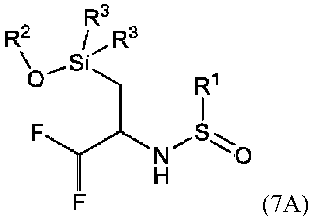 oder ein Salz davon,
wobei:
R¹ ein gegebenenfalls substituiertes C₁₋₁₂-Alkyl, ein gegebenenfalls substituiertes C₃₋₁₄-Cycloalkyl oder ein gegebenenfalls substituiertes C₆₋₁₄-Aryl ist;
R² ein gegebenenfalls substituiertes C₁₋₁₂-Alkyl oder ein gegebenenfalls substituiertes C₆₋₁₄-Aryl ist; und jedes R³ unabhängig voneinander ein gegebenenfalls substituiertes C₁₋₁₂-Alkyl, ein gegebenenfalls substituiertes C₆₋₁₄-Aryl oder OR² ist.

5. Verbindung nach Anspruch 4, wobei die Verbindung die Formel (7) aufweist: 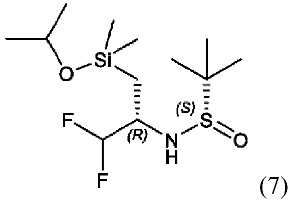 oder ein Salz davon ist.

6. Verfahren zur Herstellung einer Verbindung der Formel (8C): 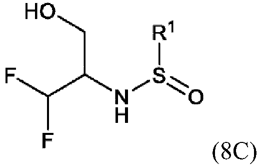 oder eines Salzes davon,
wobei:
R¹ ein gegebenenfalls substituiertes C₁₋₁₂-Alkyl, ein gegebenenfalls substituiertes C₃₋₁₄-Cycloalkyl oder ein gegebenenfalls substituiertes C₆₋₁₄-Aryl ist;
wobei das Verfahren die folgenden Schritte umfasst:
(iii) Umsetzen einer Verbindung der Formel (4A): 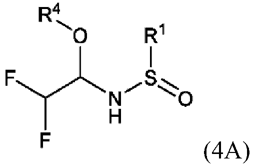 oder eines Salzes davon,
wobei:
R¹ ein gegebenenfalls substituiertes C₁₋₁₂-Alkyl, ein gegebenenfalls substituiertes C₃₋₁₄-Cycloalkyl oder ein gegebenenfalls substituiertes C₆₋₁₄-Aryl ist; und
R⁴ ein gegebenenfalls substituiertes C₁₋₆-Alkyl oder Wasserstoff ist;
mit einem Grignard-Reagens der Formel (5A): 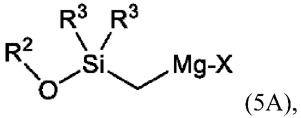 wobei:
R² ein gegebenenfalls substituiertes C₁₋₁₂-Alkyl oder ein gegebenenfalls substituiertes C₆₋₁₄-Aryl ist;
jedes R³ unabhängig voneinander ein gegebenenfalls substituiertes C₁₋₁₂-Alkyl, ein gegebenenfalls substituiertes C₆₋₁₄-Aryl oder OR² ist; und
X ein Halogenid ist;
um dadurch eine Verbindung der Formel (7A): 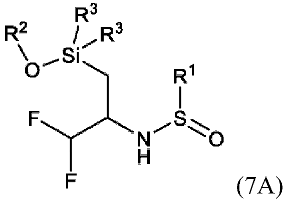 oder ein Salz davon zu bilden,
und
(iv) Umsetzen der Verbindung der Formel (7A) mit einem Fluoridsalz, einer Base und einem Oxidationsmittel, um die Verbindung der Formel (8C) zu bilden.

7. Verfahren nach Anspruch 6, ferner umfassend die folgenden Schritte:
(i) teilweises Reduzieren einer Verbindung der Formel (1A): 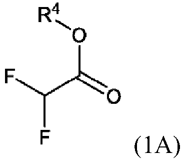 oder eines Salzes davon,
wobei R⁴ ein gegebenenfalls substituiertes C₁₋₆-Alkyl oder Wasserstoff ist, um eine Verbindung der Formel (2A): 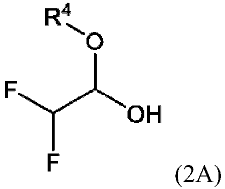 oder ein Salz davon zu bilden,
und
(ii) Umsetzen der Verbindung der Formel (2A) mit einer Sulfonamidverbindung der Formel (3A): 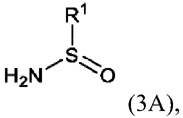 wobei R¹ ein gegebenenfalls substituiertes C₁₋₁₂-Alkyl, ein gegebenenfalls substituiertes C₃₋₁₄-Cycloalkyl oder ein gegebenenfalls substituiertes C₆₋₁₄-Aryl ist, in Gegenwart eines Dehydratisierungsreagens, um die Verbindung der Formel (4A): 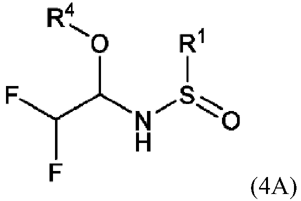 oder ein Salz davon zu bilden.

8. Verfahren nach Anspruch 6 oder Anspruch 7, ferner umfassend den folgenden Schritt:
(v) Umsetzen der Verbindung der Formel (8C): 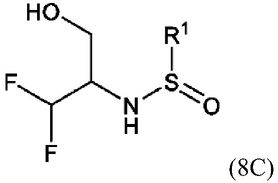 oder eines Salzes davon,
wobei R¹ wie in Anspruch 6 definiert ist, mit einer Säure, um dadurch eine Aminverbindung der Formel (9-1): 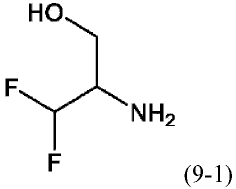 oder ein Säureadditionssalz davon zu erhalten.

9. Verfahren nach Anspruch 8, ferner umfassend den folgenden Schritt:
(vi) Umsetzen der Verbindung der Formel (9-1) oder des Säureadditionssalzes davon mit einem Acylierungsreagens, um eine Verbindung der Formel (10-1): 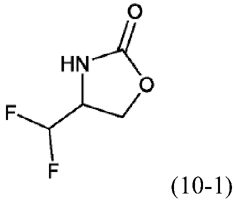 oder ein Salz davon zu bilden.

10. Verfahren nach einem der Ansprüche 6 bis 9, wobei R¹ *tert-*Butyl ist, R² 2-Propyl ist, jedes R³ Methyl ist, X Chlorid ist und R⁴ Ethyl ist.

11. Verfahren nach einem der Ansprüche 6 bis 10, umfassend die folgenden Schritte:
(iii) Umsetzen einer Verbindung der Formel (4): 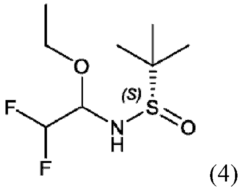 oder eines Salzes davon
mit einer Verbindung der Formel (5): 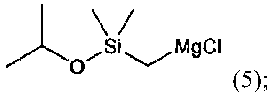 um eine Verbindung der Formel (7): 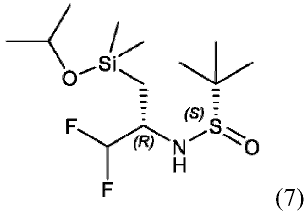 oder ein Salz davon zu bilden;
und
(iv) Umsetzen der Verbindung der Formel (7) mit Kaliumfluorid, Kaliumbicarbonat und Wasserstoffperoxid, um eine Verbindung der Formel (8-2): 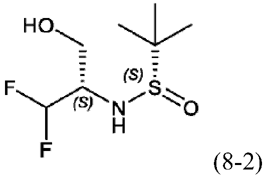 zu bilden.

12. Verfahren nach einem der Ansprüche 6 bis 11, wobei für Schritt (iv) das Fluoridsalz Kaliumfluorid ist und die Base Kaliumbicarbonat ist.

13. Verfahren nach einem der Ansprüche 8 bis 12, wobei die Säure für Schritt (v) HCl ist und das Säureadditionssalz der Verbindung der Formel (9-1) ein Hydrochloridsalz mit der Struktur (9-2) ist:

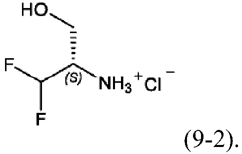

14. Verfahren zur Herstellung einer Verbindung der Formel (10-2) gemäß der folgenden Sequenz von Schritten:

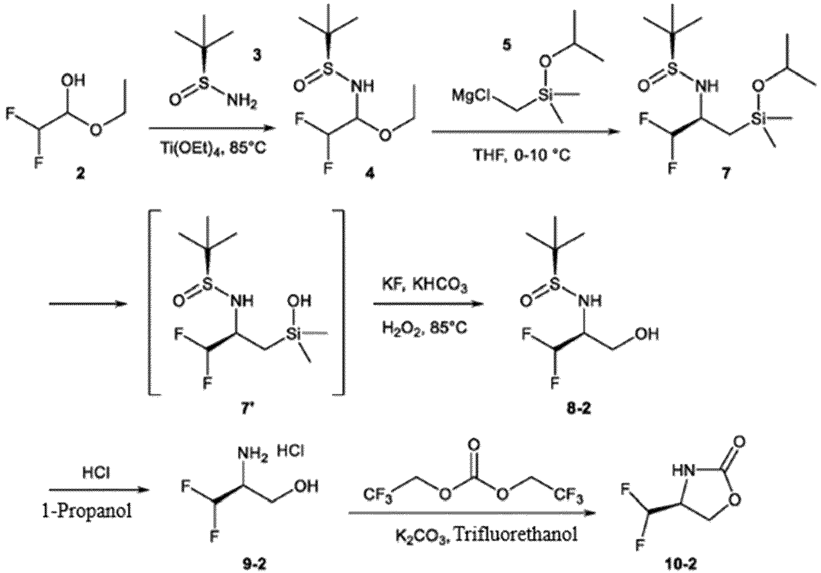

15. Verfahren zur Herstellung einer Verbindung der Formel (8A): 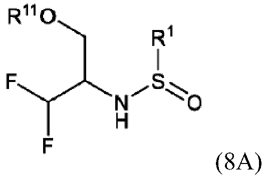 oder eines Salzes davon,
wobei:
R¹ ein gegebenenfalls substituiertes C₁₋₁₂-Alkyl, ein gegebenenfalls substituiertes C₃₋₁₄-Cycloalkyl oder ein gegebenenfalls substituiertes C₆₋₁₄-Aryl ist; und
R¹¹ eine Hydroxylschutzgruppe ist, wobei das Verfahren die folgenden Schritte umfasst:
(b) Umsetzen einer Verbindung der Formel (12A): 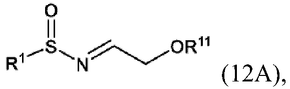 mit einer Verbindung der Formel (13A): 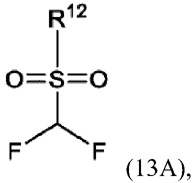 wobei R¹² gegebenenfalls substituiertes C₆₋₁₄-Aryl ist, und einer Base bei einer Temperatur unter 0 °C, um eine Verbindung der Formel (14A): 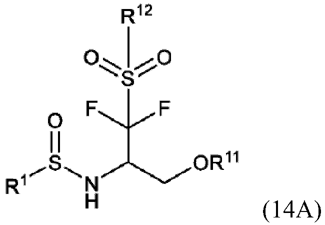 zu bilden; und
(c) Umsetzen der Verbindung der Formel (14A) mit Magnesium in Gegenwart eines Acetatpuffers, um dadurch die Verbindung der Formel (8A) zu bilden, wobei die Hydroxylschutzgruppe ausgewählt ist aus der Gruppe bestehend aus Acetyl, Trimethylacetyl, Benzyl: 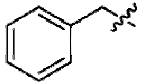 und Silylethern, einschließlich Trimethylsilyl, Triethylsilyl, Triisopropylsilyl, tert-Butyldimethylsilyl, tert-Butyldiphenylsilyl und Di-tert-butylmethylsilyl.

16. Verfahren nach Anspruch 15, ferner umfassend den folgenden Schritt:
(a) Umsetzen einer Verbindung der Formel (11A): 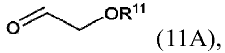 mit einer Sulfonamidverbindung der Formel (3A): 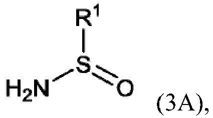 in Gegenwart eines Dehydratisierungsreagens, um die Verbindung der Formel (12A): 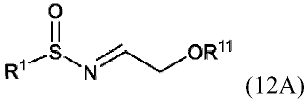 zu bilden,
wobei R¹ und R¹¹ wie in Anspruch 15 definiert sind.

17. Verfahren nach Anspruch 15 oder 16, ferner umfassend den folgenden Schritt:
(d) Umsetzen der Verbindung der Formel (8A): 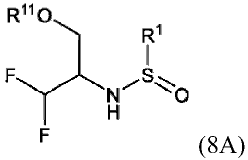 oder eines Salzes davon
mit einer Säure, um eine Aminverbindung der Formel (9A): 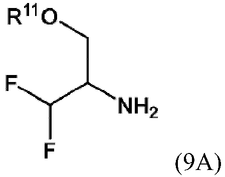 oder ein Säureadditionssalz davon zu erhalten,
wobei R¹ und R¹¹ wie in Anspruch 15 definiert sind.

18. Verfahren nach Anspruch 17, ferner umfassend die folgenden Schritte:
(e) Entfernen der Hydroxylschutzgruppe der Verbindung der Formel (9A), um eine Verbindung der Formel (9-1): 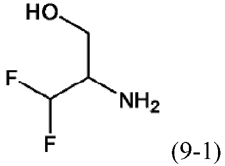 oder ein Säureadditionssalz davon zu bilden,
und
(f) Umsetzen der Verbindung der Formel (9-1) oder eines Säureadditionssalzes davon mit einem Acylierungsreagens, um eine Verbindung der Formel (10-1): 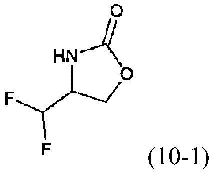 zu bilden.

19. Verfahren nach einem der Ansprüche 15 bis 18, wobei die Verbindung der Formel (12A) die Formel (12B) aufweist: 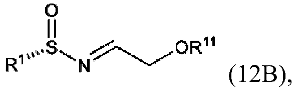 die Verbindung der Formel (14A) die Formel (14B) aufweist: 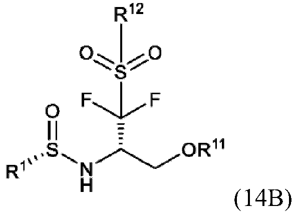 und die Verbindung der Formel (8A) die Formel (8B) aufweist: 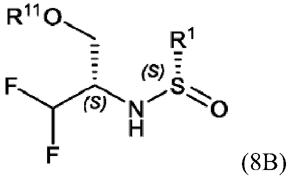 oder ein Salz davon ist,
wobei R¹, R¹¹ und R¹² wie in Anspruch 15 definiert sind.

20. Verfahren nach einem der Ansprüche 16 bis 19, wobei die Verbindung der Formel (3A) die Formel (3B) aufweist: 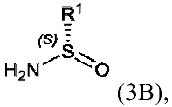 wobei R¹ wie in Anspruch 16 definiert ist.

21. Verfahren nach einem der Ansprüche 17 bis 20, wobei die Verbindung der Formel (9A) die Formel (9B) aufweist: 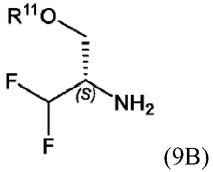 oder ein Salz davon ist,
wobei R¹¹ wie in Anspruch 17 definiert ist.

22. Verfahren nach einem der Ansprüche 18 bis 21, wobei die Säure in Schritt (d) HCl ist und das Säureadditionssalz der Verbindung der Formel (9A) oder (9B) ein Hydrochloridsalz mit der Struktur (9C) ist:

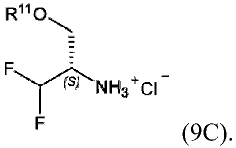

23. Verfahren nach einem der Ansprüche 18 bis 22, wobei die Verbindung der Formel (9-1) die Formel (9-3) aufweist: 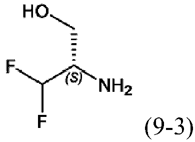 oder das Säureadditionssalz davon ist;
und die Verbindung der Formel (10-1) die Formel (10-2) aufweist:

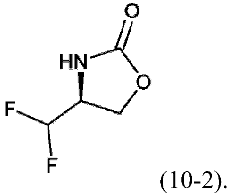

24. Verfahren nach einem der Ansprüche 10-14 und 18-23, ferner umfassend das Umsetzen einer Verbindung der Formel (10-1) oder einer Verbindung (10-2) mit den Strukturen: 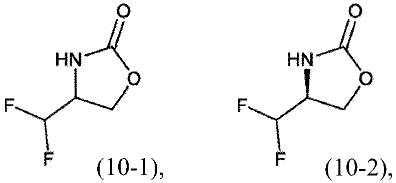 mit Verbindung 15 mit der Struktur: 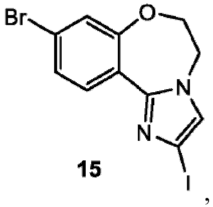 einem Kupfersalz und einem Liganden, um Verbindung 16 mit der Struktur: 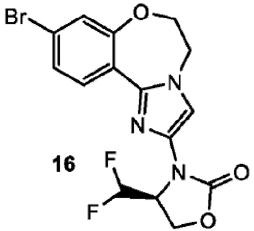 zu bilden.

25. Verfahren nach Anspruch 24, wobei das Kupfersalz Kupfer(II)acetat oder Kupfer(I)iodid ist und der Ligand trans-N,N-Dimethylcyclohexan-1,2-diamin ist.

26. Verfahren nach Anspruch 24 oder 25, ferner umfassend das Umsetzen von Verbindung 16 mit (S)-2-Aminopropansäure und einem Kupfer(I)-Katalysator, um Verbindung 17 mit der Struktur: 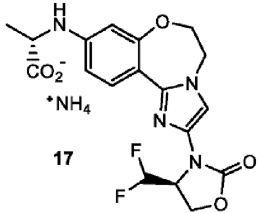 zu bilden.

27. Verfahren nach Anspruch 26, wobei der Kupfer(I)-Katalysator Kupfer(I)oxid ist.

28. Verfahren nach Anspruch 26 oder 27, ferner umfassend das Umsetzen von Verbindung **17** mit Ammoniak oder einem Ammoniakäquivalenten und einem Peptidkopplungsreagens, um Verbindung **18** mit der Struktur: 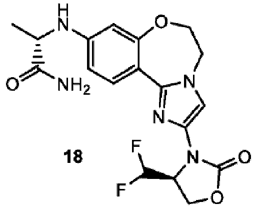 zu bilden.

29. Verfahren zur Herstellung einer Verbindung der Formel (8A): 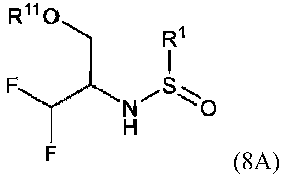 oder eines Salzes davon,
wobei:
R¹ ein gegebenenfalls substituiertes C₁₋₁₂-Alkyl, ein gegebenenfalls substituiertes C₃₋₁₄-Cycloalkyl oder ein gegebenenfalls substituiertes C₆₋₁₄-Aryl ist; und
R¹¹ eine Hydroxylschutzgruppe ist, wobei das Verfahren die folgenden Schritte umfasst:
(ii) Umsetzen einer Verbindung der Formel (4A): 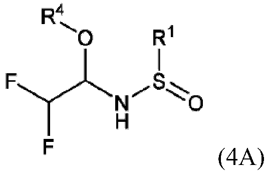
oder eines Salzes davon,
wobei R⁴ ein gegebenenfalls substituiertes C₁₋₆-Alkyl oder Wasserstoff ist;
mit einem Grignard-Reagens, um dadurch die Verbindung mit der Formel (8-A) herzustellen.

30. Verfahren nach Anspruch 29, wobei das Grignard-Reagens durch Umsetzen von Iodmethylpivalat mit sec-Butylmagnesiumchlorid hergestellt wird.

31. Verfahren nach Anspruch 29, ferner umfassend:
(iii) Hydrolysieren der Verbindung mit der Formel (8-A) mit einer Säure, um dadurch eine Aminverbindung der Formel (9-1): 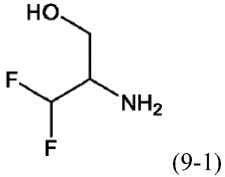 oder ein Säureadditionssalz davon zu erhalten.

32. Verfahren zur Herstellung einer Verbindung der Formel (9-1): 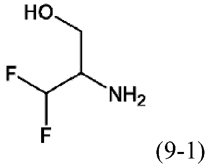 oder eines Säureadditionssalzes davon;
wobei das Verfahren Folgendes umfasst:
(i) Umsetzen einer Verbindung der Formel (2A) 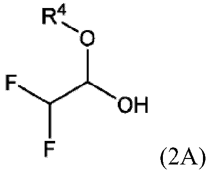 oder eines Salzes davon;
wobei R⁴ ein gegebenenfalls substituiertes C₁₋₆-Alkyl oder Wasserstoff ist;
mit (S)-2-Methylpropan-2-sulfinamid, um dadurch (*S,E*)-N-(2,2-Difluorethyliden)-2-methylpropan-2-sulfinamid mit der Struktur: 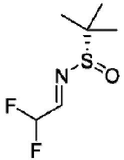 herzustellen;
(ii) Umsetzen von (*S,E*)-N-(2,2-Difluorethyliden)-2-methylpropan-2-sulfinamid mit Trimethylsilylcyanid, um das Aminonitril (S)-N-((S)-1-Cyano-2,2-difluorethyl)-2-methylpropan-2-sulfinamid mit der Struktur: 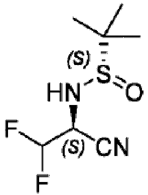 zu erhalten;
(iii) sieren von (S)-N-((S)-1-Cyano-2,2-difluorethyl)-2-methylpropan-2-sulfinamid mit einer Säure, um das Produkt (S)-2-(Chlor-λ⁵-azaneyl)-3,3-difluorpropansäure: 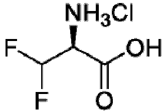 zu erhalten; und
(iv) Reduzieren von (S)-2-(Chlor-λ⁵-azaneyl)-3,3-difluorpropansäure, um die Zwischenverbindung der Formel (9-1) oder das Säureadditionssalz davon bereitzustellen.

## Revendications

1. Composé de formule (8A) : 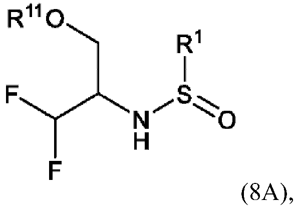 ou un sel de celui-ci, dans lequel :
R¹ représente un alkyle en C₁₋₁₂ éventuellement substitué, un cycloalkyle en C₃₋₁₄ éventuellement substitué ou un aryle en C₆₋₁₄ éventuellement substitué ; et
R¹¹ représente un hydrogène ou un groupe protecteur d'hydroxyle, dans lequel le groupe protecteur d'hydroxyle est choisi dans le groupe constitué par un acétyle, un triméthylacétyle, un benzyle : 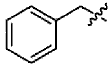 et des éthers de silyle comprenant un triméthylsilyle, un triéthylsilyle, un triiso-propylsilyle, un *tert*-butyldiméthylsilyle, un *tert*-butyldiphénylsilyle et un di-*tert*-butylméthylsilyle.

2. Composé selon la revendication 1, dans lequel R¹ est choisi dans le groupe constitué par un *tert*-butyle, un *tert*-pentyle, un 3-éthylpentan-3-yle, un 1-méthylcyclohexyle, un 1-adamantyle, un phényle et un naphtyle et dans lequel R¹¹ représente un hydrogène ou un benzyle.

3. Composé selon la revendication 1, dans lequel le composé est de formule (8-1) ou
de formule (8-2) : 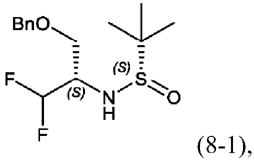 ou un sel de celui-ci ; ou 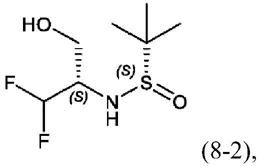 ou un sel de celui-ci.

4. Composé de formule (7A) : 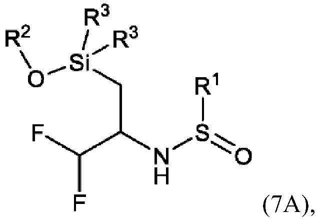 ou un sel de celui-ci,
dans lequel :
R¹ représente un alkyle en C₁₋₁₂ éventuellement substitué, un cycloalkyle en C₃₋₁₄ éventuellement substitué ou un aryle en C₆₋₁₄ éventuellement substitué ;
R² représente un alkyle en C₁₋₁₂ éventuellement substitué ou un aryle en C₆₋₁₄ éventuellement substitué ; et chaque R³ représente indépendamment un alkyle en C₁₋₁₂ éventuellement substitué, un aryle en C₆₋₁₄ éventuellement substitué ou OR².

5. Composé selon la revendication 4, dans lequel le composé est de formule (7) : 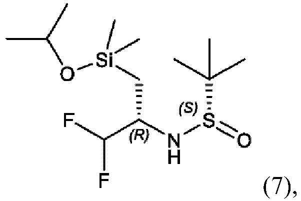 ou un sel de celui-ci.

6. Procédé de préparation d'un composé de formule (8C) : 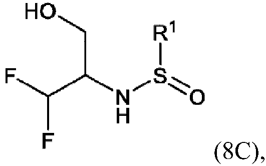 ou d'un sel de celui-ci,
dans lequel :
R¹ représente un alkyle en C₁₋₁₂ éventuellement substitué, un cycloalkyle en C₃₋₁₄ éventuellement substitué ou un aryle en C₆₋₁₄ éventuellement substitué ;
le procédé comprenant les étapes de :
(iii) réaction d'un composé de formule (4A) : 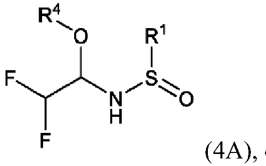 ou d'un sel de celui-ci,
dans lequel :
R¹ représente un alkyle en C₁₋₁₂ éventuellement substitué, un cycloalkyle en C₃₋₁₄ éventuellement substitué ou un aryle en C₆₋₁₄ éventuellement substitué ; et
R⁴ représente un alkyle en C₁₋₆ éventuellement substitué ou un hydrogène ;
avec un réactif de Grignard de formule (5A) : 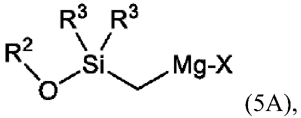 dans lequel :
R² représente un alkyle en C₁₋₁₂ éventuellement substitué ou un aryle en C₆₋₁₄ éventuellement substitué ;
chaque R³ représente indépendamment un alkyle en C₁₋₁₂ éventuellement substitué, un aryle en C₆₋₁₄ éventuellement substitué ou OR² ; et
X représente un halogénure ;
pour former ainsi un composé de formule (7A) : 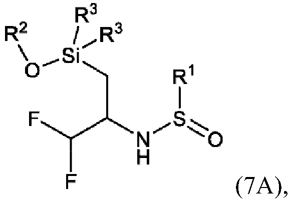 ou un sel de celui-ci, et
(iv) réaction du composé de formule (7A) avec un sel de fluorure, une base et un oxydant pour former le composé de formule (8C).

7. Procédé selon la revendication 6 comprenant en outre les étapes de :
(i) réduction partielle d'un composé de formule (1A) : 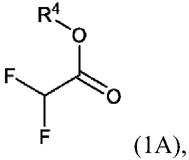 ou d'un sel de celui-ci,
dans lequel R⁴ représente un alkyle en C₁₋₆ éventuellement substitué ou un hydrogène, pour former un composé de formule (2A) : 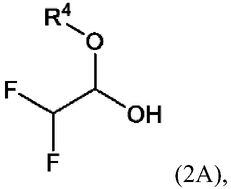 ou un sel de celui-ci,
et
(ii) réaction du composé de formule (2A) avec un composé sulfonamide de formule (3A) : 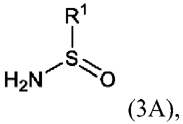
dans lequel R¹ représente un alkyle en C₁₋₁₂ éventuellement substitué, un cycloalkyle en C₃₋₁₄ éventuellement substitué ou un aryle en C₆₋₁₄ éventuellement substitué, en présence d'un réactif de déshydratation
pour former le composé de formule (4A) : 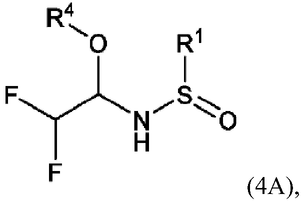 ou un sel de celui-ci.

8. Procédé selon la revendication 6 ou la revendication 7, comprenant en outre l'étape de :
(v) réaction du composé de formule (8C) : 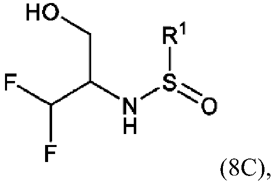 ou d'un sel de celui-ci,
dans lequel R¹ est tel que défini dans la revendication 6, avec un acide pour obtenir ainsi un composé aminé de formule (9-1) : 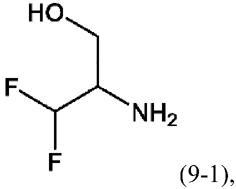 ou un sel d'addition d'acide de celui-ci.

9. Procédé selon la revendication 8, comprenant en outre l'étape de :
(vi) réaction du composé de formule (9-1), ou du sel d'addition d'acide de celui-ci, avec un réactif d'acylation pour former un composé de formule (10-1) : 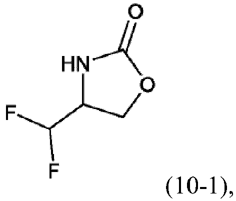 ou un sel de celui-ci.

10. Procédé selon l'une quelconque des revendications 6 à 9, dans lequel R¹ représente un *tert-*butyle ; R² représente un 2-propyle, chaque R³ représente un méthyle, X représente un chlorure ; et R⁴ représente un éthyle.

11. Procédé selon l'une quelconque des revendications 6 à 10, comprenant les étapes de :
(iii) réaction d'un composé de formule (4) : 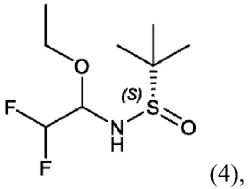 ou d'un sel de celui-ci,
avec un composé de formule (5) : 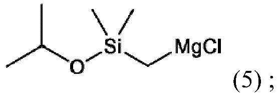 pour former un composé de formule (7) : 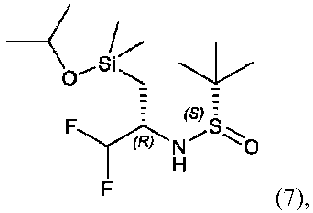 ou un sel de celui-ci ;
et
(iv) réaction du composé de formule (7) avec du fluorure de potassium, du bicarbonate de potassium et du peroxyde d'hydrogène pour former un composé de formule (8-2) :

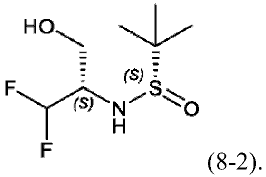

12. Procédé selon l'une quelconque des revendications 6 à 11, dans lequel le sel de fluorure est le fluorure de potassium et la base est le bicarbonate de potassium pour l'étape (iv).

13. Procédé selon l'une quelconque des revendications 8 à 12, dans lequel l'acide pour l'étape (v) est HCl, et le sel d'addition d'acide du composé de formule (9-1) est un sel de chlorhydrate ayant la structure (9-2) :

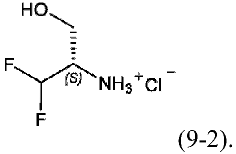

14. Procédé de préparation d'un composé de formule (10-2) selon la séquence d'étapes suivante :

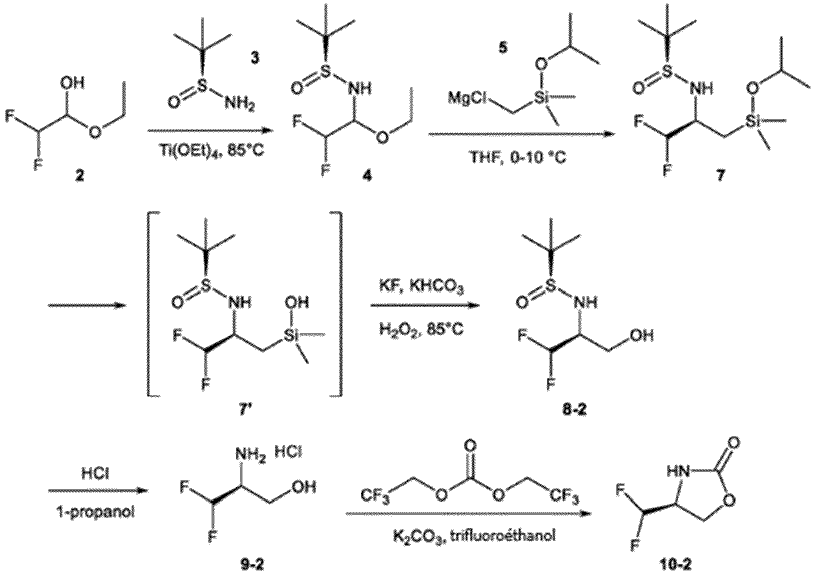

15. Procédé de préparation d'un composé de formule (8A) : 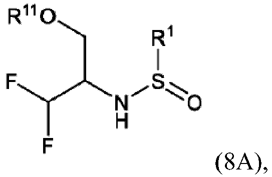 ou d'un sel de celui-ci,
dans lequel :
R¹ représente un alkyle en C₁₋₁₂ éventuellement substitué, un cycloalkyle en C₃₋₁₄ éventuellement substitué ou un aryle en C₆₋₁₄ éventuellement substitué ; et
R¹¹ représente un groupe protecteur d'hydroxyle, le procédé comprenant les étapes de :
(b) réaction d'un composé de formule (12A) : 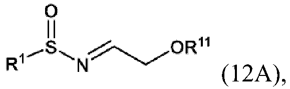 avec un composé de formule (13A) : 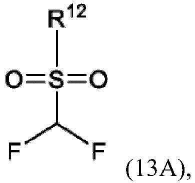 dans lequel R¹² représente un aryle en C₆₋₁₄ éventuellement substitué et une base à une température inférieure à 0 °C pour former un composé de formule (14A) : 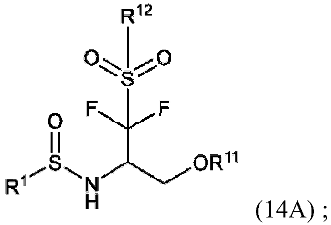 et
(c) réaction du composé de formule (14A) avec du magnésium en présence d'un tampon acétate pour former ainsi le composé de formule (8A), dans lequel le groupe protecteur d'hydroxyle est choisi dans le groupe constitué par un acétyle, un triméthylacétyle, un benzyle : 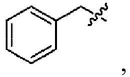 et des éthers de silyle comprenant un triméthylsilyle, un triéthylsilyle, un triiso-propylsilyle, un tert-butyldiméthylsilyle, un tert-butyldiphénylsilyle et un di-*tert*-butylméthylsilyle.

16. Procédé selon la revendication 15, comprenant en outre l'étape de :
(a) réaction d'un composé de formule (11A) : 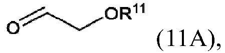 avec un composé sulfonamide de formule (3A) : 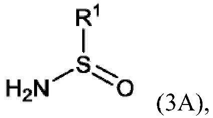 en présence d'un réactif de déshydratation pour former le composé de formule (12A) : 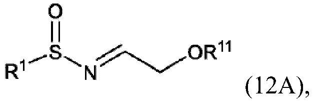 dans lequel R¹ et R¹¹ sont tels que définis dans la revendication 15.

17. Procédé selon la revendication 15 ou 16, comprenant en outre l'étape de :
(d) réaction du composé de formule (8A) : 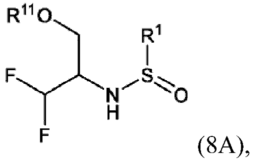 ou d'un sel de celui-ci,
avec un acide pour obtenir un composé aminé de formule (9A) : 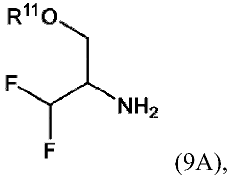 ou un sel d'addition d'acide de celui-ci,
dans lequel R¹ et R¹¹ sont tels que définis dans la revendication 15.

18. Procédé selon la revendication 17, comprenant en outre les étapes de :
(e) élimination du groupe protecteur d'hydroxyle du composé de formule (9A) pour former un composé de formule (9-1) : 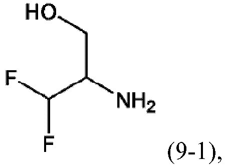 ou un sel d'addition d'acide de celui-ci,
et
(f) réaction du composé de formule (9-1), ou d'un sel d'addition d'acide de celui-ci, avec un réactif d'acylation pour former un composé de formule (10-1) :

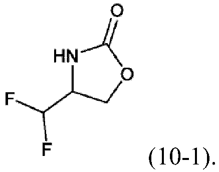

19. Procédé selon l'une quelconque des revendications 15 à 18, dans lequel le composé de formule (12A) est de formule (12B) : 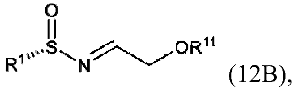 le composé de formule (14A) est de formule (14B) : 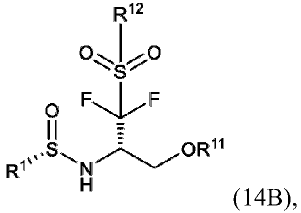 et le composé de formule (8A) est de formule (8B) : 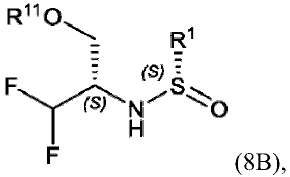 ou un sel de celui-ci,
dans lequel R¹, R¹¹ et R¹² sont tels que définis dans la revendication 15.

20. Procédé selon l'une quelconque des revendications 16 à 19, dans lequel le composé de formule (3A) est de formule (3B) : 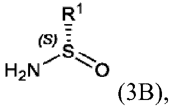 dans lequel R¹ est tel que défini dans la revendication 16.

21. Procédé selon l'une quelconque des revendications 17 à 20, dans lequel le composé de formule (9A) est de formule (9B) : 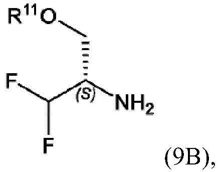 ou un sel de celui-ci,
dans lequel R¹¹ est tel que défini dans la revendication 17.

22. Procédé selon l'une quelconque des revendications 18 à 21, dans lequel l'acide à l'étape (d) est HCl, et le sel d'addition d'acide du composé de formule (9A) ou (9B) est un sel de chlorhydrate ayant la structure (9C) :

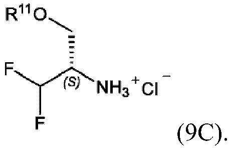

23. Procédé selon l'une quelconque des revendications 18 à 22, dans lequel le composé de formule (9-1) est de formule (9-3) : 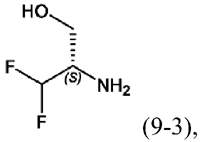 ou le sel d'addition d'acide de celui-ci ;
et le composé de formule (10-1) est de formule (10-2) :

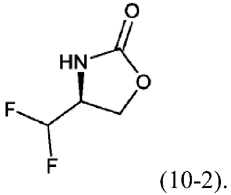

24. Procédé l'une quelconque des revendications 10 à 14 et 18 à 23, comprenant en outre la réaction d'un composé de formule (10-1) ou d'un composé (10-2) ayant les structures : 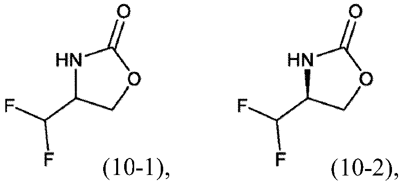 avec le composé 15, ayant la structure : 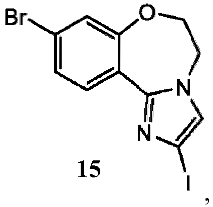 un sel de cuivre et un ligand pour former le composé **16,** ayant la structure :

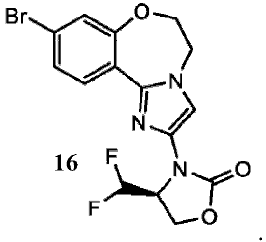

25. Procédé selon la revendication 24, dans lequel le sel de cuivre est l'acétate de cuivre (II) ou l'iodure de cuivre (I) et le ligand est la *trans-N,N*-diméthylcyclohexane-1,2-diamine.

26. Procédé selon la revendication 24 ou 25, comprenant en outre la réaction du composé **16** avec de l'acide (*S*)-2-aminopropanoïque et un catalyseur de cuivre (I) pour former le composé **17**, ayant la structure :

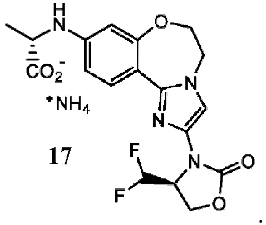

27. Procédé selon la revendication 26, dans lequel le catalyseur de cuivre (I) est l'oxyde de cuivre (I).

28. Procédé selon la revendication 26 ou 27, comprenant en outre la réaction du composé **17** avec de l'ammoniac ou un équivalent d'ammoniac et un réactif de couplage peptidique pour former le composé **18**, ayant la structure :

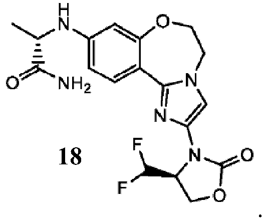

29. Procédé de préparation d'un composé de formule (8A) : 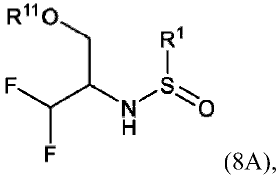 ou d'un sel de celui-ci,
dans lequel :
R¹ représente un alkyle en C₁₋₁₂ éventuellement substitué, un cycloalkyle en C₃₋₁₄ éventuellement substitué ou un aryle en C₆₋₁₄ éventuellement substitué ; et
R¹¹ représente un groupe protecteur d'hydroxyle, le procédé comprenant les étapes de :
(ii) réaction d'un composé de formule (4A) : 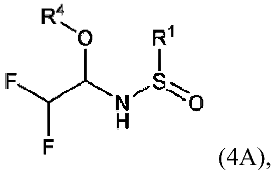 ou d'un sel de celui-ci, dans lequel R⁴ représente un alkyle en C₁₋₆ éventuellement substitué ou un hydrogène ; avec un réactif de Grignard, pour préparer ainsi le composé ayant la formule (8-A).

30. Procédé selon la revendication 29, dans lequel le réactif de Grignard est préparé en faisant réagir du pivalate d'iodométhyle avec du chlorure de sec-butylmagnésium.

31. Procédé selon la revendication 29, comprenant en outre :
(iii) l'hydrolyse du composé ayant la formule (8-A) avec un acide pour obtenir ainsi un composé aminé de formule (9-1) : 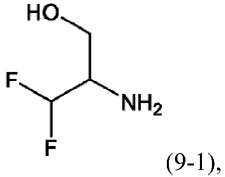 ou un sel d'addition d'acide de celui-ci.

32. Procédé de préparation d'un composé de formule (9-1) : 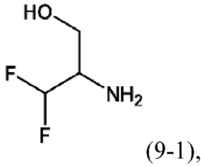 ou d'un sel d'addition d'acide de celui-ci ;
le procédé comprenant :
(i) la réaction d'un composé de formule (2A) 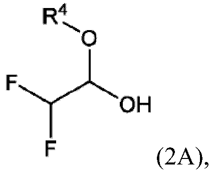 ou d'un sel de celui-ci ;
dans lequel R⁴ représente un alkyle en C₁₋₆ éventuellement substitué ou un hydrogène ;
avec du (S)-2-méthylpropane-2-sulfinamide pour préparer ainsi le (*S,E*)-N-(2,2-difluoroéthylidène)-2-méthylpropane-2-sulfinamide ayant la structure : 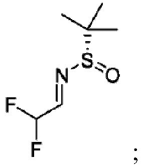
(ii) la réaction du (*S,E*)-N-(2,2-difluoroéthylidène)-2-méthylpropane-2-sulfinamide avec du cyanure de triméthylsilyle pour obtenir l'aminonitrile (S)-N-((S)-1-cyano-2,2-difluoroéthyl)-2-méthylpropane-2-sulfinamide ayant la structure : 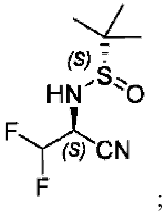
(iii) l'hydrolyse du (S)-N-((S)-1-cyano-2,2-difluoroéthyl)-2-méthylpropane-2-sulfinamide dans de l'acide pour obtenir le produit acide (S)-2-(chloro-λ⁵-azanéyl)-3,3-difluoropropanoïque : 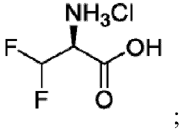 et
(iv) la réduction de l'acide (S)-2-(chloro-λ⁵-azanéyl)-3,3-difluoropropanoïque pour obtenir le composé intermédiaire de formule (9-1) ou un sel d'addition d'acide de celui-ci.
